(19) Europäisches Patentamt / European Patent Office / Office européen des brevets

(11) **EP 4 410 952 B1**

(12) **EUROPEAN PATENT SPECIFICATION**

(45) Date of publication and mention of the grant of the patent:
**24.06.2026 Bulletin 2026/26**

(21) Application number: **22875453.7**

(22) Date of filing: **31.05.2022**

(51) International Patent Classification (IPC):
***C12N 1/02*** *(2006.01)* ***G01N 15/14*** *(2024.01)*
***G01N 21/49*** *(2006.01)* ***G01N 33/48*** *(2006.01)*
***G01N 33/483*** *(2006.01)* ***G01N 33/50*** *(2006.01)*
***G01N 33/68*** *(2006.01)* ***G01N 1/40*** *(2006.01)*
***G01N 15/149*** *(2024.01)* ***G01N 1/30*** *(2006.01)*

(52) Cooperative Patent Classification (CPC):
**G01N 1/30; C12N 1/02; G01N 1/40; G01N 15/1459; G01N 15/149; G01N 21/49; G01N 33/48; G01N 33/483; G01N 33/50; G01N 33/68;**
G01N 2015/1006

(86) International application number:
**PCT/JP2022/022101**

(87) International publication number:
**WO 2023/053574 (06.04.2023 Gazette 2023/14)**

(54) **METHOD FOR ENRICHING CELLS OR CELL NUCLEI**

VERFAHREN ZUR ANREICHERUNG VON ZELLEN ODER ZELLKERNEN

PROCÉDÉ D'ENRICHISSEMENT DE CELLULES OU DE NOYAUX CELLULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2021 JP 2021159878**

(43) Date of publication of application:
**07.08.2024 Bulletin 2024/32**


(73) Proprietor: **Nitto Boseki Co., Ltd.**
**Fukushima-shi,**
**Fukushima 960-8161 (JP)**

(72) Inventors:
- **SATO, Natsuki**
  **Kawasaki-shi, Kanagawa 210-0821 (JP)**
- **NAKATSUJI, Masatoshi**
  **Kawasaki-shi, Kanagawa 210-0821 (JP)**

(74) Representative: **Hoffmann Eitle**
**Patent- und Rechtsanwälte PartmbB**
**Arabellastraße 30**
**81925 München (DE)**



(56) References cited:
**WO-A1-2009/157385 WO-A1-2019/168085**
**JP-A- 2020 511 636 JP-A- S63 252 251**
**US-A1- 2020 408 770**

- **"Current Protocols in Cytometry", 1 January 2011, JOHN WILEY & SONS, INC., Hoboken, NJ, USA, ISBN: 978-0-47-114295-9, article WILLEM E. CORVER ET AL: "High-Resolution Multiparameter DNA Flow Cytometry for the Detection and Sorting of Tumor and Stromal Subpopulations from Paraffin-Embedded Tissues", XP055194143, DOI: 10.1002/0471142956.cy0737s55**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a method for obtaining a sample enriched with cells or cell nuclei of interest from a piece of tissue.

BACKGROUND ART

In recent years, a very large number of biomarkers have been used in various diagnostic purposes, and one of the major specimens for use in analysis of the biomarkers is tissues obtained from a subject. In particular, FFPE tissue sections have been used over the world in the immunohistochemistry (IHC), and recently, components of interest such as DNA have been extracted from the tissue sections for use in various analysis.

On the other hand, one of the problems in extracting a component of interest from a piece of tissue for analysis is that the collected tissue is not always abundant in cells or markers of interest and may not allow us to detect a target marker at a sufficient detection sensitivity or detection rate. For example, a polynucleotide such as DNA has been extracted from a FFPE tissue section and used for quantitative analysis of cancer-related genes, and it has been recommended that the tissue section contains 20% or more of tumor cells as a condition for performing appropriate analysis (Non-Patent Literature). However, meeting such a condition is not always easy from a practical point of view.

Therefore, in case of analyzing various biomarkers with samples prepared from tissue, it will be convenient if a sample enriched with cells or markers of interest can be obtained easily and reliably.

PRIOR ART DOCUMENTS

PATENT DOCUMENTS

Patent Document 1: JP 2005-315862 A
Patent Document 2: JP 2014-1949 A
Patent Document 3: JP 2019-207201 A
Patent Document 4: WO 2010/041423
Patent Document 5: JP 2009-63508 A
Patent Document 6: JP 2009-122115
Patent Document 7: JP 2009-537822
Patent Document 8: WO 2018/203568
Patent Document 9: JP 2015-190922
Patent Document 10: WO 2019/168085
US2020/408770 discloses a sample pretreatment of a piece of tissue using thrombin, proline endopeptidase or hyaluronidase, and water flow crushing.

NON-PATENT DOCUMENTS

Non-Patent Document 1: Mei-Yin C Polley, et al. An international Ki67 reproducibility study. J Natl Cancer Inst. 2013 Dec 18;105(24):1897-906.
Non-Patent Document 2: https://www.bc-cytometry.com/FCM/whats_cytometry-5.html
Non-Patent Document 3: Leers MP, et al. Multi-parameter flow cytometric analysis with detection of the Ki67-Ag in paraffin embedded mammary carcinomas. Cytometry. 1997 Mar 1;27(3):283-9.
Non-Patent Document 4: The Japanese Society of Pathology, Guidelines on the Handling of Pathological Tissue Samples for Clinical Genomics
Non-Patent Document 5: Atsuko Kitano et al. Tumour-infiltrating lymphocytes are correlated with higher expression levels of PD-1 and PD-L1 in early breast cancer. ESMO Open. 2017 May 2;2(2):e000150.
Non-Patent Document 6: Hiroki Kusama et al. Prognostic value of tumor cell DNA content determined by flow cytometry using formalin-fixed paraffin-embedded breast cancer tissues. Breast Cancer Res Treat (2019) 176:75-85
Willem E. Corver ET AL: "High-Resolution Multiparameter DNA Flow Cytometry for the Detection and Sorting of Tumor and Stromal Subpopulations from Paraffin-Embedded Tissues" iIn: "Current Protocols in Cytometry", 1 January 2011, discloses a method for obtaining a sample enriched with cells or cell nuclei of interest from a piece of tissue using flow cytometry.

## SUMMARY OF INVENTION

## TECHNICAL PROBLEM

In view of the aforementioned needs, the present invention provides a method for obtaining a sample enriched with cells or cell nuclei of interest from a piece of tissue. The present invention also provides a method for analyzing biomarkers at an improved detection rate with the sample obtained by the above method. The present invention further provides a kit, an apparatus and a program for use in implementing these methods.

## SOLUTION TO PROBLEM

To achieve the aforementioned objects, the present inventors took an approach of crushing a collected piece of tissue to cellular level to obtain a population of particles including cells or cell nuclei, and sorting or fractionating to concentrate cells or cell nuclei of interest from the obtained population of particles. As a result of studying various conditions for pretreatment of crushing tissue, the present inventors have found that crushing the tissue through specific pretreatment is necessary for sorting or fractionating cells or cell nuclei of interest. It has also been found that cells obtained through specific pretreatment under particular conditions can be used to sort or fractionate certain cells of interest from other cells based on the optical or physical characteristics of the cells or cell nuclei of interest without using biomarkers. The present invention is based on these findings, and provides the following method, kid, apparatus and program.

The invention provides for a method for obtaining a sample enriched with cells or cell nuclei of interest from a piece of tissue according to claim 1.

The method of the present invention can concentrate biomarkers present in cells or cell nuclei of interest, thereby improving the detection rate of a biomarker of interest. In particular, the method allows even a piece of tissue with a low proportion of tumor to be used and is expected to contribute to a more precise cancer gene panel diagnosis and a novel cancer classification. The method of the present invention also allows cells or nuclei of normal tissue, and lymphocytes or nuclei thereof to be separated or isolated. For example, a tumor tissue-infiltrating lymphocytes (TIL) may serve as a predictive factor for prognosis of breast cancer, and therefore if a sample enriched with lymphocytic nuclei as well as a sample enriched with cancer cell nuclei can be fractionated from tissue from a subject, the samples providing analytical advantages, leading to clinical usefulness. The involvement of cells other than tumor cells in a local tumor microenvironment and immune response has been being researched, and these samples are also expected to be useful in this field (Non-Patent Document 5).

## BRIEF DESCRIPTION OF DRAWINGS

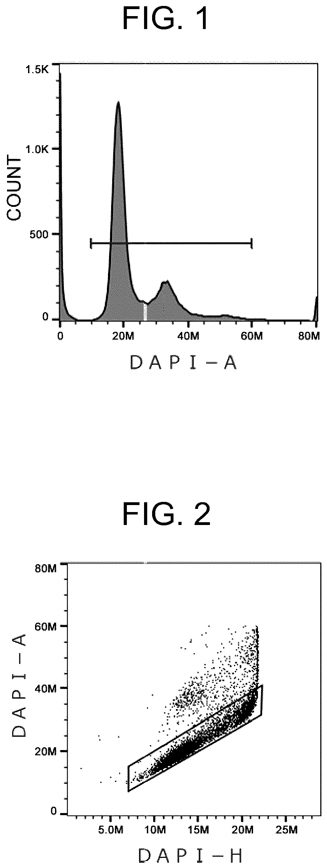

[Fig. 1] Fig. 1 shows a histogram with the fluorescent intensity of DAPI in the horizontal axis and the number of particles in the vertical axis. A formalin-fixed breast cancer cell line was heated, subjected to water-flow crushing, then immunofluorescently stained with an anti-lamin antibody and an isotype control antibody, stained with DAPI, and then analyzed by a flow cytometer. The histogram was generated based on the obtained data. An area indicated by I----- ---I is a region which is considered to have 2n or more nuclear chromosomes. This area was thus gated as being from cells.

[Fig. 2] Fig. 2 shows a scattergram with DAPI-H (height) in the horizontal axis and DAPI-A (area) in the vertical axis, which was developed from the data of a population obtained by gating the area indicated by I--------I. A framed area was selected as a main region of nuclei.

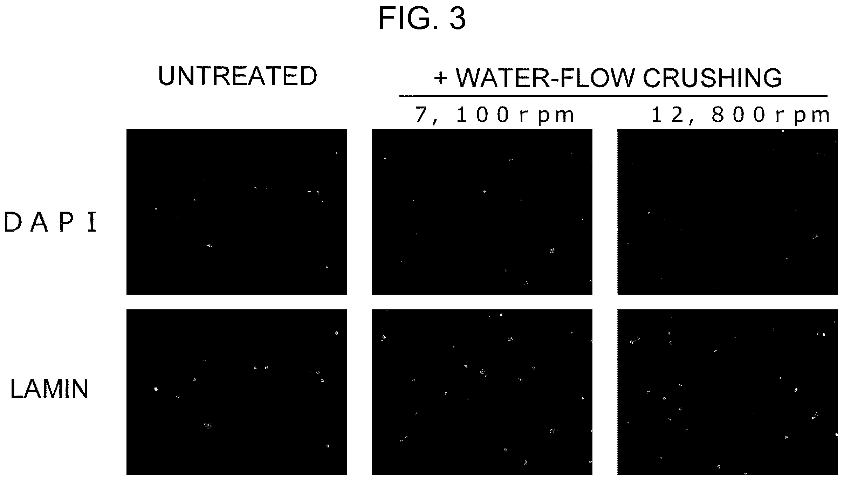

[Fig. 3] Fig. 3 shows images of lamin and DAPI, which were observed with a fluorescence microscope. A formalin-fixed breast cancer cell line was heated, subjected to water-flow crushing at a rotation speed of 7,100 or 12,800 rpm, then immuno-fluorescently stained with an anti-lamin antibody, and stained with DAPI to obtain a sample for microscopic observation.

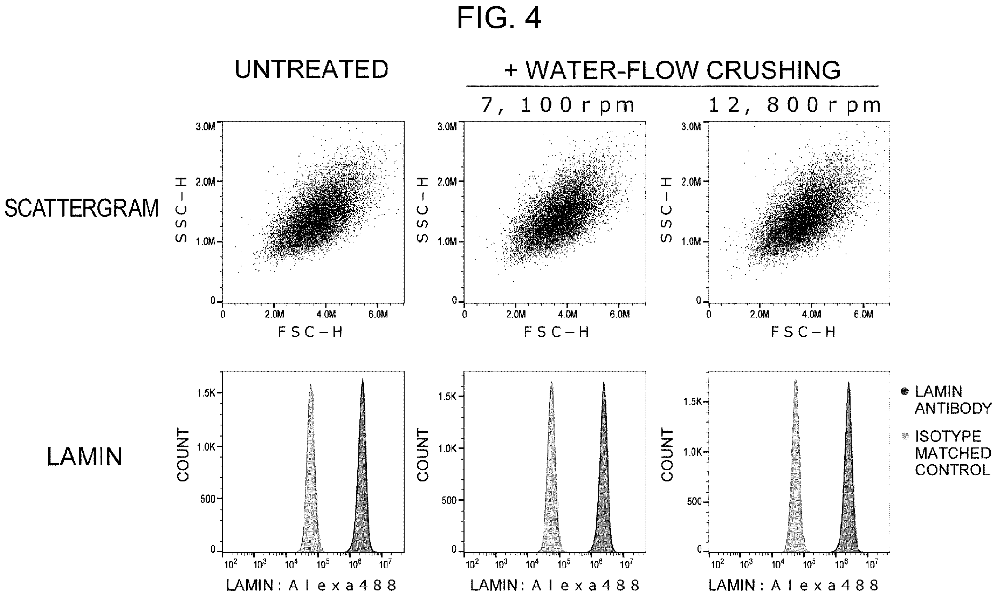

[Fig. 4] Scattergrams with the forward scatter in the horizontal axis and the side scatter in the vertical axis are shown at the upper row, which were generated from data of a population obtained by gating the framed region in the scattergram set forth in Fig. 2. Histograms with the fluorescence intensity of Alexa Fluor 488 (corresponding to the amount of lamin) in the horizontal axis and the number of particles in the vertical axis are shown at the lower row. On each of the histograms, a histogram of an isotype matched control is superimposed.

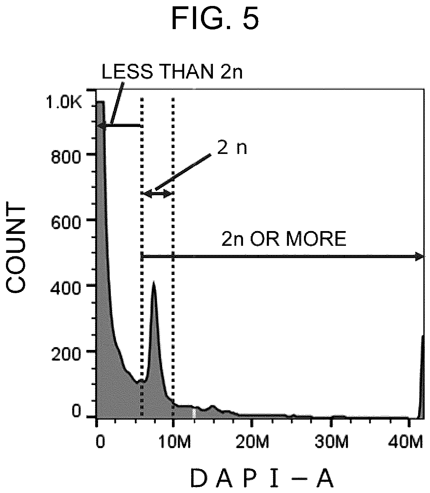

[Fig. 5] Fig. 5 shows a histogram with the fluorescent intensity of DAPI-A in the horizontal axis and the number of particles in the vertical axis. A breast cancer FFPE tissue section was deparaffinized, hydrophilized, heated, subjected to water-flow crushing, stained with DAPI, and then analyzed by a flow cytometer. A histogram was generated based on the obtained data. A region of 2n or more nuclear chromosomes was considered as being from a population in which two or more cells are bound, a region of 2n nuclear chromosomes was considered as being from a single cell, and a region of less than 2n nuclear chromosomes was considered as being from non-cell tissue debris.

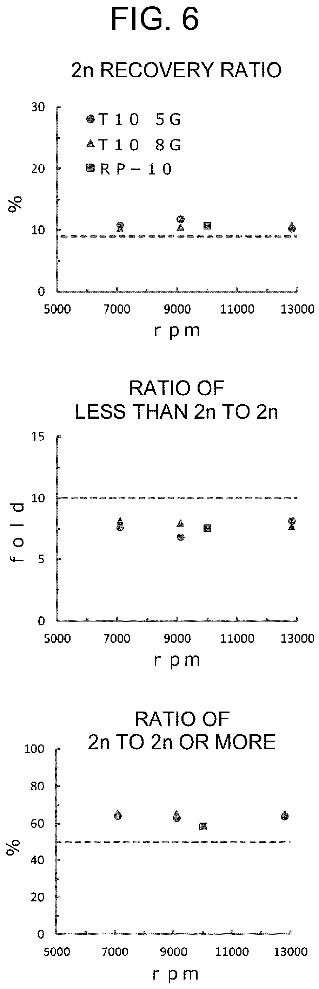

[Fig. 6] Fig. 6 shows effects of difference in conditions for water-flow crushing regarding a crushing apparatus, a rotation speed, and a clearance between a rotary blade and a stationary blade on the ratio of the number of 2n cells (2n recovery ratio), the ratio of the number of less than 2n cells to the number of 2n cells, and the ratio of the number of 2n cells to the number of 2n or more represented by Fig. 5. A breast cancer FFPE tissue section was deparaffinized, hydrophilized, heated, then subjected to water-flow crushing under different conditions regarding the crushing apparatus, the rotation speed, and the clearance between a rotary blade and a stationary blade, stained with DAPI, and then analyzed by a flow cytometer. Based on the obtained data, the ratio of the number of 2n cells (2n recovery ratio), the ratio of the number of less than 2n cells to the number of 2n cells, and the ratio of the number of 2n cells to the number of 2n or more cells represented by Fig. 5 were calculated.

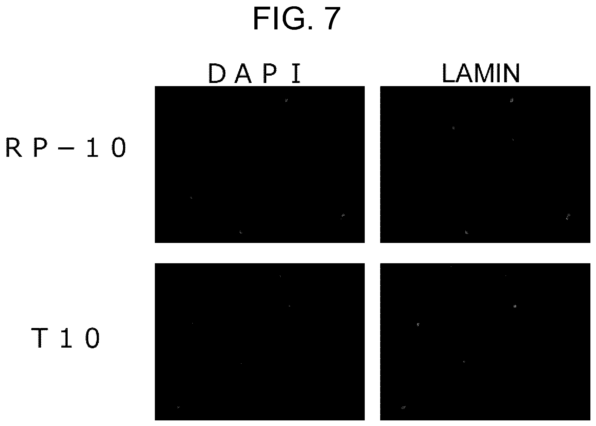

[Fig. 7] Fig. 7 shows images of lamin and DAPI, which were observed with a fluorescence microscope. A FFPE tissue section of breast cancer tissue was deparaffinized, hydrophilized, heated, and subjected to water-flow crushing, then immuno-fluorescently stained with an anti-lamin antibody, and stained with DAPI to prepare a sample for microscopic observation.

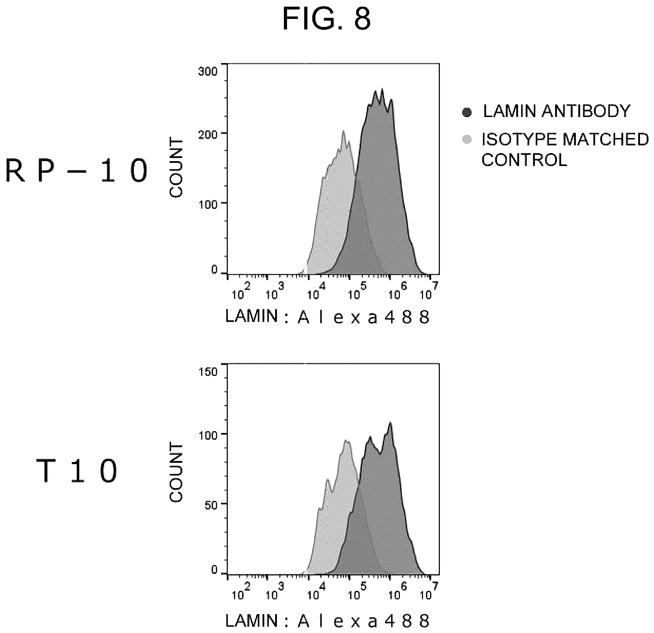

[Fig. 8] Fig. 8 shows histograms with the fluorescent intensity of Alexa Fluor 488 (corresponding to the amount of lamin) in the horizontal axis and the number of particles in the vertical axis. On each of the histograms, a histogram of an isotype matched control is superimposed. A FFPE tissue section of breast cancer tissue was deparaffinized, hydrophilized, heated, and subjected to water-flow crushing, then immuno-fluorescently stained with an anti-lamin antibody, stained with DAPI, and then analyzed by a flow cytometer. A region selected as a cell nuclei region was gated, and histograms were generated based on the data of the gated region in the same procedure as in Test 1.

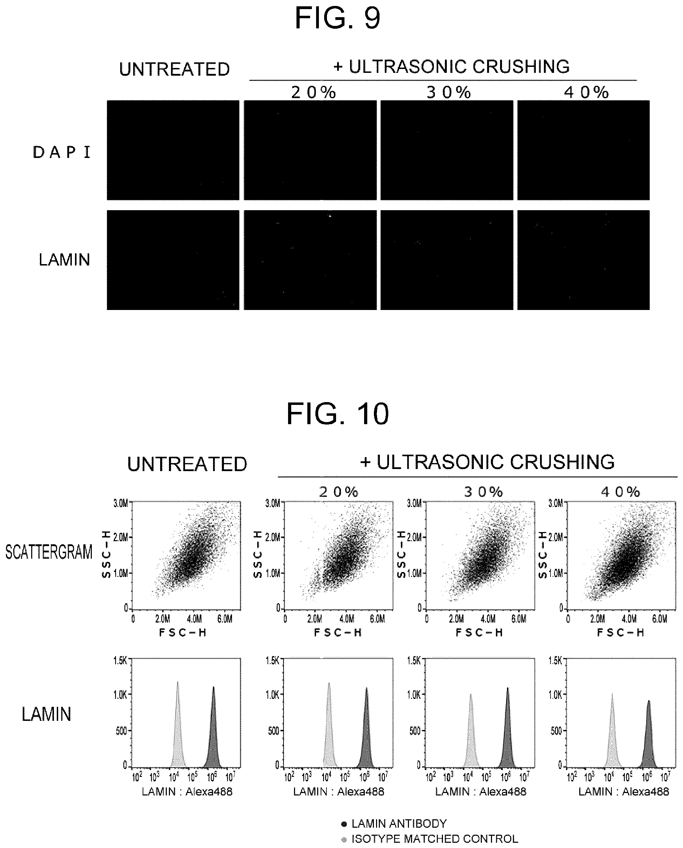

[Fig. 9] Fig. 9 shows images of lamin and DAPI, which were observed with a fluorescence microscope. A formalin-fixed breast cancer cell line was heated, subjected to ultrasonic crushing at an output intensity of 20%, 30% or 40% in terms of an amplitude, then immuno-fluorescently stained with an anti-lamin antibody, and stained with DAPI to obtain a sample for microscopic observation.
[Fig. 10] Scattergrams with the forward scatter in the horizontal axis and the side scatter in the vertical axis are shown at the upper raw. A formalin-fixed breast cancer cell line was heated and subjected to ultrasonic crushing at an output intensity of 20%, 30% or 40% in terms of an amplitude. Subsequently, in the same manner as in Test 1, the breast cancer cell line was fluorescently stained and analyzed by a flow cytometer, and data analysis was performed. A population with 2n or more nuclear chromosomes was gated and based on the data of the gated population scattergrams were generated. Histograms with the fluorescence intensity of Alexa Fluor 488 (corresponding to the amount of lamin) in the horizontal axis and the number of particles in the vertical axis are shown at the lower raw, which were generated from the data of the gated population. On each of the histograms, a histogram of an isotype matched control is superimposed.

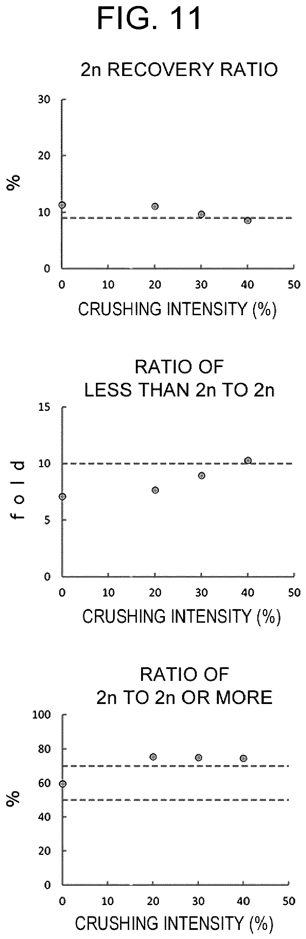

[Fig. 11] Fig. 11 shows effects of change in the output intensity for ultrasonic crushing in pretreatment including water-flow crushing and ultrasonic crushing on the ratio of the number of 2n cells (2n recovery ratio), the ratio of the number of less than 2n cells to the number of 2n cells, and the ratio of the number of 2n cells to the number of 2n or more cells. A breast cancer FFPE tissue section was deparaffinized, hydrophilized, heated, and then subjected to water-flow crushing and to ultrasonic crushing at an output intensity of 20%, 30% or 40% in terms of an amplitude. The treated sample was stained with DAPI, and then analyzed by a flow cytometer. Based on the obtained data, the ratio of the number of 2n cells (2n recovery ratio), the ratio of the number of less than 2n cells to the number of 2n cells, and the ratio of the number of 2n cells to the number of 2n or more cells were calculated.

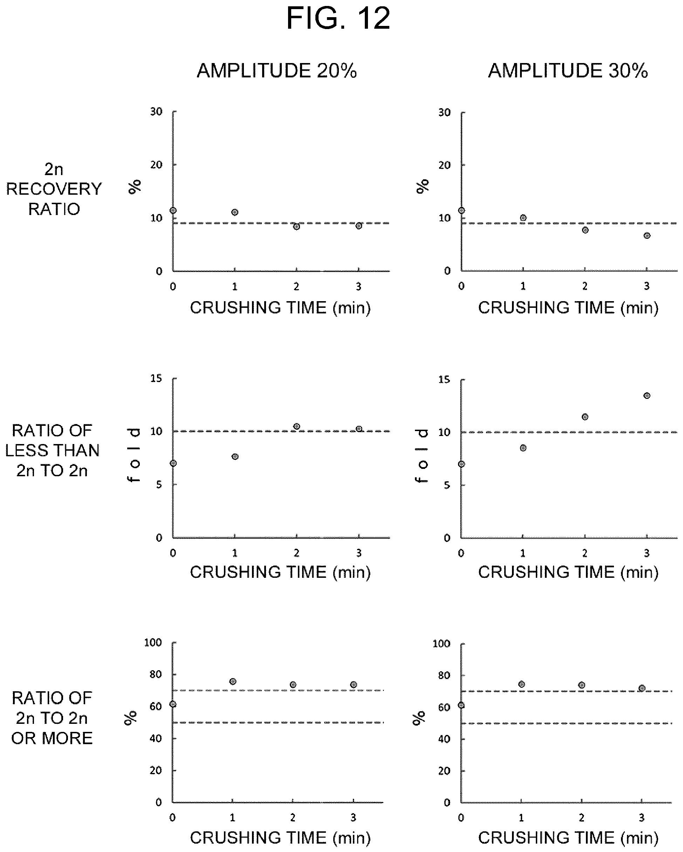

[Fig. 12] Fig. 12 shows effects of change in the output intensity and the crushing time for ultrasonic crushing in pretreatment including water-flow crushing and ultrasonic crushing on the ratio of the number of 2n cells (2n recovery ratio), the ratio of the number of less than 2n cells to the number of 2n cells, and the ratio of the number of 2n cells to the number of 2n or more cells. A breast cancer FFPE tissue section was deparaffinized, hydrophilized, heated, and then subjected to water-flow crushing and to ultrasonic crushing at an output intensity of 20% or 30% in terms of an amplitude for 1, 2 or 3 minutes. The treated sample was stained with DAPI, and then analyzed by a flow cytometer. Based on the obtained data, the ratio of the number of 2n cells (2n recovery ratio), the ratio of the number of less than 2n cells to the number of 2n cells, and the ratio of the number of 2n cells to the ratio of the number of 2n or more cells were calculated.

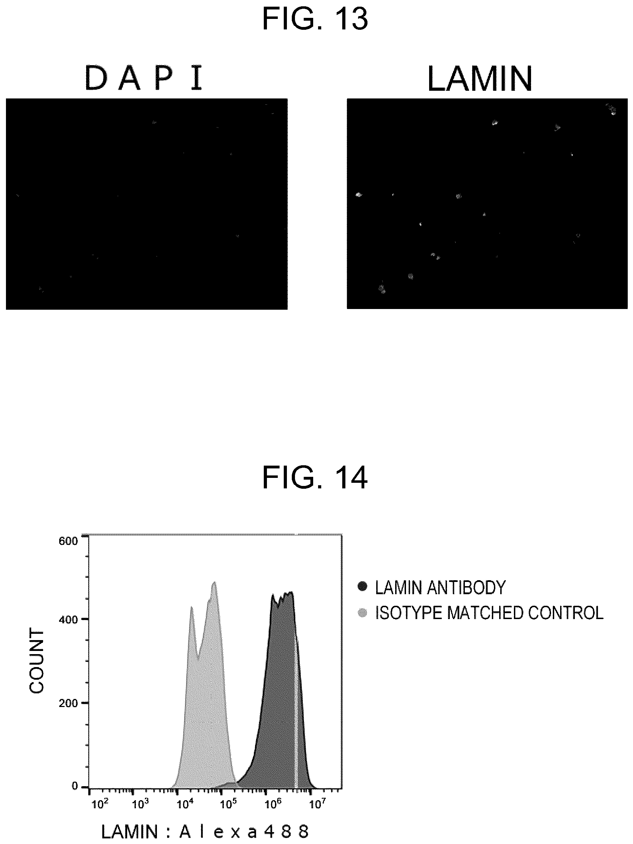

[Fig. 13] Fig. 13 shows images of lamin and DAPI, which were observed with a fluorescence microscope. A breast cancer FFPE tissue section was subjected to pretreatment including water-flow crushing and ultrasonic crushing, then immuno-fluorescently stained with an anti-lamin antibody, and stained with DAPI to obtain a sample for microscopic observation.

[Fig. 14] Fig. 14 shows a histogram with the fluorescent intensity of Alexa Fluor 488 (corresponding to the amount of lamin) in the horizontal axis and the number of particles in the vertical axis. On each of the histograms, a histogram of an isotype matched control is superimposed. A FFPE tissue section of breast cancer tissue was deparaffinized, hydrophilized, heated, subjected to water-flow crushing and ultrasonic crushing, then immuno-fluorescently stained with an anti-lamin antibody, stained with DAPI, and then analyzed by a flow cytometer. A region selected as a cell nuclei region was gated and histograms were generated based on the data of the gated region in the same procedure as in Test 1.

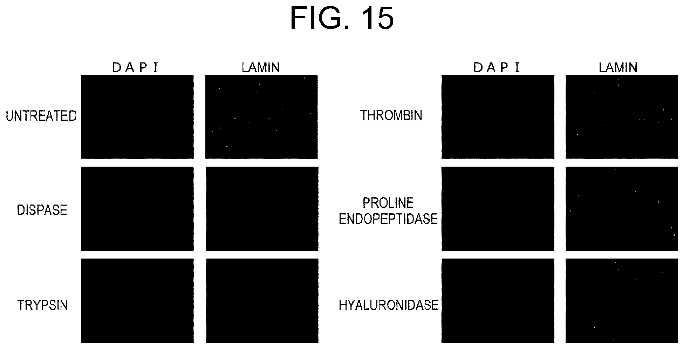

[Fig. 15] Fig. 15 shows images of lamin and DAPI which were observed with a fluorescence microscope. A formalin-fixed breast cancer cell line was heated, subjected to enzymatic treatment with various enzymes (thrombin, dispase, trypsin, proline endopeptidase and hyaluronidase) or not subjected to the enzymatic treatment, immuno-fluorescently stained with an anti-lamin antibody, and stained with DAPI to obtain a sample for microscopic observation.

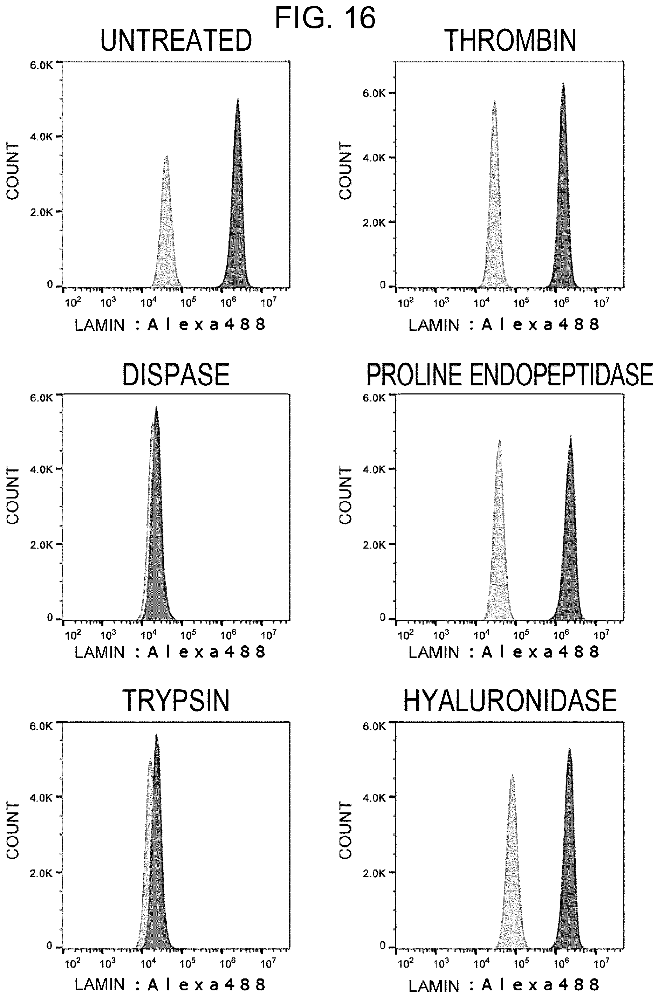

[Fig. 16] Fig. 16 shows histograms with the fluorescent intensity of Alexa Fluor 488 (corresponding to the amount of lamin) in the horizontal axis and the number of particles in the vertical axis. On each of the histograms, a histogram of an isotype matched control is superimposed. A human breast cancer cell fixed with formalin was heated, subjected to enzymatic treatment with various enzymes (thrombin, dispase, trypsin, proline endopeptidase and hyaluronidase) or not subjected to the enzymatic treatment, immuno-fluorescently stained with an anti-lamin antibody, stained with DAPI, and then analyzed by a flow cytometer. A region selected as a cell nuclei region was gated, and histograms were generated based on the data of the gated region in the same procedure as in Test 1.

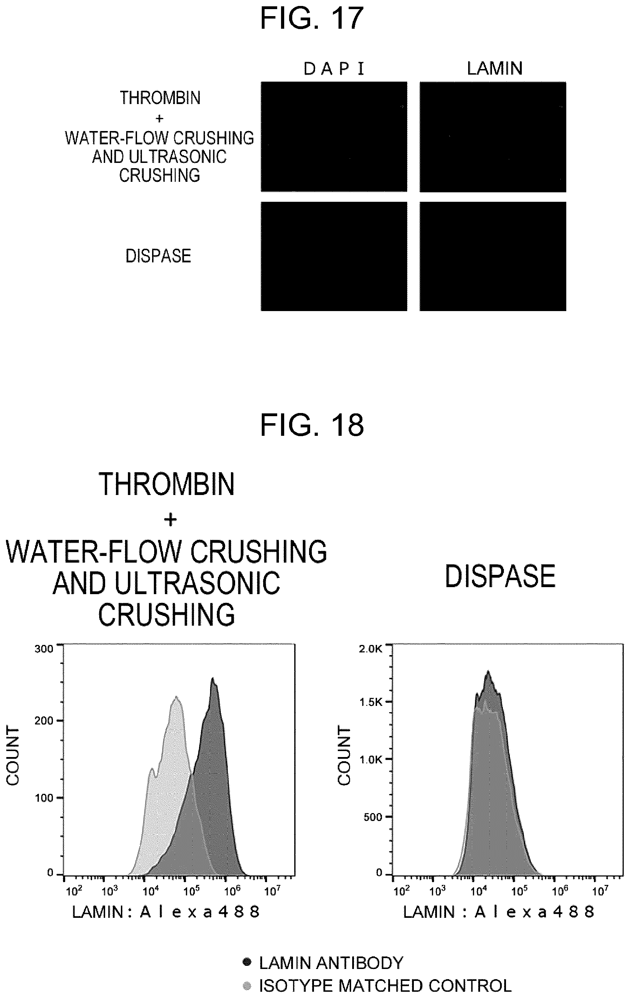

[Fig. 17] Fig. 17 shows images of lamin and DAPI, which were observed with a fluorescence microscope. A FFPE tissue section of breast cancer tissue was deparaffinized, hydrophilized, heated, enzymatically treated with thrombin or dispase, subjected to water-flow crushing and ultrasonic crushing, then immuno-fluorescently stained with an anti-lamin antibody, and stained with DAPI to obtain the microscopic test sample.

[Fig. 18] Fig. 18 shows histograms with the fluorescent intensity of Alexa Fluor 488 (corresponding to the amount of lamin) in the horizontal axis and the number of particles in the vertical axis. On each of the histograms, a histogram of an isotype matched control is superimposed. A FFPE tissue section of breast cancer tissue was deparaffinized, hydrophilized, heated, enzymatically treated with thrombin or dispase, subjected to water-flow crushing and ultrasonic crushing, then immuno-fluorescently stained with an anti-lamin antibody, stained with DAPI, and then analyzed by a flow cytometer. A region selected as a cell nuclei region was gated and histograms were generated based on the data of the gated region in the same procedure as in Test 1.

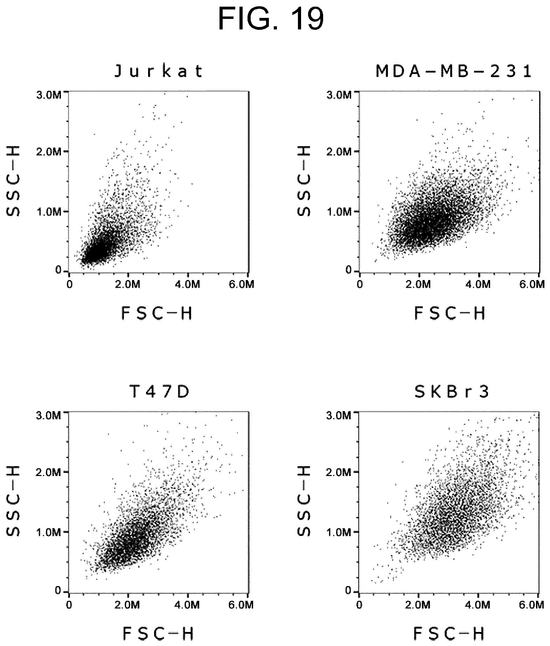

[Fig. 19] Fig. 19 shows scattergrams with the forward scatter in the horizontal axis and the side scatter in the vertical axis. Various tumor cell lines (MB231, T47D and SKBR3) and a lymphoblastic cell line (Jurkat) fixed with formalin were heated, treated with thrombin, stained with DAPI, and then analyzed by a flow cytometer. The obtained data was used to gate a population from the gated population and the scattergrams were generated in the same manner as in Test 1.

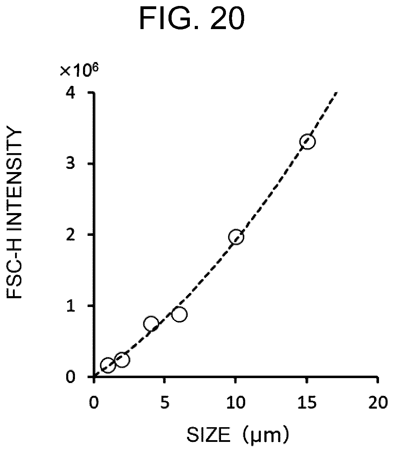

[Fig. 20] Fig. 20 shows a graph (secondary regression curve) generated by analyzing a size calibration bead solution by a flow cytometer and from the obtained data plotting the particle size on the horizontal axis and the forward scatter intensity on the vertical axis .

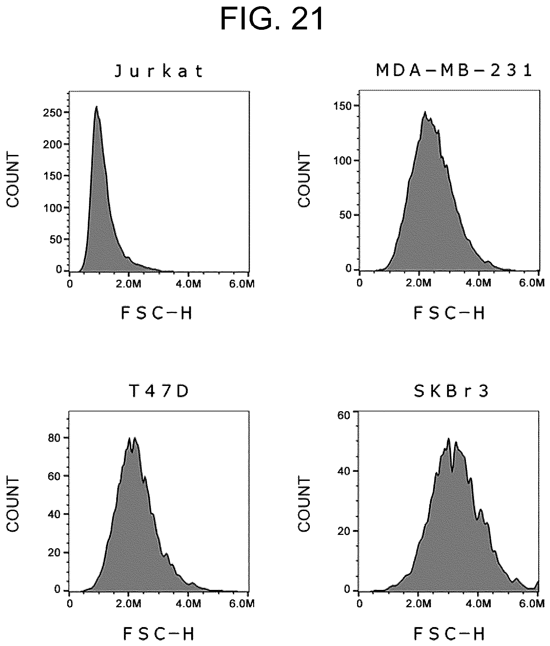

[Fig. 21] Fig. 21 shows histograms with the forward scatter in the horizontal axis and the number of particles in the vertical axis. Various tumor cell lines (MB231, T47D and SKBR3) and a lymphoblastic cell line (Jurkat) fixed with formalin were heated, treated with thrombin, stained with DAPI, and then analyzed by a flow cytometer. The obtained data was used to gate a population and from the gated population histograms were generated in the same manner as in Test 1.

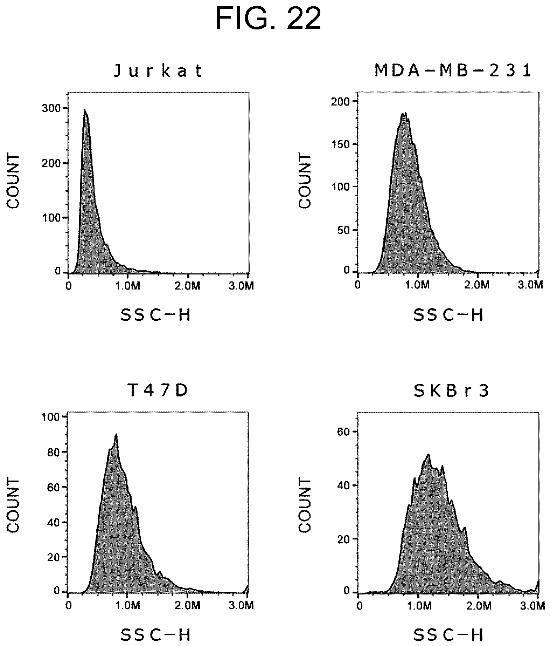

[Fig. 22] Fig. 22 shows histograms with the side scatter in the horizontal axis and the number of particles in the vertical axis. Various tumor cell lines (MB231, T47D and SKBR3) and a lymphoblastic cell line (Jurkat) fixed with formalin were heated, treated with thrombin, stained with DAPI, and then analyzed by a flow cytometer. The obtained data was used to gate a population and from the gated population histograms were generated in the same manner as in Test 1.

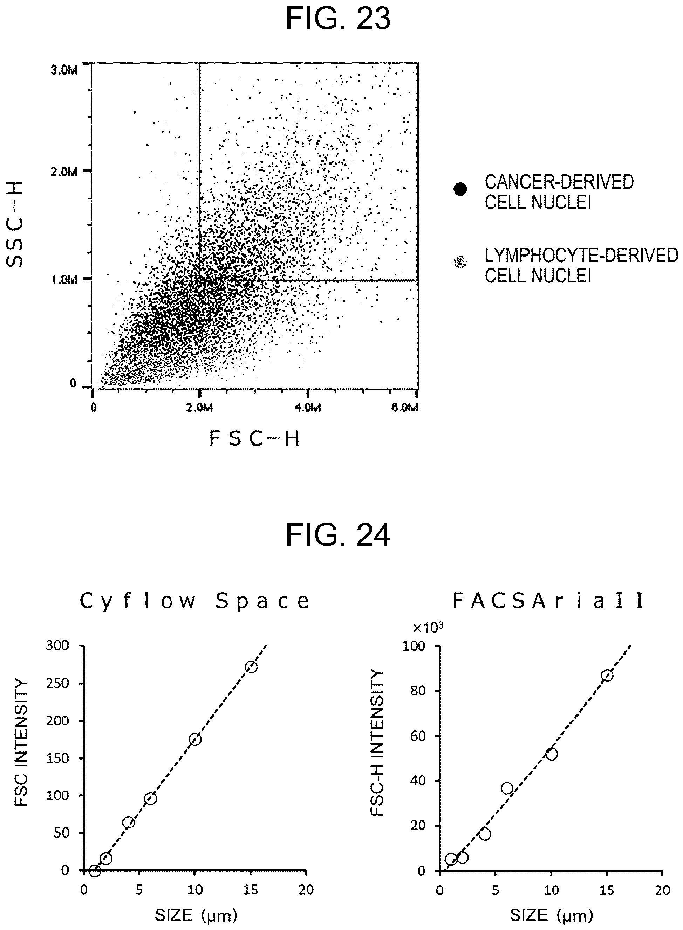

[Fig. 23] Fig. 23 shows a diagram obtained by overlaying scattergrams of a breast cancer tissue and a normal lymph node with the forward scatter in the horizontal axis and the side scatter in the vertical axis. FFPE tissue sections of a breast cancer tissue and a normal lymph node were deparaffinized, hydrophilized, heated, treated with thrombin, subjected to water-flow crushing and ultrasonic crushing, stained with DAPI, and then analyzed by a flow cytometer, and the obtained data was used to gate a population in the same manner as in Test 1. From the gated population, the scattergrams were prepared.

[Fig. 24] Fig. 24 shows graphs (secondary regression curves) obtained by plotting the particle size on the horizontal axis and the forward scatter (DSC-H) intensity modal value on the vertical axis from data obtained by testing a size calibration bead solution with different flow cytometers (Cyflow Space from Sysmex Corporation and FACSAriaII from Becton Dickinson and Co).

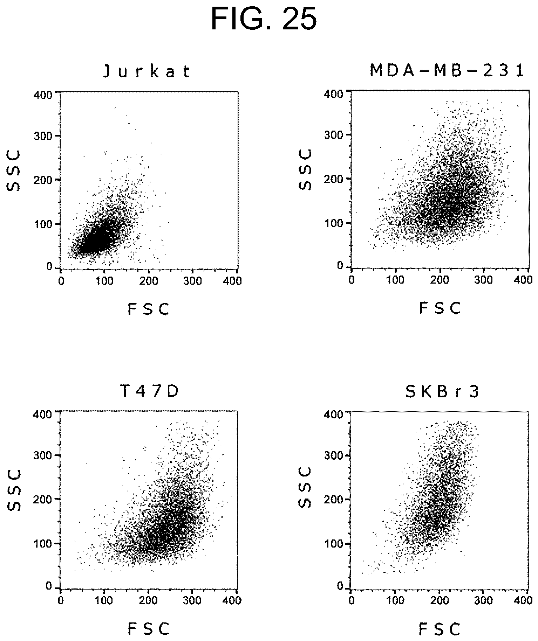

[Fig. 25] Fig. 25 shows scattergrams with the forward scatter in the horizontal axis and the side scatter in the vertical axis. Various tumor cell lines (MB231, T47D and SKBR3) and a lymphoblastic cell line (Jurkat) fixed with formalin were heated, treated with thrombin, stained with DAPI, and then analyzed by a different flow cytometer (Cyflow Space from Sysmex Corporation). The obtained data was used to gate a population and from the gated population scattergrams were generated in the same manner as in Test 1.

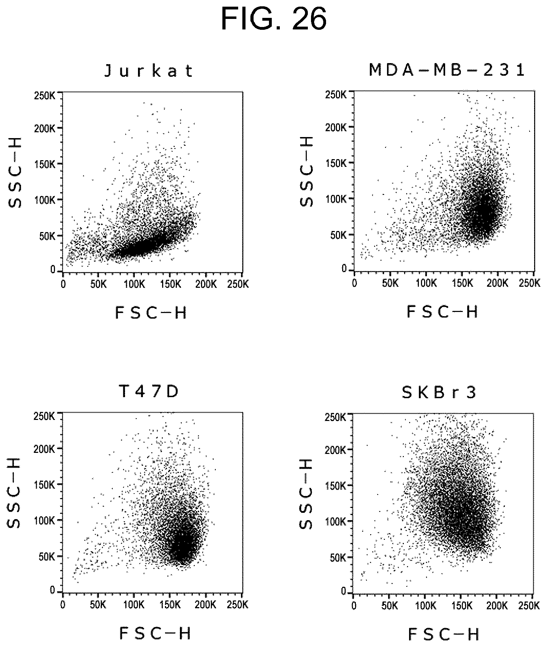

[Fig. 26] Fig. 26 shows scattergrams with the forward scatter (FSC-H) in the horizontal axis and the side scatter (SSC-H) in the vertical axis. Various tumor cell lines (MB231, T47D and SKBR3) and a lymphoblastic cell line (Jurkat) fixed with formalin were heated, treated with thrombin, stained with DAPI, and then analyzed by a different flow cytometer (FACSAriaII from Becton Dickinson and Co). The obtained data was used to gate a population and from the gated population scattergrams were generated in the same manner as in Test 1.

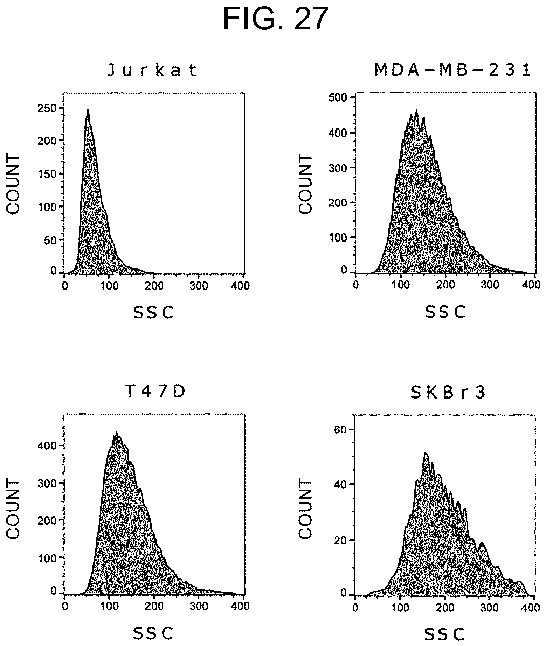

[Fig. 27] Fig. 27 shows histograms with the side scatter (SSC) intensity in the horizontal axis and the number of particles in the vertical axis. Various tumor cell lines (MB231, T47D and SKBR3) and a lymphoblastic cell line (Jurkat) fixed with formalin were heated, treated with thrombin, stained with DAPI, and then analyzed by a different flow cytometer (Cyflow Space from Sysmex Corporation). The obtained data was used to gate a population and from the gated population histograms were generated in the same manner as in Test 1.

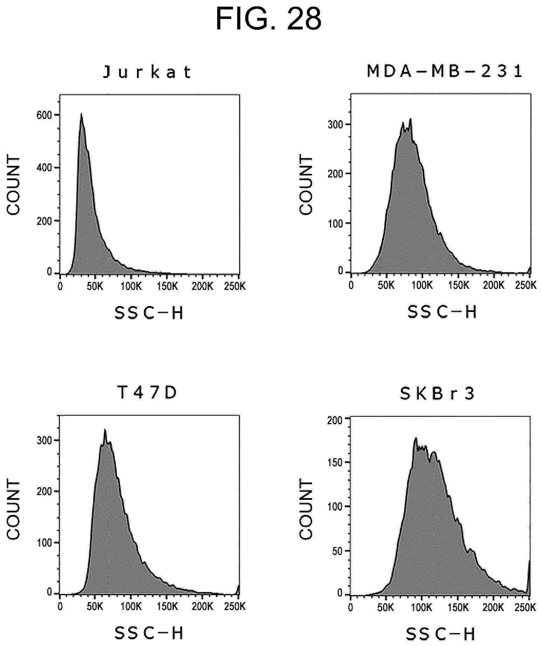

[Fig. 28] Fig. 28 shows histograms with the side scatter (SSC) intensity in the horizontal axis and the number of particles in the vertical axis. Various tumor cell lines (MB231, T47D and SKBR3) and a lymphoblastic cell line (Jurkat) fixed with formalin were heated, treated with thrombin, stained with DAPI, and then analyzed by a different flow cytometer (FACSAriaII from Becton Dickinson and Co). The obtained data was used to gate a population and from the gated population histograms were generated in the same manner as in Test 1.

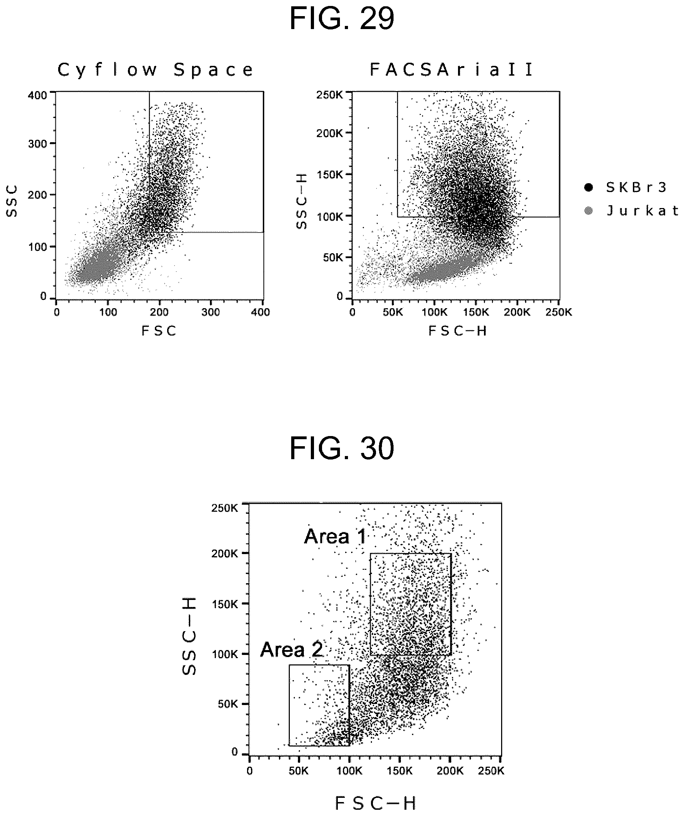

[Fig. 29] Fig. 29 shows a diagram obtained by overlaying the scattergrams with the forward scatter in the horizontal axis and the side scatter in the vertical axis regarding the breast cancer cell line (SKBR3) and the lymphoblastic cell line (Jurkat) set forth in Figs. 25 and 26. The data of the flow cytometer analysis regarding the breast cancer cell line (SKBR3) and the lymphoblastic cell line (Jurkat) set forth in Figs. 25 and 26 was used to gate a population and from the gated population these scattergrams were generated in the same manner as in Test 1.

[Fig. 30] Fig. 30 shows a scattergram with the forward scatter in the horizontal axis and the side scatter in the vertical axis. A FFPE tissue section of breast cancer tissue was deparaffinized, hydrophilized, heated, treated with thrombin, subjected to water-flow crushing and ultrasonic crushing, then immuno-fluorescently stained with an anti-pan-cytokeratin antibody, stained with DAPI, and then analyzed by a different flow cytometer (FACSAriaII from Becton Dickinson and Co). A region selected as a cell nuclei region was gated and a scattergram was generated based on the data of the gated region in the same procedure as in Test 1.

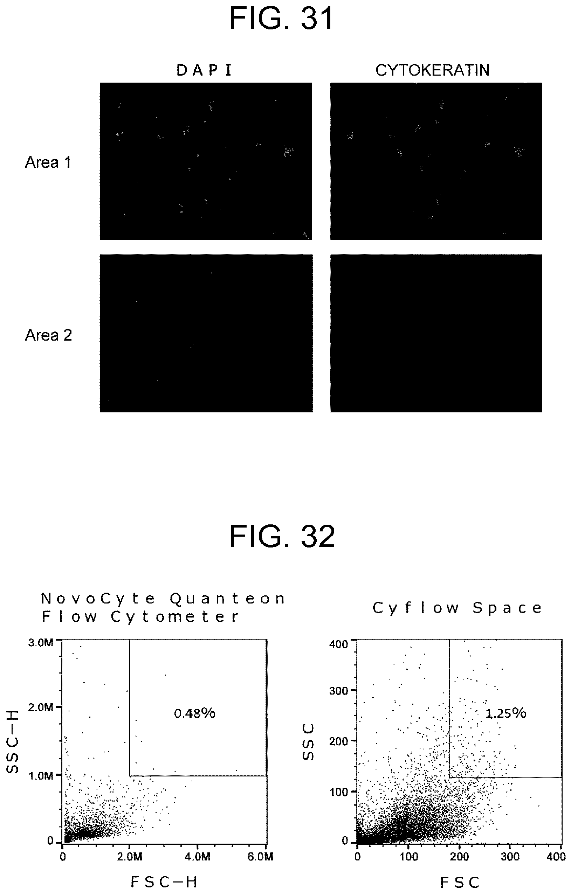

[Fig. 31] Fig. 31 shows microscopic images of DAPI and cytokeratin in Areas 1 and 2 on Fig. 30.

[Fig. 32] Fig. 32 shows scattergrams with the forward scatter in the horizontal axis and the side scatter in the vertical axis. A population of particles in Area 2 on Fig. 30 was fractionated, and analyzed by two flow cytometers (NovoCyte Quanteon Flow Cytometer manufactured by Agilent Technologies and Cyflow Space manufactured by Sysmex Corporation) to obtain data, from which the scattergrams were prepared.

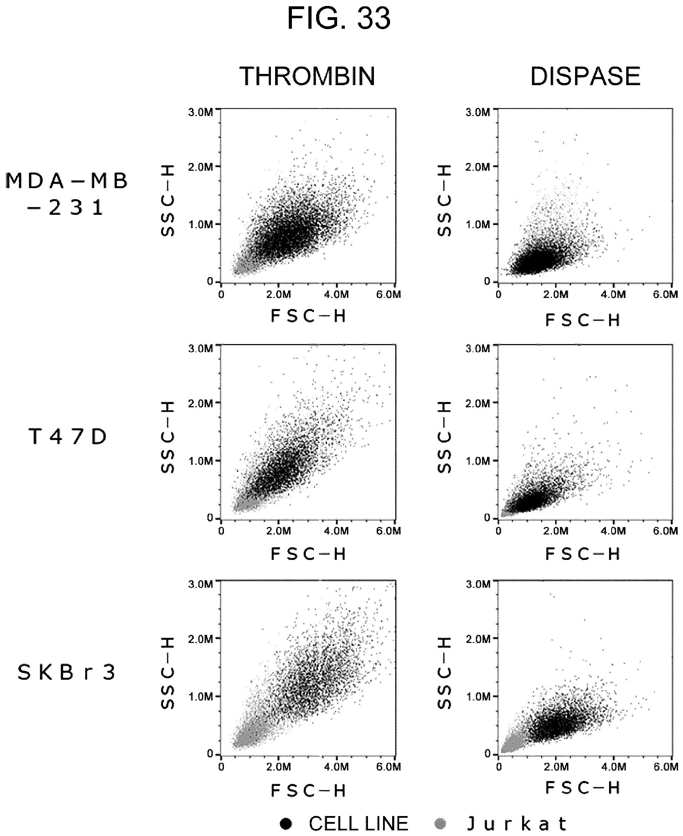

[Fig. 33] Fig. 33 shows scattergrams with the forward scatter in the horizontal axis and the side scatter in the vertical axis. Various tumor cell lines (MB231, T47D and SKBR3) and a lymphoblastic cell line (Jurkat) fixed with formalin were heated, treated with different enzymes (thrombin and dispase), stained with DAPI, and then analyzed by a flow cytometer. A region selected as a cell nuclei region was gated and scattergrams were generated based on the data of the gated region in the same procedure as in Test 1.

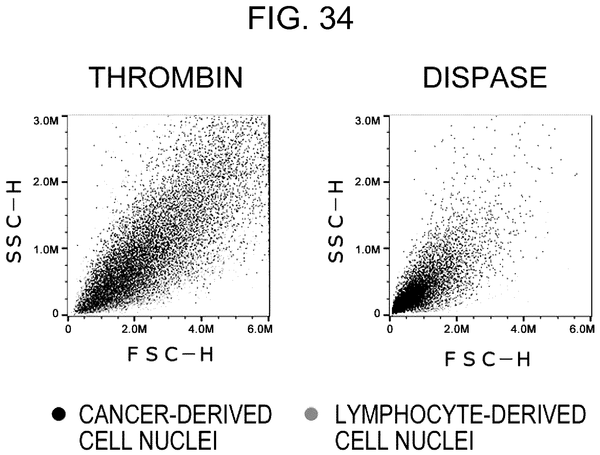

[Fig. 34] Fig. 34 shows scattergrams with the forward scatter in the horizontal axis and the side scatter in the vertical axis. FFPE tissue sections of breast cancer tissue and a normal lymph node were deparaffinized, hydrophilized, heated, treated with different enzymes (thrombin and dispase), stained with DAPI, and then analyzed by a flow cytometer. A region selected as a cell nuclei region was gated and scattergrams were generated based on the data of the gated region in the same procedure as in Test 1.

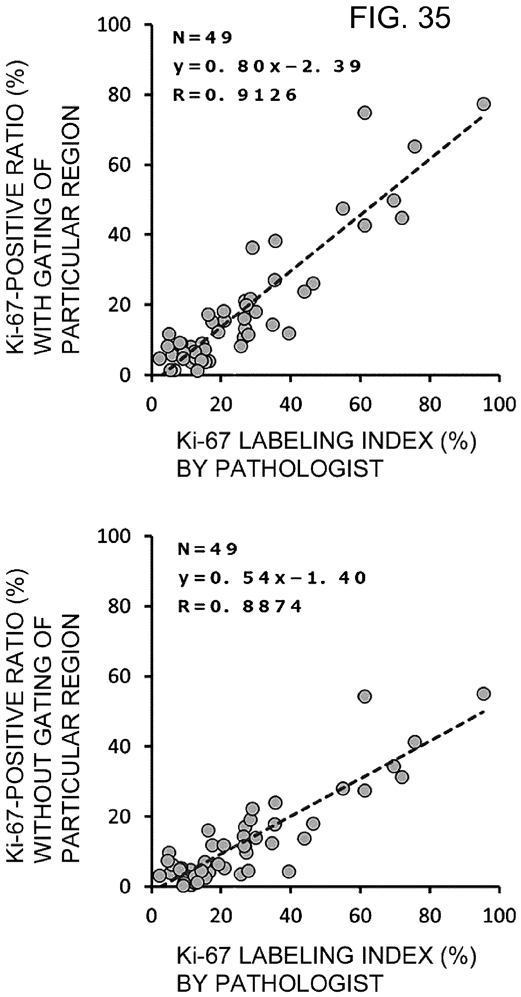

[Fig. 35] Fig. 35 shows correlation graphs between the results of analysis by a flow cytometer and the results of the IHC analysis by a pathologist for the Ki-67-positive ratio in a FFPE tissue section of breast cancer tissue. The ratio of Ki-67-positive nuclei by the flow cytometer in the correlation graph at the upper side was produced by gating a cancer cell nuclei region having a FSC-H intensity of $2.00 \times 10^6$ to $5.00 \times 10^6$ and a SSC-H intensity of $1.00 \times 10^6$ to $2.00 \times 10^6$ on a scattergram with the FSC-H (forward scatter) in the horizontal axis and the SSC-H (side scatter) in the vertical axis, and calculating the ratio of the number of Ki-67-positive nuclei to the number of all the gated cell nuclei. On the other hand, the ratio of Ki-67-positive nuclei by the flow cytometer in the correlation diagram at the lower raw was produced by calculating the ratio of the number of Ki-67-positive nuclei to the number of all the cell nuclei in the scattergram without performing the gating.

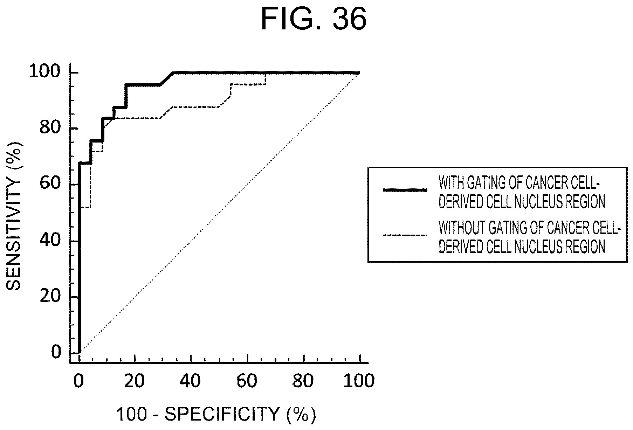

[Fig. 36] Fig. 36 shows the results of ROC analysis with 100 - specificity (%) as X and the sensitivity (%) as Y regarding scattergrams with the FSC-H (forward scatter) in the horizontal axis and the SSC-H (side scatter) in the vertical axis with and without gating of the cancer cell nucleus region.

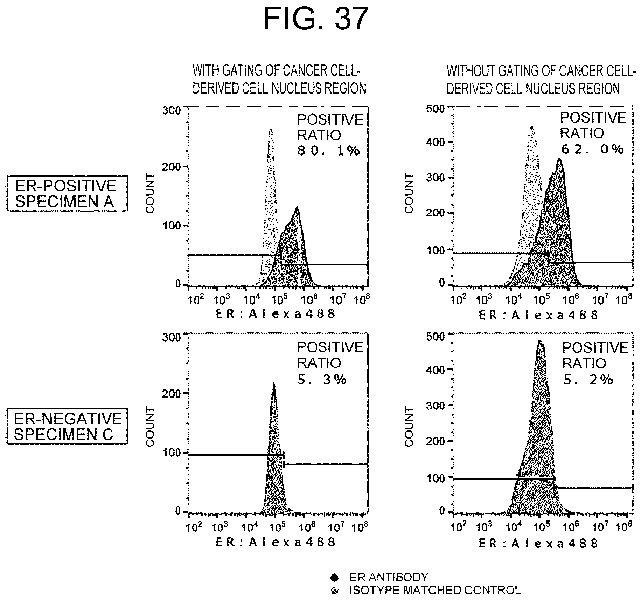

[Fig. 37] Fig. 37 shows histograms with the fluorescence intensity in the horizontal axis and the number of particles in the vertical axis, which were generated with or without gating of the cancer cell nucleus region after immunofluorescent staining with an anti-ER antibody. On each of the histograms, a histogram of an isotype matched control is superimposed. An ER-positive FFPE tissue section was deparaffinized, hydrophilized, heated, treated with thrombin, subjected to water-flow crushing and ultrasonic crushing, fluorescently stained with an anti-ER antibody, stained with DAPI, and then analyzed by a flow cytometer. The obtained data was used to generate a histogram with the fluorescence intensity of DAPI-A in the horizontal axis and the number of particles in the vertical axis to gate a main region of 2n or more nuclear chromosomes, and from the gated region a scattergram with the FSC-H (forward scatter) in the horizontal axis and the SSC-H (side scatter) in the vertical axis was developed to gate a cancer cell nucleus region. The aforementioned histograms were generated from the population of the gated cancer cell nucleus region (left) and the non-gated population (right). For each population, the ratio of the number of ER-positive nuclei to the number of all cell nuclei was calculated as an ER-positive ratio.

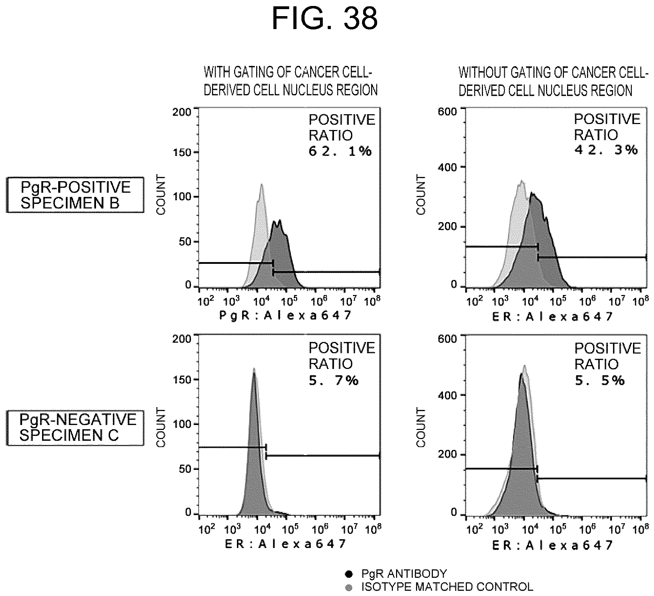

[Fig. 38] Fig. 38 shows histograms with the fluorescence intensity in the horizontal axis and the number of particles in the vertical axis, which were generated with or without gating of the cancer cell nucleus region after immunofluorescent staining with an anti-PgR antibody. On each of the histograms, a histogram of an isotype matched control is superimposed. A PgR-positive FFPE tissue section was deparaffinized, hydrophilized, heated, treated with thrombin, subjected to water-flow crushing and ultrasonic crushing, fluorescently stained with an anti-PgR antibody, stained with DAPI, and then analyzed by a flow cytometer. The obtained data was used to generate a histogram with the fluorescence intensity of DAPI-A in the horizontal axis and the number of particles in the vertical axis to gate a main region of 2n or more nuclear chromosomes, and from the gated region a scattergram with the FSC-H (forward scatter) in the horizontal axis and the SSC-H (side scatter) in the vertical axis was developed to gate a cancer cell nucleus region. The aforementioned histograms were generated from the population of the gated cancer cell nucleus region (left) and the non-gated population (right). For each population, the ratio of the number of PgR-positive nuclei to the number of all cell nuclei was calculated as an PgR-positive ratio.

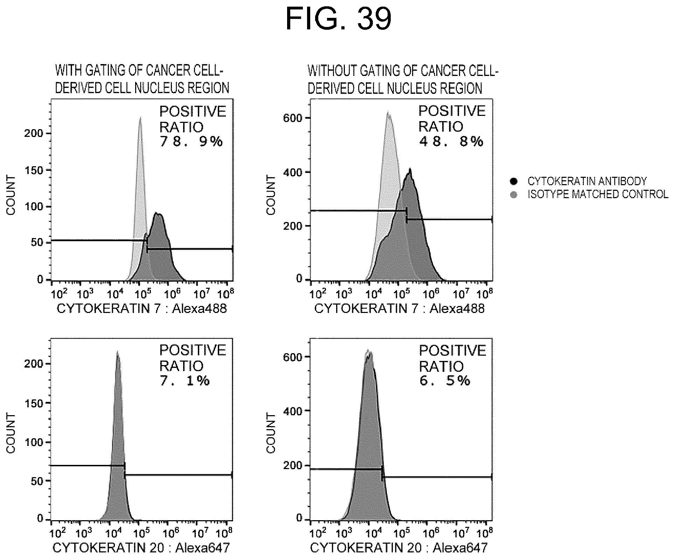

[Fig. 39] Fig. 39 shows histograms with the fluorescence intensity from each antibody in the horizontal axis and the number of particles in the vertical axis, which were generated with or without gating of the cancer cell nucleus region after dual immunofluorescent staining with an anti-cytokeratin 7 antibody and an anti-cytokeratin 20 antibody. On each of the histograms, a histogram of an isotype matched control is superimposed. A breast cancer FFPE tissue section was deparaffinized, hydrophilized, heated, treated with thrombin, subjected to water-flow crushing and ultrasonic crushing, fluorescently stained with an anti-cytokeratin 7 antibody and an anti-cytokeratin 20 antibody, and then analyzed by a flow cytometer. The obtained data was used to generate a histogram with the fluorescence intensity of DAPI-A in the horizontal axis and the number of particles in the vertical axis to gate a main region of 2n or more nuclear chromosomes, and from the gated region a scattergram with the FSC-H (forward scatter) in the horizontal axis and the SSC-H (side scatter) in the vertical axis was developed to gate a cancer cell nucleus region. The aforementioned histograms were generated from the population of the gated cancer cell nucleus region(left) and the non-gated population (right). For each population, the ratios of the numbers of cytokeratin 7-positive nuclei and cytokeratin 20-positive nuclei to the number of all cell nuclei were calculated as a cytokeratin 7-positive ratio and a cytokeratin 20-positive ratio, respectively.

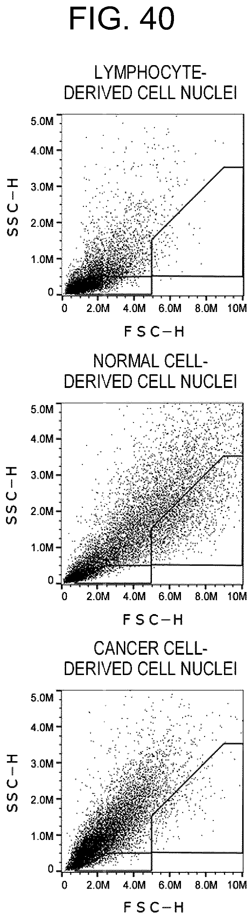

[Fig. 40] Fig. 40 shows scattergrams with the FSC-H (forward scatter) in the horizontal axis and the SSC-H (side scatter) in the vertical axis, which were generated by analyzing FFPE tissue sections of a cancer part, a non-cancerous part and a normal lymph node of one patient with breast cancer by a flow cytometer after predetermined pretreatment. FFPE tissue sections of a cancer part, a non-cancerous part and a normal lymph node of one patient with breast cancer were deparaffinized, hydrophilized, heated, treated with thrombin, strained with DAPI, and then analyzed by a flow cytometer. A region selected as a cell nuclei region was gated and scattergrams were generated from the gated region in the same procedure as in Test 1.

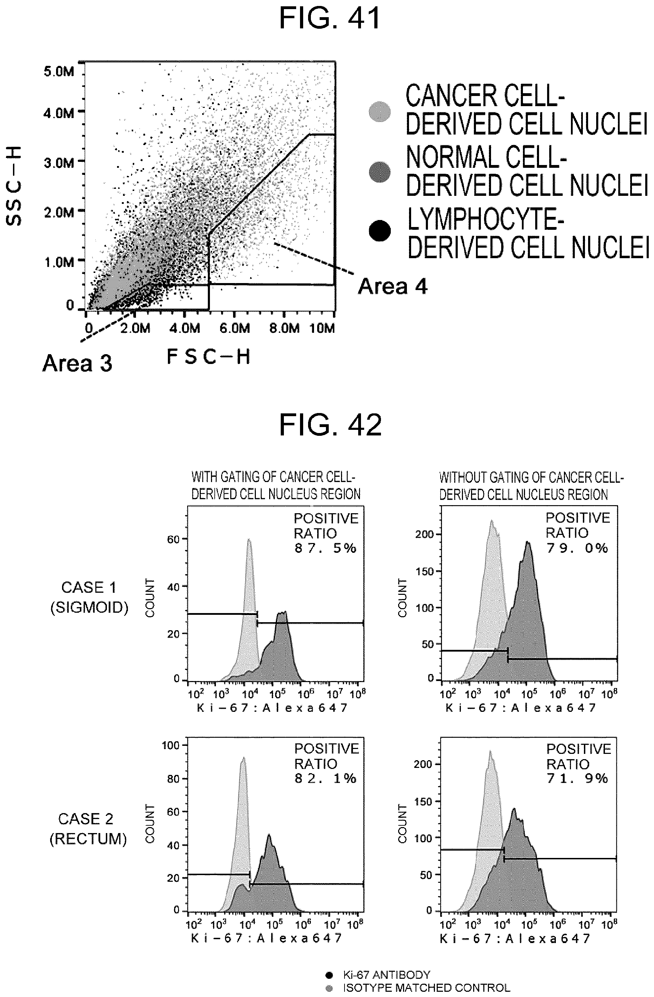

[Fig. 41] Fig. 41 shows a diagram obtained by overlaying the scattergrams of the different tissue sections set forth in Fig. 40.

[Fig. 42] Fig. 42 shows histograms with the fluorescence intensity in the horizontal axis and the number of particles in the vertical axis, which were generated with or without gating of the cancer cell nucleus region after immunofluorescent staining with an anti-Ki-67 antibody. On each of the histograms, a histogram of an isotype matched control is superimposed. FFPE tissue sections of cancer parts from patients with sigmoid colon and rectal cancer were deparaffinized, hydrophilized, heated, treated with thrombin, subjected to water-flow crushing and ultrasonic crushing, immuno-fluorescently stained with an anti-Ki-67 antibody and stained with DAPI, and then analyzed by a flow cytometer. The obtained data was used to generate a histogram with the fluorescence intensity of DAPI-A in the horizontal axis and the number of particles in the vertical axis to gate a main region of 2n or more nuclear chromosomes, and from the gated region a scattergram with the FSC-H (forward scatter) in the horizontal axis and the SSC-H (side scatter) in the vertical axis was developed to gate a cancer cell nucleus region. The aforementioned histograms were generated from a population of the gated cancer cell nucleus regions are gated (left) and the non-gated population (right). For each population, the ratio of the number of Ki-67-positive nuclei to the number of all cell nuclei was calculated as a Ki-67-positive ratio.

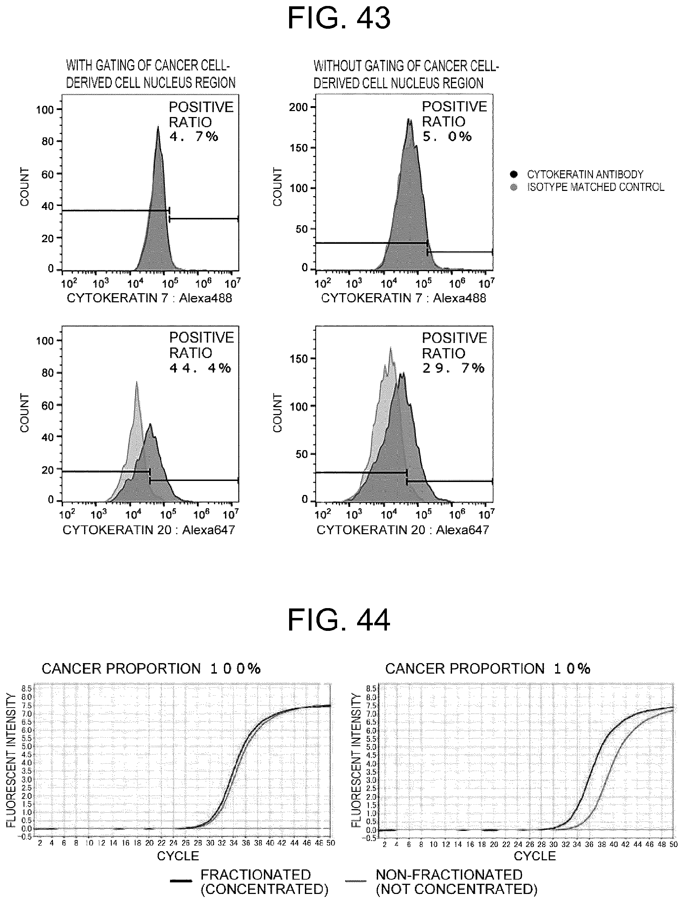

[Fig. 43] Fig. 43 shows histograms with the fluorescence intensity in the horizontal axis and the number of particles in the vertical axis, which were generated with or without gating of the cancer cell nucleus region after immunofluorescent staining of anti-cytokeratin 7 and 20 antibodies. On each of the histograms, a histogram of an isotype matched control is superimposed. A bowel cancer FFPE tissue section was deparaffinized, hydrophilized, heated, treated with thrombin, subjected to water-flow crushing and ultrasonic crushing, immuno-fluorescently stained with cytokeratin 7 and 20 antibodies, stained with DAPI, and then analyzed by a flow cytometer. The obtained data was used to generate a histogram with the fluorescence intensity of DAPI-A in the horizontal axis and the number of particles in the vertical axis to gate a main region of 2n or more nuclear chromosomes, and from the gated region a scattergram with the FSC-H (forward scatter) in the horizontal axis and the SSC-H (side scatter) in the vertical axis was developed to gate a cancer cell nucleus region. The aforementioned histograms were generated from the gated cancer cell nucleus region, and the non-gated population. For each population, the ratios of the numbers of cytokeratin 7- and 20-positive nuclei to the number of all cell nuclei are calculated as cytokeratin 7- and 20-positive ratios, respectively.

[Fig. 44] Fig. 44 is graphs showing difference in PCR amplification rate between cells fractionated from Area 1 and non-fractionated cells using samples containing cells from a cancer region in different proportions (10% and 100%).

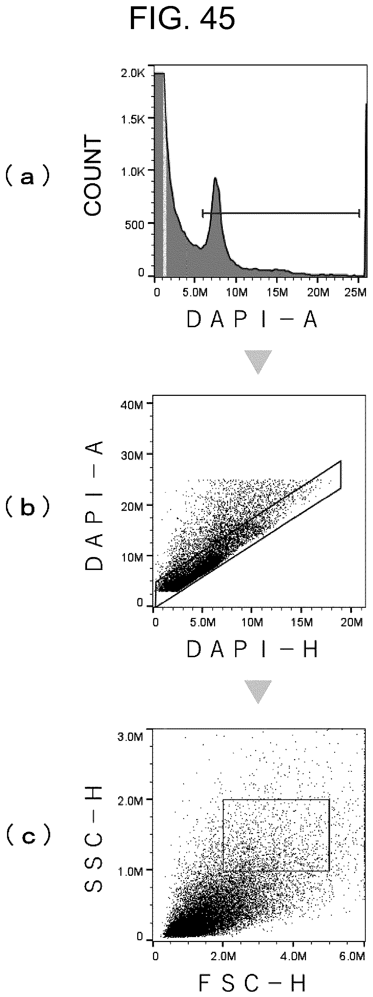

[Fig. 45] Fig. 45(a) shows a histogram with the fluorescent intensity of DAPI-A in the horizontal axis and the number of particles in the vertical axis. A breast cancer FFPE tissue section was deparaffinized, hydrophilized, heated, treated with thrombin, subjected to water-flow crushing and ultrasonic crushing, stained with DAPI, and then analyzed by a flow cytometer. The histogram was generated based on the obtained data. An area indicated by I--------I was considered to be a region of 2n or more nuclear chromosomes. This area was thus gated as being from cells. Fig. 45(b) shows a scattergram with DAPI-H (height) in the horizontal axis and DAPI-A (area) in the vertical axis, which was developed from the data of a population obtained by gating the area indicated by I--------I on Fig. 45(a). A framed area was selected as a main region of nuclei, and doublets were removed. Fig. 45(c) shows a scattergram with the FSC-H (forward scatter) in the horizontal axis and the SSC-H (side scatter) in the vertical axis, which was generated from the data of the population from the framed area in the scattergram of Fig. 45(b). Particles having a FSC-H intensity of $2.00 \times 10^6$ to $5.00 \times 10^6$ and a SSC-H intensity of $1.00 \times 10^6$ to $2.00 \times 10^6$ were gated.

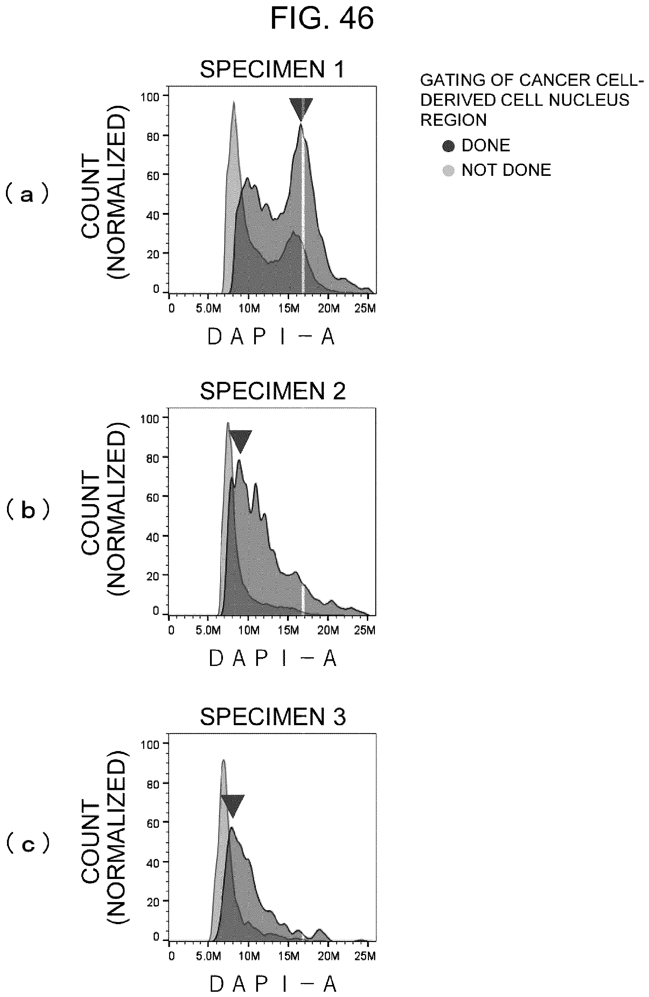

[Fig. 46] Fig. 46 shows histograms with the fluorescence intensity of DAPI-A in the horizontal axis and the number of particles in the vertical axis, where the number of particles is normalized so that the maximum number of particles is 100. The histograms cover three FFPE tissue specimens obtained from patients with breast cancer and were generated from populations gated as the cancer cell nucleus region set forth in Fig. 45(c) from the FFPE tissue sections and a non-gated population from the same. Histograms of Specimens 1 to 3 are shown in Figs. 46(a), 46(b) and 46(c), respectively.

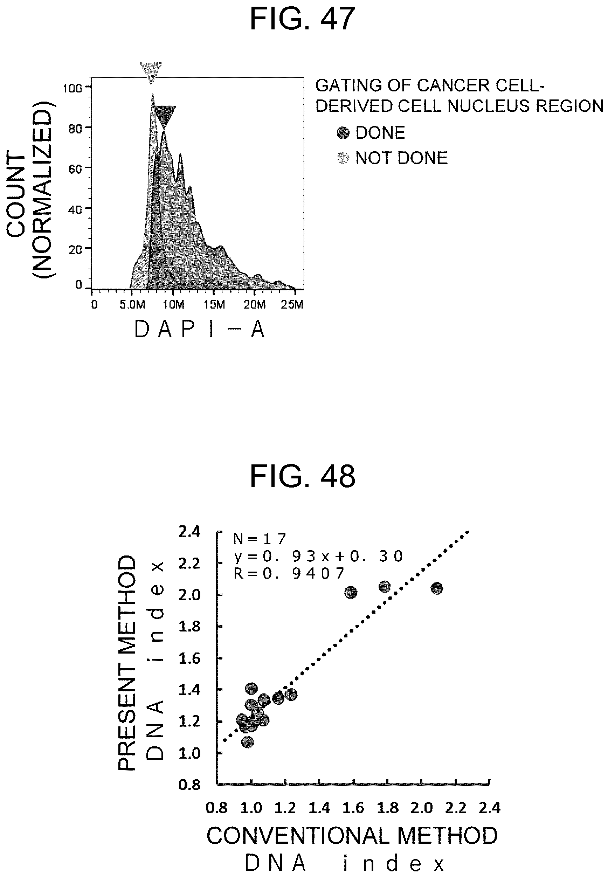

[Fig. 47] Fig. 47 exemplifies the fluorescence intensity of DAPI at the largest number of particles for populations gated as a cancer cell nucleus region and a non-gated population. The most frequent value for the non-gated population was regarded as a fluorescence intensity of 2n chromosomes , and the ratio of the fluorescence intensity at the most frequent value for the population gated as the cancer cell nuclei region to the fluorescence intensity at the most frequent value for the non-gated population was calculated as a DNA index.

[Fig. 48] Fig. 48 shows a correlation graph between a method for determining a DNA index without using biomarkers (present method) which was carried out in Test 28, and a conventional method for determining a DNA index using biomarkers (n = 17). The values from the present method are on the y-axis, and the values from the conventional method are on the x-axis.

## DESCRIPTION OF EMBODIMENTS

The present invention relates to a method for obtaining a sample enriched with cells or cell nuclei of interest, a method for analyzing biomarkers with the sample obtained by the above method, as well as a kit, a system and a program for use in implementing these methods. Embodiments thereof will be described in detail below, but the present invention is not limited to the embodiments described below.

### 1. Method for Preparing Sample Enriched with Cells or Cell Nuclei of Interest

In one embodiment, the present invention provides a method comprising subjecting a piece of tissue to particular pretreatment to obtain individually separated cells or cell nuclei, and sorting or fractionating a population of particles enriched with cells or cell nuclei of interest based on optical characteristics or physical characteristics of the cell nuclei.

The "piece of tissue" is usually obtained by biopsy, which involves removing a piece of tissue from a subject with a scalpel or needle. Examples of the biopsy include excision biopsy (entire removal), incision biopsy (partial removal), core biopsy (removal of a part of tissue with a large-bore needle) and fine needle aspiration (FNA) biopsy (removal of a part of tissue or a body fluid with a fine needle).

A subject may be a mammal or another animal species. The mammal is typically a human being but may be a non-human mammal. Tissue may be collected from any organs of a subject. Tissue is often collected from pathological sites, but in some cases, it is collected from normal sites, such as non-tumor tissue and lymph nodes.

The pathological sites typically include, but not being limited to, sites infiltrated with malignant tumor cells. Even when tissue is collected from a site infiltrated with malignant tumor cells, the tissue contains non-cancerous cells (e.g., normal cells, and lymphocytes). In particular, a collected tissue may contain a very small malignant tumor tissue, and therefore a large number of other cells such as normal cells. Thus, a collected tissue is usually not composed of only one type of cells.

Malignant tumors are tumors that infiltrate into surrounding tissues or metastasize, among cell populations (benign tumors and malignant tumors) that proliferate in an autonomous and uncontrollable manner due to genetic mutations. In pathology, the term "malignant tumor" is classified into:

1) carcinoma: malignant tumor derived from epithelial tissue,
2) sarcoma: malignant tumor derived from non-epithelial tissue, and
3) others: leukemia etc.

Examples of the carcinomas include head and neck cancers (upper jaw cancer, (upper, middle and lower) pharyngeal cancers, laryngeal cancer, tongue cancer and thyroid cancer), thoracic or chest cancers (breast cancer, lung cancers (non-small cell lung cancer and small cell lung cancer)), digestive organ cancers (esophagus cancer, stomach cancer, duodenal cancer, bowel cancers (colon cancer and rectal cancer), liver cancers (liver cell cancer and bile duct cell cancer), gallbladder cancer, bile duct cancer, pancreas cancer, anus cancer, urinary organ cancers (kidney cancer, ureter cancer, bladder cancer, prostate cancer, penis cancer and testicle (testis) cancer), genital cancers (uterus cancers (uterine cervical cancer and uterine body cancer), ovary cancer, vulva cancer and vaginal cancer), and skin cancers (basal cell cancer and squamous cell cancer). Examples of the sarcoma include osteosarcoma, chondrosarcoma, and Ewing's sarcoma.

The "piece of tissue" may be enzymatically treated by the method of the present invention immediately after collection but is usually cryopreserved or fixed to be suitable for preservation for a certain period of time.

The temperature for "cryopreservation " is usually freezing at -80°C or lower.

The "fixation" is a process of stabilizing cell morphology and tissue structure by immersing a sample in a fixing solution to cause molecular cross-linking and protein insolubilization. A known method may be used for the fixation, and examples of the method include treatments with a formalin solution, a para-formaldehyde solution, a glutaraldehyde solution, an osmium tetroxide solution, alcohol acetate, methanol, ethanol, acetone or the like. In particular, formalin fixation has been widely used as a method for fixing a pathological tissue, and use of a formalin-fixed tissue is therefore convenient in the method of the present invention,.

When a fixed tissue is used, the tissue is usually embedded in an embedding agent to impart a uniform hardness to a piece of tissue (mass), and fill hollow parts in the tissue, thereby imparting a strength suitable for slicing, as well as enhance preservability. Examples of the embedding agent include, but not being limited to, paraffin, paraffin derivatives, celloidin, carbowax, agarose, non-heparin-treated serum, collagen, cellulose derivatives, chitin derivatives, chitosan derivatives and mixtures thereof. In particular, paraffin has been widely used, and formalin-fixed paraffin-embedded sections (FFPE sections) are tissue sections which have been most commonly used in pathological diagnosis. In the present invention, use of such a tissue section is convenient, accordingly.

In the method of the present invention, a piece of tissue may be preferably sliced or finely cut for efficiently obtaining a single cell or cell nucleus separated from other cells with pretreatment described later,. For example, in the case of slicing, a frozen tissue or an embedded tissue can be sliced to an appropriate thickness with a microtome. A section with a thickness of 60 μm or less is convenient because pretreatment can be efficiently performed, and the section can be used for detection of various markers by the immunohistochemistry (IHC) technique. As for the thickness of tissue sections, 60 μm or less is convenient for efficient pretreatment and for use of sections in detection of various markers by immunohistochemistry (IHC), and 20 μm or less is more preferable. The number and the thickness of tissue sections may be adjusted dependent on the size of a tissue to be sliced, and the number of cells or cell nuclei required for marker analysis.

When the method of the present invention is carried out with a tissue embedded in an embedding agent, it is preferable that the embedding agent (e.g., paraffin) be removed from the embedded tissue preferably after slicing or finely cutting (de-embedding), and that the organic solvent used for the removal of the embedding agent be replaced by an aqueous solvent (hydrophilization). The embedding agent can be removed by immersing the section in an organic solvent, such as xylene. The de-embedded tissue section can also be hydrophilized by immersing the tissue section in multiple concentrations of ethanol solutions with a concentration gradient in order from higher to lower concentrations. Examples of the ethanol concentration gradient include, but not being limited to, 100%-95%-90%-70%-50%. For frozen sections which are not embedded, de-embedding and hydrophilization are not required.

When a fixed tissue is used, it is preferable to perform heat treatment to destroy a crosslinked structure formed by fixation. In the fixed tissue, a crosslinked structure is formed between peptides, which makes it difficult to access a biomarker of interest and also make it difficult for an enzyme to act on a targeted component during enzymatic treatment. The heat treatment cleaves the formed crosslinked structured, facilitating access to a biomarker and enabling efficient degradation of the tissue by enzymatic treatment.

Heat treatment is preferably performed by immersing the tissue in a solution containing a citric acid buffer solution, a surfactant, a chelating agent and/or a reducing agent. A solution containing such components is not particularly limited but includes Histo VT One (nacalai tesque), Antigen Activation Solution pH 9 (Nichirei Bioscience Inc.) and ImmunoSaver (Nisshin EM Co., Ltd.) which are commercially available as antigen retrieval agents with heating. The treatment may be performed according to the accompanying instructions, but the heating temperature may be 90°C to 100°C, and the treatment time may be 20 to 60 minutes.

In a preferred embodiment of the present invention, tissue is treated with a hydrolase which does not degrade a protein necessary to retain cell nuclear morphology prior to water flow crushing and/or ultrasonic crushing described below.

As used herein, the terms "cell nuclear morphology" and "cell nuclei morphology" relate to a size, a shape and a structure of a cell nuclear or cell nuclei. The wording "protein necessary for maintaining cell nuclear (nuclei) morphology" means a protein the degradation of which changes cell nuclear (nuclei) morphology, and examples thereof include lamins such as lamin A, lamin B1, lamin B2 and lamin C. Whether cell nuclear morphology has been changed or not can be determined from optical characteristics or physical characteristics of cell nuclei described later. Preferably, enzymes for use in the present invention do not degrade such proteins.

Hydrolases with such properties can be found from known databases (e.g., http://web.expasy.org/peptide_cutter/). Whether an enzyme has such properties or not can be confirmed by treating a "protein necessary for maintaining cell nuclear morphology" with the enzyme, and then determining whether the protein is cleaved with a known method, such as agarose gel electrophoresis.

Examples of the hydrolase "which does not degrade a protein necessary for maintaining cell nuclear morphology" include thrombin, proline endopeptidase, enterokinase, granzyme B, and hyaluronidase. On the other hand, hydrolases that affect cell nuclear morphology, such as pepsin, trypsin, collagenase and dispase, are not used.

Of the aforementioned enzymes, thrombin, proline endopeptidase and hyaluronidase are preferable, and thrombin and hyaluronidase are more preferable.

The "enzymatic treatment" can be performed by, for example, dissolving an enzyme in a buffer solution adjusted to an appropriated pH, immersing a piece of tissue in the solution, stirring the mixture for a predetermined time at a temperature suitable for an enzyme reaction to allow a hydrolytic reaction to occur, and then quenching the reaction as necessary.

As the buffer solution, a tris(hydroxymethyl)aminomethane buffer solution, a phosphate buffer solution, a carbonate buffer solution, a glycine buffer solution, an acetate buffer solution, a tartrate buffer solution, a citrate buffer solution, a triethanol amine buffer solution, a borate buffer solution, and a Good buffer solution may be used. It is preferable to use a buffer solution to which a surfactant is added because a surfactant promotes disruption of cell membranes. The surfactant is not particularly limited as long as it has no effect on cell nuclei, and examples thereof include anionic surfactants (carboxylic acid type, sulfonic acid type, sulfuric acid ester type, phosphoric acid ester type and the like), cationic surfactants (quaternary ammonium salt type, alkylamine salt type, pyridine ring-containing type and the like), ampholytic surfactants (betaine type, sulfobetaine type, amine oxide type, alkylimidazole type, amino acid type and the like), and nonionic surfactants (ester type, ether type, ester ether type, alkanolamide type, alkyl glycoside type and the like). The surfactant may be preferably a nonionic surfactant, which includes TritonX-100, TritonX-405, NP-40, Briji-35, Briji-58, Tween-20, Tween-80, BPSH-25, Octyl Glucoside and Octylthio Glucoside. The pH may be adjusted dependent on an enzyme used, and an optimum pH for each enzyme is well known in the art. The temperature may also be determined dependent on an enzyme used. In many cases, the temperature is in the range of 20°C to 40°C. The reaction time may be appropriately determined dependent on the size of a tissue and the like. The reaction may be quenched by removing reagents, changing the temperature, adding a chelator, or the like, and specific procedures are well known in the art.

In the method of the present invention, tissue or an enzymatically treated product is subjected to water-flow crushing and/or ultrasonic crushing instead of the enzymatic treatment or after the enzymatic treatment. In particular, from the viewpoint of obtaining more single cells from the tissue while maintaining the morphology of the cell nuclei, "crushing with a water flow" is preferable, and it is particularly preferable to perform "crushing with a water flow" and "crushing by ultrasonication" in this order.

The term "crushing with a water flow" or "water-flow crushing" means that a tissue or, in some case, cells are crushed with a shear force from a water flow. The condition for "water-flow crushing" is that tissue can be broken down to the level of a single cell, while the morphology of the cell nucleus is not affected. In this respect, the conditions in which a rotary blade rotates at 6,000 rpm to 13,000 rpm are preferable, and the conditions in which a rotary blade rotates at 7,000 rpm to 13,000 rpm are more preferable. The treatment time may be usually 30 seconds to 3 minutes, but preferably 45 seconds to 2 minutes, and particularly preferably 1 minute. When using a water-flow crushing apparatus, the clearance between an inner blade and an outer blade may be usually 0.01 mm to 1.00 mm, but is preferably 0.02 mm to 0.50 mm, more preferably 0.03 mm to 0.30 mm.

For water-flow crushing, commercially available apparatuses may be used, such as a water flow shear apparatus (RP-10) from Sysmex Corporation, and a homogenizer (T10) from IKA. Preferably, water-flow crushing is performed in an ice-cooled environment.

The term "crushing with ultrasonication" or "ultrasonic crushing" means that a tissue or, in some case, cells are crushed with a shear force from ultrasonication. The conditions for "ultrasonic crushing" are also that tissue can be broken down to the level of a single cell, while the morphology of the cell nucleus is not affected. In this respect, the output intensity is preferably 20 to 40% in terms of an amplitude, more preferably 20 to 30% in terms of an amplitude, particularly preferably 20% in terms of an amplitude.

From the same viewpoint, the treatment time may be usually 30 seconds to 3 minutes, but preferably 45 seconds to 2 minutes, and particularly preferably 1 minute.

For ultrasonic crushing, commercially available apparatuses may be used, such as an ultrasonic crushing apparatus (VCX130PB) from SONICS&MATERIALS. Preferably, ultrasonic crushing is performed in an ice-cooled environment.

Accordingly, a preferred embodiment of the present invention provides a process comprising the following steps:

1) deparaffinizing and hydrophilizing a FFPE section,
2) destroying a crosslinked structure by heat treatment,
3) treatment with an enzyme, and
4) crushing a tissue (and cells) with a water flow and/or ultrasonication.

As described above, the method of the present invention provides individually separated cells or cell nuclei from tissue by shear stress of water-flow crushing and/or ultrasonic crushing, optionally following heat treatment and enzymatic treatment.

As used herein, the term "individually separated cells or cell nuclei" refers to single cells or cell nuclei present in isolation from other cells. As used in the context of "cells or cell nuclei" after enzymatic treatment, the term "cells or cell nuclei" includes not only intact cells and cell nuclei with no other cellular structures, but also cells (particles) of which part of cellular structures are destroyed, cleaved or lost as long as the cell nuclei morphology is maintained. For example, cells (particles) in which the cell membrane is destroyed or lost, but the cytoskeleton or all or part of proteins constituting the cytoskeleton is maintained, also fall within the term "cells or cell nuclei".

In the method of the present invention, a treated product including "individually separated cells or cell nuclei" obtained through the aforementioned pretreatment is used to sort or fractionate a population of particles significantly abundant in cells or cell nuclei of interest.

The term "cells or cell nuclei of interest" means cells or cell nuclei intended to be enriched or concentrated by the method of the present invention. The term "significantly abundant" means that the proportion of cells or cell nuclei of interest is the highest among all cells or cell nuclei and other particulate substances.

In the method of the present invention, as described above, a population of particles including the "cells or cell nuclei of interest " is obtained without affecting the morphology of the cell nuclei through the treatment of obtaining single level cells or cell nuclei from tissue. In the present invention, therefore, optical characteristics characteristics reflecting the maintained morphology of cell nuclei, i.e., size, shape and/or structure, including density, specific gravity, particle size and staining, are measured, and "a population of particles significantly abundant in cells or cell nuclei of interest is sorted or fractionated" based on a difference in the above characteristics between "cells or cell nuclei of interest" and the other particles. Therefore, in an embodiment of the method of the present invention, "cells or cell nuclei of interest" are concentrated without using biomarkers specifically expressed in a particular cell type.

It should be noted that as demonstrated in Examples below, the aforementioned pretreatment employed in the present invention makes it possible to "sort or fractionate a population of particles significantly abundant in cells or cell nuclei of interest" using optical characteristics.

In an embodiment involving analysis of optical characteristics, a treated product after the pretreatment is optically analyzed, after components in cell nuclei, typically nucleic acids, are visualized, for example, by staining, and a population of particles significantly abundant in cells or cell nuclei of interest is sorted or fractionated based on a difference in optical characteristics between the cell nuclei of interest and the other particles.

The optical analysis is performed using flow cytometry.

For analysis by flow cytometry, for example, a treated product after pretreatment is subjected to fluorescent staining of cell nuclei if necessary, and then subjected to flow cytometry, and based on the obtained forward scatter light (FSC) intensity, side scatter light (SSC) intensity and/or fluorescent intensity, a population of particles significantly abundant in "cells or cell nuclei of interest" is sorted from other cells or cell nuclei or other particles by gating. The forward scatter light (FSC) intensity is an index of the size of particles. The side scatter light (SSC) intensity is an index of the granularity and complexity of cell nuclei, where cell nuclei with high granularity like tumor cells exhibit a higher scatter light intensity. The "sorted" population of particles can be " fractionated " to a given chamber.

As used herein, the term "sort" means that data obtained by analysis is processed to select a population of particles in a specific area, but the population of particles is not required to be physically separated from other populations of particles. On the other hand, the term "fractionate" means that a population of particles including "cells or cell nuclei of interest" are obtained in a state of being physically separated from other populations of particles. The term "obtain a sample enriched with cells or cell nuclei of interest" means that a sample enriched with cells or cell nuclei of interest is obtained on data or physically by any of "sorting" and "fractionation". The term "enriched" means that the ratio of the number of cells or cell nuclei of interest with respect to the number of all cells increases as compared to that before treatment. The term "concentration", as used herein, has the same meaning as "enrichment", and the terms "enrichment" and "concentration" are interchangeably used.

When "a population of particles significantly abundant in cells and cell nuclei of interest" is selected based on the forward scatter (FSC) intensity and/or the side scatter (SSC) intensity, the cells or cell nuclei of interest can be sorted or fractionated by, for example, the following procedure.

A plurality of known cell types are analyzed by flow cytometry to generate a histogram of forward scatter (FSC) intensity or side scatter (SSC) intensity and the number of particles or a scattergram of forward scatter (FSC) intensity and side scatter (SSC) intensity for cell nuclei derived from each cell type, and an area or region more abundant in nuclei of each cell type than in nuclei of other cell types is determined based on the forward scatter (FSC) intensity and/or side scatter (SSC) intensity; and

a treated product after the pretreatment is then measured by flow cytometry to generate a histogram or scattergram in the same manner, and in the histogram or scattergram, a population of particles present in the area or region pre-determined to be more abundant in cell nuclei identical in type to the cell nuclei of interest is sorted or fractionated.

When "a population of particles significantly abundant in cells or cell nuclei of interest" is sorted based on the forward scatter (FSC) intensity, a correlation between the forward scatter (FSC) intensity and particle sizes is determined in advance, the particle size is calculated from the forward scatter (FSC) intensity obtained by analyzing a treated product after pretreatment by flow cytometry, and a population of particles with particle sizes in a particular range specific for each cell type are sorted or fractionated.

For example, standard particles having different known particle sizes are analyzed by flow cytometry, and a calibration curve is produced from the obtained forward scatter (FSC) intensity and the particle sizes of the standard substances. The treated product after the pretreatment is measured by flow cytometry, and the obtained forward scatter (FSC) intensity is applied to the calibration curve to calculate a particle size.

The calculated particle sizes are used to sort or fractionate, for example, a population of particles significantly abundant in particles having particle sizes of 10 μm or more, 95% or more of which have particle sizes of 11 μm to 20 μm to obtain a population of particles enriched with tumor cells or cell nuclei thereof.

Similarly, for example, a population of particles significantly abundant in particles having particle sizes of 8 μm or less, 95% or more of which have particle sizes of 4 μm to 7 μm, is selected to obtain a population of particles enriched with lymphocytes or cell nuclei thereof.

When "a population of particles significantly abundant in cells or cell nuclei of interest" is sorted by flow cytometry, the population of particles is sorted so that the "cells or cell nuclei of interest" account for preferably 60% or more, more preferably 70% or more, further more preferably 80% or more, even more preferably 90% or more, particularly preferably 95% or more, of the total particles.

When image cytometry is applied, a suspension liquid of the pretreated particles is subjected to fluorescent staining of cell nuclei, then placed on a multi-well plate or slide glass, and laser-scanned to obtain a fluorescent image; and cell nucleus morphology (e.g., size, perimeter, and circularity) is analyzed to sort or fractionate "cells or cell nuclei of interest" from other cells or cell nuclei based on the results of the analysis,.

Alternatively, polyacrylamide electrophoresis, a filtration method, an immunoprecipitation method, a centrifugal separation method or the like can be used to fractionate "cells or cell nuclei of interest" based on a difference in particle size or specific gravity (density).

Thus, the "optical characteristics or physical characteristics of cell nuclei" can vary depending on a methodology for use in sorting or fractionation. The "optical characteristics of cell nuclei" include fluorescent intensity, visible light intensity, ultraviolet intensity, absorbance, scatter light intensity and the like, and the "physical characteristics of cell nuclei" include size, specific gravity (density) and the like. However, various characteristics may be used dependent on a methodology to be selected, and other characteristics may be utilized as long as they reflect cell nucleus morphology .

In this way, the method of the present invention enables sorting or fractionation of a population of particles more abundant in "cells or cell nuclei thereof of interest" than in other cells or cell nuclei thereof or other particles. For example, as demonstrated in Examples below, a sample enriched with tumor cells or cell nuclei thereof can be obtained by sorting or fractionating tumor cells or cell nuclei thereof, based on optical characteristics or physical characteristics reflecting the morphology of the cell nuclei, from other cells such as lymphocytes and normal cells derived from a non-cancerous part of tissue or cell nuclei thereof. Also, in case of obtaining a sample enriched with lymphocytes, lymphocytes or cell nuclei thereof are sorted or fractionated, based on optical or physical characteristics reflecting the morphology of the cell nuclei, from other cells such as tumor cells and normal cells derived from a non-cancerous part of tissue or cell nuclei thereof. A sample enriched with normal cells derived from a non-cancerous part of tissue can be obtained in a similar manner.

2. Method for Examining Biomarker in Cells or cell Nuclei

In another embodiment, the present invention provides a method for analyzing biomarkers present in cells or cell nuclei of interest using a sample enriched with the cells or cell nuclei obtained by the aforementioned method.

A sample obtained by the aforementioned method is enriched with cells or cell nuclei of interest, and biomarkers present in the cells or cell nuclei are contained at a high concentration in the sample. Therefore, use of the sample in examination for the biomarkers enables improvement of the detection and positive rates. As demonstrated in Examples below an improved correlation with a conventional IHC method is also expected. Even when it is necessary to examine a biomarker in a piece of tissue with few cells or cell nuclei, a sample enriched with a targeted marker can be prepared from a collected tissue to facilitate detecting the targeted marker.

Accordingly, the method for examination according to the present invention is not particularly limited in terms of biomarkers or samples, any tissue can be used as examination samples, and any biomarkers in cells or cell nuclei can be analyzed. Examples of the biomarker include, but not being limited to, polynucleotides, nuclear proteins, nuclear membrane proteins, and intracellular proteins.

Examples of the polynucleotide include genomic DNA, cDNA, and mRNA. In particular, an extremely large number of various disease-related genes have been identified in recent years, and can be subject to the method for examination according to the present invention. Examples of the disease-related gene include cancer-related genes such as ABL1, ACVR1B, AKT1, AKT2, AKT3, ALK, ALOX12B, AMER1, APC, AR, ARAF, ARFFP1, ARD1A, ASXL1, ATM, ATR, ATRX, AURKA, AUFKB, AXIN1, AXL, BAP1, BARD1, BCL2, BCL2L1, BCL2L2, BCL6, BCOR, BCORL1, BRAF, BRCA1, BRCA2, BRD4, BRIP1, BTG1, BTG2, BTX, C11orf30, CALR, CARD11, CASP8, CBFB, CBL, CCND1, CCND2, CCND3, CCNE1, CD22, CD274, CD70, CD79A, CD79B, CDC73, CDH1, CDK12, CDK4, CDK6, CDK8, CDKN1A, CDKN1B, CDKN2A, CDKN2B, CDKN2C, CEBPA, CHEK1, CHEK2, CIC, CREBBP, CRKL, CSF1R, CSF3R, CTCF, CTNNA1, CTNNB1, CUL3, CUL4A, CXCR4, CYP17A1, DAXX, DDR1, DDR2, DIS3, DNMT3A, DOT1L, EED, EGFR, EP300, EPHA3, EPHB1, EPHB4, ERBB2(HER2), ERBB3, ERBB4, ERCC4, ERG, ERRFI1, ESR1, EZH2, FAM46C, FANCA, FANCC, FANCG, FANCL, FS, FBXW7, FGF10, FGF12, FGF14, FGF19, FGF23, FGF3, FGF4, FGF6, FGFR1, FGFR2, FGFR3, FGFR4, FH, FLCN, FLT1, FLT3, FOXL2, FUPB1, GABRA6, GATA3, GATA4, GATA6, GID4(C17orf39), GNA11, GNA13, GNAQ, GNAS, GRM3, GSK3B, H3F3A, HDAC1, HGF, HNF1A, HRAS, HSD3B1, ID3, IDH1, IDH2, IGF1R, IKBKE, IKZF1, INPP4B, IRF2, IRF4, IRS2, JAK1, JAK2, JAK3, JUN, KDM5A, KDM5C, KDM6A, KDR, KEAP1, KEL, KIT, KIHL6, KMT2A(MLL)KMT2D(MLL2), KRAS, LTK, LYN, MAF, MAP2K1, MAP2K2, MAP2K4, MAP3K1, MAP3K13, MAPK1, MCL1, MDM2, MDM4, MED12, MEF2B, MEN1, MERTK, MET, MITF, MKNK1MLH1, MPL, MRE11A, MSH2, MSH3, MSH6, MAT1R, MAp, MTOR, MUTYH, MYC, MYCL, MYCN, MYD88, NBN, NF1, NF2, NFE2L2, NFKBIA, NKX2-1, NOTCH1, NOTCH2, NOTCH3, NPM1, NRAS, NT5C2, NTRK1, NTRK2, NTRK3, P2RY8, PALB2, PARK2, PARP1, PARP2, PARP3, PAX5, PBRM1, PDCD1, PDCD1L G2, PDGFRA, PDGFRB, PDK1, PIK3C2B, PIK3C2G, PIK3CA, PIK3CB, PIK3R1, PIM1, PMS2, POLD1, POLE, PPARG, PPP2R1A, PPP2R2A, PPDM1, PRKAR1A, PRKCI, PTCH1, PTEN, PTPN11, PTPRO, qKI, RAC1, RAD21, RAD51, RAD51B, RAD51C, RAD51D, RAD52, RAD54L, RAF1, RARA, RB1, RBM10, REl, RET, RICTOR, RNF43, ROS1, RPTOR, SDHB, SDHC, SDHD, SETD2, SF3B1, SGK1, SMAD2, SMAD4, SMARC A4, SMARC B1, SMO, SNCAIP, SOCS1, SOX2, SOX9, SPEN, SPOP, SRC, STAG2, STAT3, STK11, SUFU, SYK, TBX3, TEK, TET2, TGFBR2, TIPARP, TNFAIP3, TNFRSF14, TP53, TSC1, TSC2, TYRO3, U2AF1, VEGFA, VHL, WHSC1, WHSC1L1, WT1, XPO1, WRCC2, ZNF217, and ZNF703.

Examples of the nuclear protein include transcription factors, and cell cycle-related proteins.

Examples of the transcription factors include, but not being limited to, homeodomains, bHLH, bZIP, Forkhead, nuclear reptors, HMG/Sox, Ets, T-Box, AT hook, POU, Myb/SANT, THAP finger, CENPB, E2F, BED ZF, GATA, Rel, CxxC, IRF, SAND, SMAD, HSF, MBD, RFX, CUT, DM, STAT, ARID/BRIGHT, Grainylhead, MADS box, Ap-2, CSD, and PAX. Examples of the cell cycle-related proteins include, but not being limited to, Ki-67, CDK (CDK1 to CDK20 are known as subtypes), Cyclin A, Cyclin B, WEE1, MYT1, CDC25B, CDC25C, ATM, ATR, CHK1, CHK2, MRE11, RAD50, NBS1, ATRIP, RAD9, RAD1, HUS1, MDC1, TopBP1, Claspin, Timeless, Tipin, Plk1-4, Aurora A/B, CDC20, CCH1, MAD1, MAD2, BUBR1, BUB1, BUB3, MST1/2, LATS1/2, MOB1A,B, and CDC14.

Examples of the nuclear membrane proteins include, but not being limited to, estrogen receptors, progesterone receptors.

Examples of the intracellular protein include, but not being limited to, factors involved in signal transduction, and cytoplasmic skeletal proteins. Examples of the factor involved in signaling include, but not being limited to, PTEN, PI3K, Akt, SRC, Ras, Raf, ERK, MEK, Myc, mTORC1/2, S6K, JAK, STAT, p53, SMAD, and JNK. Examples of the cytoplasmic skeletal proteins include, but not being limited to, cytokeratin, and vimentin.

Biomarkers may be measured by known methods applicable to individual biomarkers. However, in the case where cells or cell nuclei of interest have been sorted by an optical analysis method such as cytometry, biomarkers of interest may be labeled after the pretreatment in advance, then a population significantly abundant in cells or cell nuclei of interest is sorted by the aforementioned method, and the sorted population is continuously analyzed for the labeled biomarkers using the same analyzer.

For example, after the pretreatment, a biomarker of interest in the pretreated product can be directly labeled with a labeling substance. Alternatively, a ligand that specifically binds to a biomarker of interest, such as an antibody or a nucleic acid probe, or a secondary ligand that specifically binds to the above ligand is labeled with a labeling substance, the first ligand is allowed to be bound to the biomarker of interest in the pretreated product, optionally the secondary ligand is bound to the first one, and/or the cell nuclei are stained. The treated product is analyzed by cytometry, a population significantly abundant in cells or cell nuclei of interest is sorted, and the sorted population is analyzed for the labeled biomarkers.

Examples of the labeling substance include avidin-biotin complexes (ABC), fluorescent compounds, enzymes, and chemiluminescent substances.

The fluorescent compounds are not particularly limited, but examples thereof include cyanine-based dyes such as Cy3, fluorescein isothiocyanate (FITC), allophycocyanin and rhodamine. When a plurality of biomarkers are analyzed, ligands (antibodies or secondary antibodies thereto, nucleic acid probes which hybridize with targeted polynucleotides, and the like) can be labeled with a plurality of fluorescent substances which emit light with different fluorescence wavelengths. For this purpose, fluorescent substances such as Alexa Fluor (registered trademark) series can be used.

The labeling enzymes are not particularly limited, but examples thereof include alkali phosphatase and horseradish peroxidase.

The chemiluminescent substances are not particularly limited, but examples thereof include luminol, AMPPD (registered trademark), CSPD (registered trademark) and CDP-Star (registered trademark).

Examples of compounds for staining cell nuclei include fluorescent substances such as DAPI and propidium iodide (PI).

In one embodiment, when a population of cells in a tissue section may have a plurality of targeted biomarkers,

1) in sample obtained from a single (FFPE) tissue section, ligands for respective biomarkers may be labeled with labels which enable the ligands to be discriminated from each other such as fluorescent substances with different fluorescence wavelengths, and the biomarkers are measured (detection, quantitative determination or analysis); or
2) a sample may be obtained from each of a plurality of (FFPE) tissue sections, which are supposed to be homogeneous from the same tissue block, one targeted biomarker is labeled (e.g., immuno-fluorescent staining, FISH) per sample, and the different biomarkers in the individual samples are measured (detection, quantitative determination or analysis).

After a sample containing cells or cell nuclei of interest is fractionated, alternatively, an organelle (e.g., nucleic acid) having a targeted biomarker may be extracted by a known method from the fractionated sample and used in analysis. Even in this process, the fractionated sample is enriched with targeted biomarkers, which may contribute to an improved detection rate and the like.

One embodiment of the present invention provides a method for analyzing DNA aneuploidy with a sample obtained by the aforementioned method for obtaining a sample enriched with tumor cells or cell nuclei thereof without using biomarkers (specific to cell types).

Herein, the term "DNA aneuploidy" means abnormal cells different in amount of DNA in cell nuclei from normal cells, and the DNA aneuploidy can be evaluated by a DNA index expressed as a numerical value obtained by dividing the amount of DNA (indicated by, for example, amount of fluorescence of DAPI-A) at the most population of cells (usually, a population of G0/1 cells) in a specimen by the amount of DNA at the most population of G0/1 cells in normal diploid cells.

Specifically, the aforementioned method, preferably the method comprising the step of fractionating or sorting a population of particles significantly abundant in tumor cells or cell nuclei thereof from a tissue by flow cytometry provides a sample enriched with tumor cells or cell nuclei thereof (tumor cell nucleus-enriched sample); whereas a sample which is not enriched with tumor cells or cell nuclei thereof (tumor cell nuclei-non-enriched sample or normal cell sample) is obtained from the same tissue or a normal tissue (e.g., if the proportion of tumor cells is high) in the same manner except that fractionation or sorting of a population of particles significantly abundant in tumor cells or cell nuclei thereof is not performed. A histogram of the number of particles and the amount of DNA (indicated by, for example, amount of fluorescence of DAPI-A) is generated for each of the samples, the amount of DNA at the largest number of particles is identified, and the DNA index is determined by the following equation, whereby the DNA aneuploidy can be evaluated:

DNA index = amount of DNA at largest number of particles in tumor cell nucleus- enriched sample / amount of DNA at largest number of particles in tumor cell nucleus-non-enriched sample or normal cell sample.

Conventionally, for example, a scattergram with the fluorescence intensity of cytokeratin in the horizontal axis and the fluorescence intensity of vimentin in the vertical axis is developed, a cytokeratin-positive and vimentin-negative region is gated as a cancer cell region, a cytokeratin-negative and vimentin-positive region is gated as an interstitial cell region, the most frequent value of DAPI-A fluorescence is determined for each of the gated populations, and the ratio of the most frequent value of the cancer cell region to the most frequent value of the interstitial cell region is calculated to determine a DNA index (Non-Patent Literature 6). However, according to the aforementioned embodiment of the present invention, the DNA index can be determined without relying on such a biomarker. This provides an advantage that it can be applied to a case in which a biomarker is deficient (e.g., cytokeratin-negative breast cancer) and a population of cancer cells that have undergone epithelial-mesenchymal transition, whereby vimentin is positive. As described later, the method according to the embodiment of the present invention has been confirmed to have a high correlation with a conventional method for determining the DNA index with a biomarker.

3. Kit

In an example not part of the present invention a kit is provided for preparing a sample containing concentrated cells of interest or cell nuclei thereof from a piece of tissue. The kit comprises a hydrolase which does not degrade a protein necessary for retaining or maintaining cell nuclear morphology but can degrade at least one of components of the extracellular matrix or cell membrane proteins involved in intercellular adhesion.

The hydrolase in the kit is the same as described for the method for preparing a sample enriched with cells or cell nuclei thereof of interest from a piece of tissue, and examples thereof include thrombin, proline endopeptidase and hyaluronidase, with thrombin and/or hyaluronidase being particularly preferable.

The hydrolase may be in a dried solid state, or in a state of being dissolved in a buffer solution. When the hydrolase is in a solid state, the kit may contain a solution used to dissolve the enzyme.

To facilitate crushing of the tissue with a shear force, the kit may contain a buffer solution for immersing or dispersing an enzyme-treated product during crushing. Preferably, the buffer solution contains a surfactant. As the surfactant, CHAPS, NP-40 and Triton-X100 are preferable, and NP-40 and Triton-X100 are more preferable.

When fixed tissue is used, the kit may contain a solution for heating treatment. The solution for heating treatment is preferably a solution containing a citrate buffer solution, a surfactant, a chelating agent, and/or a reducing agent.

When embedded tissue is used, the kit may contain a de-embedding agent and a hydrophilizing agent. Examples of the de-embedding agent include organic solvents such as xylene, and examples of the hydrophilizing agent include ethanol solutions with such different concentrations as to generate a concentration gradient.

In another example not part of the present invention a kit is provided for preparing a sample enriched with cells or cell nuclei thereof of interest from a piece of tissue, and analyzing biomarkers present in the cells or cell nuclei thereof using the sample. The kit comprises the aforementioned hydrolase, optionally other components for the aforementioned pretreatment, and reagents for assay of biomarkers.

The reagents for use in assay (detection, quantitative determination or analysis) of biomarkers may be known reagents which have been used for respective biomarkers. As described above, the biomarker is not particularly limited, and examples thereof include polynucleotides, nuclear proteins, nuclear membrane proteins, and intracellular proteins. The kit usually contains a ligand (e.g., antibody or probe) binding to a targeted biomarker, or a labeling substance which visualizes a targeted biomarker. The ligand (e.g., antibody or probe) binding to a targeted biomarker or a secondary ligand binding to the first ligand is usually labeled with a labeling substance. Usually, the kit contains a nucleic acid staining compound (e.g., DAPI or propidium iodide (PI)).

4. System and Program

In an example not part of the present invention a system is provided for obtaining a sample enriched with cells or cell nuclei of interest from a piece of tissue. In one embodiment, the system carries out the steps of: analyzing a sample by flow cytometry to determine a region more abundant in a known cell type than in populations of cells or cell nuclei of other types; and obtaining a sample enriched with cells or cell nuclei of interest, based on the determined region, from a sample prepared by the pretreatment of tissue to be examined.

In the first step, particles containing cell nuclei obtained by subjecting a plurality of known cell types to the aforementioned pretreatment are analyzed by flow cytometry to obtain a histogram or scattergram of the forward scatter (FSC) intensity and/or the side scatter (SSC) intensity, and a region more abundant in cell nuclei of each cell type than in cell nuclei of other cell types is determined in the obtained histogram or scattergram.

In the second step, a sample prepared by subjecting a piece of the tissue to be examined to the aforementioned pretreatment is analyzed by flow cytometry to obtain a histogram or scattergram of the forward scatter (FSC) intensity and/or the side scatter (SSC) intensity, and a population of particles in the region predetermined to be more abundant in cell nuclei of cells identical in type to the cells of interest in the obtained histogram or scattergram is sorted or fractionated.

In a second example not part of the present invention the system carries out the steps of: preparing calibration data with a standard substance; and comparing data obtained by analyzing a test sample by flow cytometry with the calibration data to sort or fractionate a population of particles having particle sizes in a particular range.

In the first step, standard particles having known different particle sizes are analyzed by flow cytometry to produce calibration curve data of the obtained forward scatter (FSC) intensity and the particle size of the standard particles.

In the second step, a sample prepared by subjecting a piece of tissue to be examined to the aforementioned pretreatment is analyzed by flow cytometry to obtain forward scatter (FSC) intensity data, and the obtained forward scatter (FSC) intensity is compared with the calibration data to sort or fractionate a population of particles having particle sizes in a particular range.

In the second example, for example, a population of particles in a region composed of a population of particles having particle sizes of 10 μm or more, 95% or more of which have particle sizes of 11 μm to 20 μm, is sorted or fractionated to obtain a sample enriched with tumor cells or nuclei thereof.

A population of particles in a region composed of a population of particles having particle sizes of 8 μm or less, 95% or more of which have particle sizes of 4 μm to 7 μm, is sorted or fractionated to obtain a sample enriched with lymphocytes or nuclei thereof.

In a third example not part of the present invention the system provides a system for carrying out the aforementioned process for sorting or fractionating a population of particles enriched with cells or cell nuclei of interest, as well as carrying out the step of performing the aforementioned pretreatment to obtain a population of particles including individually separated cells or cell nuclei.

In a fourth example not part of the present invention a system is provided for carrying out the aforementioned process for sorting or fractionating a population of particles enriched with cells or cell nuclei of interest, and optionally the aforementioned pretreatment, as well as carrying out analysis of a biomarker with the sample enriched with cells or cell nuclei of interest.

These systems carry out the aforementioned steps usually in accordance with a predefined program. As an example not part of the present invention a program is provided for instructing the system to carry out the steps.

## EXAMPLES

The present invention will be described in further detail by the experiments below, but the experiments should not be construed as limiting the present invention.

<Test 1>

### Effects of Water-Flow Crushing on Form of Cell Nuclei

Human breast cancer cells fixed with formalin were subjected to water-flow crushing, a nuclear skeletal protein was then immuno-fluorescently stained, observed with a fluorescence microscope, and analyzed by a flow cytometer to investigate effects of water-flow crushing on cell nuclear morphology.

1. Materials and Method

1-1. Cells

The human breast cancer cell line MDA-MB-231 was acquired from ATCC and used in the present test.

1-2. Cell Culture and Formalin Fixation

The cells were cultured in Leibovitz's L-15 medium supplemented with 10% fetal bovine serum (FBS). After sufficient proliferation of the cells, a culture solution was drawn out, and the cells were washed with PBS, followed by adding TrypLE Express (Thermo Fisher Scientific) and recovering the cells. Further, the cells were sufficiently suspended in PBS, then dispensed at $1 \times 10^6$ and centrifuged, PBS was removed, a 10% formalin neutral buffer solution (Wako Pure Chemical Industries, Ltd.) was added to the cellsto subject the cells to fixation at 4°C for 24 hours. Before the cells were used, the formalin solution was removed, and the cells were washed with PBS.

1-3. Destroy of Crosslinked Structure by Heating

Histo VT One (manufactured by nacalai tesque) diluted 10 times with pure water was added to formalin-fixed cells, which was heated on a heat block at 98°C for 40 minutes. After heating, the cells were placed still at room temperature for 20 minutes, followed by removing the Histo VT One liquid.

1-4. Water-Flow Crushing

The cells were crushed in 1 mL of TBS in an ice-cooled environment for 1 minute using a homogenizer (T10) from IKA -Werke GmbH & CO. The rotation speed was set to 7,100 and 12,800 rpm. The clearance between a rotary blade and a stationary blade was set to 0.1 mm as in 5G Shaft Generator.

1-5. Immunofluorescent Staining

4% BSA/TBS containing 10% normal goat serum (Wako) was added to a microtube containing the treated product obtained by water-flow crushing, and the mixture was placed still at room temperature for 30 minutes and subjected to blocking treatment. Immunofluorescent staining was performed with a mixed antibody of an anti-lamin A antibody, an anti-lamin B1 antibody and an anti-lamin C antibody (rabbit monoclonal antibodies from Abcam: Anti-Lamin A + Lamin B1 + Lamin C antibodies (ab108922)) as primary antibodies, and a goat anti-rabbit IgG antibody (Goat anti-Rabbit IgG (H+L) Cross-Adsorbed Secondary Antibody Alexa Fluor 488 from Thermo Fisher Scientific) as a secondary antibody. The primary antibody reaction was carried out at 37°C for 15 minutes, and the secondary antibody reaction was carried out at room temperature for 15 minutes. DAPI Solution (Wako) was added to stain cell nuclei 10 minutes after the addition of the secondary antibody. The reactions were carried out under a light-shielding condition after addition of the secondary antibody. 0.5% BSA/TBS was used as an antibody diluting liquid, and washing with 0.5% BSA/TBS was carried out three times between the steps. Instead of the primary antibody, a corresponding antibody identical in type to the primary antibody was used as an isotype matched control.

1-6. Observation with Fluorescence Microscope

For microscopic observation, All-in-One Fluorescence Microscope BZ-X810 (KEYENCE CORPORATION) was used. DAPI Filter (Ex 360 nm, Em 460 nm) and GFP Filter (Ex 470 nm, Em 510 nm) were used for observation. An objective lens with a magnification of 20 times was used.

1-7. Flow Cytometer Measurement

The cells were caused to pass through a 35 μm filter (380 meshes, for flow cytometer) for Falcon (registered trademark) Cell Strainer 5 mL Tube, followed by measurement with a laser intensity gain value (FSC: 400, SSC: 400) using a flow cytometer (NovoCyte Quanteon Flow Cytometer from Agilent Technologies).

1-8 Data Analysis

For analysis of the obtained measurement data, software FlowJo v10.6.2 manufactured by Becton Dickinson and Co was used. The data was analyzed by the following procedure. A graph with the fluorescence intensity of DAPI-A in the horizontal axis and the number of particles in the vertical axis was prepared (Fig. 1), and a region of 2n or more nuclear chromosomes (area indicated by I----I) was gated as cells. Next, for the gated region, a scattergram with DAPI-H (height) in the horizontal axis and DAPI-A (area) in the vertical axis was generated (Fig. 2), and the framed area was selected as a main region of nuclei. Cells in the selected region were subjected to water-flow crushing under different conditions, and for the treated cells, scattergrams with the forward scatter (FSC) in the horizontal axis and the side scatter (SSC) in the vertical axis were generated (Fig. 4, upper raw), and average values of the FSC intensity and the SSC intensity were calculated (Table 1 below). For the cells in the selected region, histograms with the fluorescent intensity of Alexa Fluor 488 (corresponding to the amount of lamin) in the horizontal axis and the number of particles in the vertical axis were generated, and on each of the histograms, a histogram of an isotype matched control was superimposed (Fig. 4, lower raw).

[Table 1]

| | Untreated average intensity $\times 10^6$ | 7, 100rpm average intensity $\times 10^6$ | 12, 800rpm average intensity $\times 10^6$ |
|---|---|---|---|
| FSC | 3. 6 | 3. 7 | 3. 6 |

(continued)

| | Untreated average intensity×$10^6$ | 7, 100rpm average intensity×$10^6$ | 12, 800rpm average intensity×$10^6$ |
|---|---|---|---|
| SSC | 1. 4 | 1. 4 | 1. 4 |

2. Results

As shown in fluorescence microscope images of Fig. 3, lamin was observed in untreated cells as well as cells subjected to water-flow crushing under the condition of a rotation speed of 7,100 rpm or more. The scattergrams at upper raw on Fig. 4 and Table 1 show that there was no significant difference in FSC (particle size) and SSC (particle complexity) between water-flow crushing conditions. Further, as shown in the histograms at the lower raw on Fig. 4, the staining of lamin was also confirmed from the flow cytometer measurement data. Thus, it was indicated that water-flow crushing had no effect on cell nuclear morphology.

<Test 2>

<u>Recovery of Cell Nuclei from FFPE Tissue by Water-Flow Crushing</u>

A breast cancer FFPE tissue section was deparaffinized, hydrophilized, heated, then subjected to water-flow crushing, nucleic acid was stained, and the treated product was then analyzed by a flow cytometer to evaluate effects of the rotation speed and the clearance between a rotary blade and a stationary blade in water-flow crushing on recovery of cell nuclei.

1. <u>Materials and Method</u>

1-1. <u>FFPE Tissue Block</u>

A breast cancer FFPE tissue block purchased from ProteoGenex, Inc was used.

1-2. <u>Preparation of FFPE Section</u>

A tissue section with a thickness of 20 μm was prepared from a FFPE block using a sliding microtome manufactured by Thermo Fisher Scientific.

1-3. <u>Deparaffinization/Hydrophilization</u>

1 mL of xylene was added to the FFPE section, the section was placed still for 10 minutes, and xylene was then removed. These steps were carried out again to completely remove paraffin. Subsequently, the section was exposed to 100% ethanol, 50% ethanol and deionized water in this order for 3 minutes each to hydrophilize the section.

1-4. <u>Destruction of Crosslinked Structure by Heating</u>

The same procedure as in Test 1 was carried out.

1-5. <u>Water-Flow Crushing</u>

The tissue after heating was crushed in 1 mL of TBS in an ice-cooled environment for 1 minute using a water flow shear apparatus (RP-10) from Sysmex Corporation and a homogenizer (T10) from IKA. The rotation speed was set to 10,000 rpm for RP-10, and 7,100, 9,100 and 12,800 rpm for T10. The clearance between a rotary blade and a stationary blade was set to 0.05 mm as in the accessory for RP-10, 0.1 mm as in 5G Shaft Generator and 0.25 mm as in 8G Shaft Generator for T10.

1-6. <u>Nucleic Acid-Staining</u>

0.5% BSA/TBS and DAPI Solution (Wako) were added to the cells after crushing, and the cells were placed still for 5 minutes under a light-shielding condition.

1-7. Flow Cytometer measurement

The same procedure as in Test 1 was carried out.

1-8. Data Analysis

For analysis of the obtained measurement data, software FlowJo v10.6.2 manufactured by Becton Dickinson and Co was used. The data was analyzed by the following procedure. A graph with the fluorescent intensity of DAPI-A in the horizontal axis and the number of particles in the vertical axis was prepared, and regions with 2n or more nuclear chromosomes, 2n and less than 2n (tissue debris) were each gated (Fig. 5). The ratio of the number of 2n cells to the total number of analyzed cells (2n recovery ratio), the ratio of the number of less than 2n cells to the number of 2n cells, and the ratio of the number of 2n cells to the number of 2n or more cells were calculated.

2. Results

As shown in Fig. 6, the 2n recovery ratio was 9% or more regardless of the crushing apparatus, the rotation speed and the clearance between a rotary blade and a stationary blade (upper raw diagram). The ratio of the number of less than 2n cells (tissue debris) to the number of 2n cells was less than 10, and constant (middle raw diagram). Further, the ratio of the number of 2n cells to the number of 2n or more cells was 50% or more (lower raw diagram). From the above, it was revealed that in recovery of cell nuclei from FFPE, a constant cell nucleus recovery ratio was exhibited when the rotation speed for physical crushing was in the range of 7,100 to 2,800 rpm, and the clearance between a rotary blade and a stationary blade was in the range of 0.05 to 0.25 mm.

<Test 3>

Examination of Form of Cell Nuclei Recovered from FFPE Tissue Section by Water-Flow Crushing

The breast cancer FFPE tissue section was subjected to pretreatment including water-flow crushing, and an intranuclear skeletal protein was immuno-fluorescently stained, observed with a fluorescence microscope, and analyzed by a flow cytometer to examine effects of water-flow crushing on cell nuclear morphology.

1. Materials and Method

1-1. FFPE Tissue Block

The same procedure as in Test 2 was carried out.

1-2. Preparation of FFPE Section

The same procedure as in Test 2 was carried out.

1-3. Deparaffinization/Hydrophilization

The same procedure as in Test 2 was carried out.

1-4. Destruction of Crosslinked Structure by Heating

The same procedure as in Test 1 was carried out.

1-5. Water-Flow Crushing

The tissue after heating was crushed in 1 mL of TBS in an ice-cooled environment for 1 minute using a water flow shear apparatus (RP-10) from Sysmex Corporation and a homogenizer (T10) from IKA. The rotation speed was set to 10,000 rpm for RP-10, and 12,800 rpm for T10. The clearance between a rotary blade and a stationary blade was set to 0.05 mm as in the accessory for RP-10, and 0.1 mm as in 5G Shaft Generator for T10.

1-6. Immunofluorescent Staining

The same procedure as in Test 1 was carried out.

1-7. Flow Cytometer Measurement

The same procedure as in Test 1 was carried out.

1-8. Data Analysis

The same procedure as in Test 1 was carried out.

2. Results

As shown in Fig. 7, lamin was observed in cell nuclei recovered by subjecting the FFPE tissue section to water-flow crushing. As shown in Fig. 8, comparable results were obtained from flow cytometer measurement data. Thus, it was indicated that water-flow crushing had no effect on cell nuclear morphology.

<Test 4>

Effects of ultrasonic crushing on cell nuclear morphology

Human breast cancer cells fixed with formalin were subjected to ultrasonic crushing, an intranuclear skeletal protein was then immuno-fluorescently stained, observed with a fluorescence microscope, and analyzed by a flow cytometer to examine effects of ultrasonic crushing on cell nuclear morphology.

1. Materials and Method

1-1. Cells

The human breast cancer cell line MDA-MB-231 was acquired from ATCC, and used in the present test.

1-2. Cell Culture and Formalin Fixation

The same procedure as in Test 1 was carried out.

1-3. Destruction of Crosslinked Structure by Heating

The same procedure as in Test 1 was carried out.

1-4. Ultrasonic Crushing

The cells were crushed in 1 mL of TBS in an ice-cooled environment for 30 seconds using an ultrasonic crushing apparatus (VCX130PB) from Sonics & Materials, Inc. The output intensity was set to 20%, 30% or 40% in terms of an amplitude.

1-5. Immunofluorescent Staining

The same procedure as in test 1 was carried out.

1-6. Observation with Fluorescence Microscope

The same procedure as in test 1 was carried out.

1-7. Flow Cytometer Measurement

The same procedure as in test 1 was carried out.

1-8. Data Analysis

The same procedure as in test 1 was carried out.

2. Results

Fig. 9 shows images of lamin and DAPI, which were observed with a fluorescent microscope. The upper raw of Fig. 10 show scattergrams with FSC in the horizontal axis and SSC in the vertical axis for cells subjected to ultrasonic crushing under respective conditions, and Table 2 shows average values of the FSC intensity and the SSC intensity. The lower raw of Fig. 10 show histograms with the fluorescence intensity of Alexa Fluor 488 (corresponding to the amount of lamin) in the horizontal axis and the number of particles in the vertical axis.

As shown in the fluorescence microscope images of Fig. 9, lamin was observed in untreated cells as well as cells subjected to ultrasonic crushing regardless of the conditions of the ultrasonic crushing. As shown in the upper raw of Fig. 10 and Table 2, there was no significant difference in FSC (particle size) and SSC (particle complexity) between ultrasonic crushing conditions. Further, as shown in the lower raw of Fig. 10, the staining of lamin was also confirmed from the flow cytometer measurement data. Thus, it was indicated that the ultrasonic crushing had no effect on cell nuclear morphology.

[Table 2]

| | Untreated average intensity$\times10^6$ | 20% average intensity$\times10^6$ | 3 0 % average intensity$\times10^6$ | 40% average intensity$\times10^6$ |
|---|---|---|---|---|
| FSC | 3. 9 | 3. 8 | 3. 8 | 3. 7 |
| SSC | 1. 4 | 1. 4 | 1. 3 | 1. 3 |

<Test 5>

Recovery of Cell Nuclei from FFPE Tissue Section by Combination of Water-Flow Crushing and Ultrasonic Crushing

A breast cancer FFPE tissue section was deparaffinized, hydrophilized, heated, subjected to water-flow crushing and ultrasonic crushing, nucleic acid was stained, and the treated product was then analyzed by a flow cytometer to evaluate effects of the intensity of ultrasonic crushing on recovery of cell nuclei.

1. Materials and Method

1-1. FFPE Tissue Block

The same procedure as in Test 2 was carried out.

1-2. Preparation of FFPE Section

The same procedure as in Test 2 was carried out.

1-3. Deparaffinization/Hydrophilization

The same procedure as in Test 2 was carried out.

1-4. Antigen Retrieval

The same procedure as in Test 1 was carried out.

1-5. Water-Flow Crushing and Ultrasonic Crushing

The tissue after heating was crushed at 10,000 rpm in 1 mL of TBS in an ice-cooled environment for 1 minute using a water flow shear apparatus (RP-10) from Sysmex Corporation. Further, using an ultrasonic crushing apparatus (VCX130PB) from Sonics & Materials, Inc, the tissue after water-flow crushing was crushed at an output intensity of 20%, 30% or 40% in terms of an amplitude in 1 mL of TBS in an ice-cooled environment for 30 seconds.

1-6. Nucleic Acid-Staining

The same procedure as in Test 2 was carried out.

1-7. Flow Cytometer measurement

The same procedure as in Test 1 was carried out.

1-8. Data Analysis

The same procedure as in Test 2 was carried out.

2. Results

As shown in Fig. 11, it was confirmed that in the case of combination of water-flow crushing and ultrasonic crushing, the 2n recovery ratio decreased as the ultrasonic intensity increased. On the other hand, the ratio of the number of less than 2n cells (tissue debris) to the number of 2n cells increased, and particularly significantly increased at a crushing intensity of 40%. Thus, it was indicated that the 2n recovery ratio was apparently low due to an increase in the total number of analyzed cells (tissue debris), but ultrasonic crushing did not cause decrease in 2n recovery ratio. In fact, the ratio of the number of 2n cells to the number of 2n or more cells was 70% or more regardless of the ultrasonic intensity, and increased as compared to a case where crushing with ultrasonication was not performed. From the above, it was indicated that in recovery of cell nuclei from FFPE, the combination of water-flow crushing and ultrasonic crushing was effective for crushing aggregates of 2n or more and improved recovery efficiency although the amount of tissue debris of less than 2n increased as the ultrasonic intensity was increased.

<Test 6>

Validation of Ultrasonic Crushing Time in Combination of Water-Flow Crushing and Ultrasonic Crushing

A breast cancer FFPE tissue section was subjected to pretreatment including water-flow crushing and ultrasonic crushing, then stained with a nucleic acid, and analyzed by a flow cytometer to evaluate effects of the ultrasonic crushing time on recovery of cell nuclei.

1. Materials and Method

1-1. FFPE Tissue Block

The same procedure as in Test 2 was carried out.

1-2. Preparation of FFPE Section

The same procedure as in Test 2 was carried out.

1-3. Deparaffinization/Hydrophilization

The same procedure as in Test 2 was carried out.

1-4. Antigen Retrieval

The same procedure as in Test 1 was carried out.

1-5. Water-Flow Crushing and Ultrasonic Crushing

The tissue after heating was crushed at 10,000 rpm in 1 mL of TBS in an ice-cooled environment for 1 minute using a water flow shear apparatus (RP-10) from Sysmex Corporation. Further, using an ultrasonic crushing apparatus (VCX130PB) from Sonics & Materials, Inc, the tissue after water-flow crushing was crushed at output intensities of 20% and 30% in 1 mL of TBS in an ice-cooled environment for 1, 2 and 3 minutes.

1-6. Nucleic Acid-Staining

The same procedure as in Test 2 was carried out.

1-7. Flow Cytometer measurement

The same procedure as in Test 1 was carried out.

1-8. Data Analysis

The same procedure as in Test 2 was carried out.

2. Results

As shown in Fig. 12, it was confirmed that similarly to the increased ultrasonic intensity, the 2n recovery ratio decreased and the ratio of the number of less than 2n cells (tissue debris) to the number of 2n cells increased as the crushing time became longer at output intensities of 20% and 30% in terms of an amplitude. In particular, the ratio of the number of less than 2n cells (tissue debris) to the number of 2n cells significantly increased when the crushing time was 2 minutes or more. It was indicated as the crushing time was increased, the 2n recovery ratio apparently decreased because the amount of tissue debris increased, leading to an increase in the total number of analyzed cells. In fact, the ratio of the number of 2n cells to the number of 2n or more cells was 70% or more regardless of the crushing time, and increased as compared to a case ultrasonic crushing was not performed.

From the above, it was indicated that in recovery of cell nuclei from FFPE, the increased ultrasonic crushing time was effective for crushing aggregates of 2n or more and improved recovery efficiency although the amount of tissue debris of less than 2n was increased as in the increased crushing intensity.

<Test 7>

Examination of Form of Cell Nuclei Recovered from FFPE Tissue Section by Combination of Water-Flow Crushing and Ultrasonic Crushing

A breast cancer FFPE tissue section was subjected to pretreatment including water-flow crushing and ultrasonic crushing, and an intranuclear skeletal protein was then immuno-fluorescently stained, observed with a fluorescence microscope, and analyzed by a flow cytometer to examine effects of water-flow crushing on cell nuclear morphology.

1. Materials and Method

1-1. FFPE Tissue Block

The same procedure as in Test 2 was carried out.

1-2. Preparation of FFPE Section

The same procedure as in Test 2 was carried out.

1-3. Deparaffinization/Hydrophilization

The same procedure as in Test 2 was carried out.

1-4. Destruction of Crosslinked Structure by Heating

The same procedure as in Test 1 was carried out.

1-5. Water-Flow Crushing and Ultrasonic Crushing

The tissue after heating was crushed at 10,000 rpm in 1 mL of TBS in an ice-cooled environment for 1 minute using a water flow shear apparatus (RP-10) from Sysmex Corporation. Further, using an ultrasonic crushing apparatus (VCX130PB) from Sonics & Materials, Inc, the tissue after water-flow crushing was crushed at an output intensity of 20% in 1 mL of TBS in an ice-cooled environment for 1 minute.

1-6. Immunofluorescent Staining

The same procedure as in Test 1 was carried out.

1-7. Flow Cytometer measurement

The same procedure as in Test 1 was carried out.

1-8. Data Analysis

The same procedure as in Test 1 was carried out.

2. Results

As shown in Fig. 13, lamin was observed in cell nuclei recovered by subjecting the FFPE tissue section to water-flow crushing and ultrasonic crushing. As shown in Fig. 14, comparable results were obtained from flow cytometer measurement data. Thus, it was indicated that the combination of water-flow crushing and ultrasonic crushing had no effect on cell nuclear morphology.

<Test 8>

Effects of treatments with different enzymes on cell nuclei morphology of formalin-fixed cells

Formalin-fixed human breast cancer cells were treated with various enzymes, and an intranuclear skeletal protein was then immuno-fluorescently stained, observed with a fluorescence microscope, and analyzed by a flow cytometer.

1. Materials and Method

1-1. Cells

The human breast cancer cell line MDA-MB-231 was acquired from ATCC, and used in the present test.

1-2. Cell Culture and Formalin Fixation

The same procedure as in Test 1 was carried out.

1-3. Destruction of Crosslinked Structure by Heating

The same procedure as in Test 1 was carried out.

1-4A. Enzymatic Treatment (Thrombin)

A thrombin reagent (25 mM Tris-HCl pH 7.4, 150 mM NaCl, 1,000 KU/L thrombin, 10 mM $CaCl_2$) was added to the cells after the heating treatment, and the cells were heated on a heat block at 37°C for 20 minutes.

1-4B. Enzymatic Treatment (Dispase)

A dispase reagent (25 mM Tris-HCl pH 7.4, 150 mM NaCl, 3,000 PU/mL dispase) was added to the cells after the heating treatment, and the cells were heated on a heat block at 37°C for 20 minutes.

1-4C. Enzymatic Treatment (Trypsin)

A trypsin reagent (25 mM Tris-HCl pH 7.4, 150 mM NaCl, 2.5 mg/mL trypsin) was added to the cells after the heating treatment, and the cells were heated on a heat block at 37°C for 20 minutes.

1-4D. Enzymatic Treatment (Proline Endopeptidase)

A proline endopeptidase reagent (25 mM Tris-HCl pH 7.4, 10 KU/L proline endopeptidase) was added to the cells after the heating treatment, and the cells were heated on a heat block at 37°C for 20 minutes.

1-4E. Enzymatic Treatment (Hyaluronidase)

A hyaluronidase reagent (25 mM Tris-HCl pH 7.4, 150 mM NaCl, 4,000-10,000 KU/L hyaluronidase I-S, 7,500-30,000 KU/L hyaluronidase IV-S) was added to the cells after the heating treatment, and the cells were heated on a heat block at 37°C for 20 minutes.

1-4F. Not Treated with Enzyme

A buffer solution (25 mM Tris-HCl, pH 7.4, 150 mM NaCl) was added to the cells after the heating treatment, and the cells were heated on a heat block at 37°C for 20 minutes.

1-5. Immunofluorescent Staining

The same procedure as in Test 1 was carried out.

1-6. Flow Cytometer Measurement

The same procedure as in Test 1 was carried out.

1-7. Data Analysis

The same procedure as in Test 1 was carried out.

2. Results

As shown in Fig. 15, lamin was not observed in the fluorescence microscope images of the cells treated with dispase and trypsin, whereas lamin was observed in the fluorescence microscope images of the cells treated with thrombin, proline endopeptidase and hyaluronidase as well as the untreated cells. As shown in Fig. 16, comparable results were obtained from flow cytometer measurement. Thus, it was indicated that the treatments with thrombin, proline endopeptidase and hyaluronidase have no effect on cell nuclear morphology.

<Test 9>

Effects of Enzymatic Treatment in Recovery of Cell Nuclei from FFPE Tissue Section

A breast cancer FFPE tissue section was treated with an enzyme, then subjected to pretreatment including water-flow crushing and ultrasonic crushing, and an intranuclear skeletal protein was then immuno-fluorescently stained, observed with a fluorescence microscope, and analyzed by a flow cytometer to examine effects of enzymatic treatment on cell nuclear morphology.

1. Materials and Method

1-1. FFPE Tissue Block

The same procedure as in Test 2 was carried out.

1-2. Preparation of FFPE Section

The same procedure as in Test 2 was carried out.

1-3. Deparaffinization/Hydrophilization

The same procedure as in Test 2 was carried out.

1-4. Destruction of Crosslinked Structure by Heating

The same procedure as in Test 1 was carried out.

1-5A. Enzymatic Treatment (Thrombin)

The same procedure as in test 8 was carried out.

1-5B. Enzymatic Treatment (Dispase)

The same procedure as in test 8 was carried out.

1-6. Water-Flow Crushing and Ultrasonic Crushing

The tissue after the thrombin treatment was treated in the same manner as in test 7.

1-7. Immunofluorescent Staining

The same procedure as in Test 1 was carried out.

1-8. Flow Cytometer Measurement

The same procedure as in Test 1 was carried out.

1-9. Data Analysis

The same procedure as in Test 1 was carried out.

2. Results

As shown in Fig. 17, lamin was not observed in the fluorescence microscope images when dispase treatment was performed for recovery of cell nuclei from the FFPE tissue section. On the other hand, lamin was observed when thrombin treatment was added to water-flow crushing and ultrasonic crushing. As shown in Fig. 18, comparable results were obtained from flow cytometer measurement. Thus, it was indicated that addition of thrombin treatment to physical crushing had no effect on cell nuclear morphology.

<Test 10>

Sorting of Cancer Cells and Lymphocytic Cells in Formalin-Fixed Cells

Formalin-fixed breast cancer cells and T lymphocytes were heated, treated with thrombin, then stained with a nucleic acid, and analyzed by a flow cytometer to compare the distributions of cancer cells and lymphocytic cells.

1-1. Cells

Human breast cancer cell lines T47D, MDA-MB-231 and SKBr3 and the human T lymphocytic cell line Jurkat were acquired from ATCC, and used in the present test.

1-2. Cell Culture and Formalin Fixation

The human breast cancer cell lines T47D, MDA-MB-231 and SKBr3 were cultured, respectively, in RPMI-1640 medium, Leibovitz's L-15 medium and McCoy's 5A medium each supplemented with 10% FBS. The Jurkat cell line was cultured in RPMI-1640 medium supplemented with 10% FBS. After sufficient proliferation of the cells, the cells were recovered, and fixed with formalin as in Test 1.

1-3. Destruction of Crosslinked Structure by Heating

The same procedure as in Test 1 was carried out.

1-4. Enzymatic Treatment (Thrombin)

The same procedure as in test 8 was carried out.

1-5. Nucleic Acid-Staining

The same procedure as in Test 2 was carried out.

1-6. Flow Cytometer Measurement

The same procedure as in Test 1 was carried out.

1-7. Data Analysis

As in Test 1, on a graph with the fluorescence intensity of DAPI-A in the horizontal axis and the number of particles in the vertical axis, a region of 2n or more nuclear chromosomes was gated as cells, and the gated population was developed on a scattergram with the forward scatter (FSC) in the horizontal axis and the side scatter (SSC) in the vertical axis.

2. Results

Fig. 19 shows scattergrams with the forward scatter (FSC) in the horizontal axis and the side scatter (SSC) in the vertical axis. From the scattergrams of the respective cells, it was confirmed that for the populations of cells treated with thrombin, cancer cells and lymphocytic cells had different distributions.

<Test 11>

Quantitative Determination of Particle Sizes of Cancer Cells and Lymphocytic Cells in Formalin-Fixed Cells

Synthetic beads having known particle sizes were measured by a flow cytometer, a regression equation of the forward scatter (FSC) intensity and the particle size was determined, and the particle sizes of cancer cells and lymphocytic cells in formalin-fixed cells were quantitatively determined based on the regression equation.

1. Materials and Method

1-1. Preparation of Size Calibration Bead Solution

Size calibration beads purchased from Thermo Fisher Scientific (Flow Cytometry Size Calibration Kit) were used. A bead solution was prepared in accordance with the written instructions.

1-2. Measurement by Flow Cytometer and Preparation of Regression Equation

The bead solution was measured by a flow cytometer in the same manner as in Test 1. From the obtained data, histograms with the FSC intensity in the horizontal axis and the particle size in the vertical axis (not shown) were prepared for calibration beads of respective sizes using software FlowJo v10.6.2 manufactured by Becton Dickinson and Co, and from the histograms, the modal values of FSC intensity were calculated for beads of respective sizes. From a graph prepared by plotting the size of particles on the horizontal axis and the modal value of FSC-H intensity on the vertical axis (Fig. 20), a secondary regression equation ($y = 0.0062x^2 + 0.13x + 0.020$, $R = 0.9975$).

1-3. Quantitative Determination of Particle Sizes of Cell Lines

From the flow cytometer measurement data for the four cell lines, which had been obtained in Test 10, histograms with the FSC intensity in the horizontal axis and the number of particles in the vertical axis (Fig. 21) were prepared using FlowJo v10.6.2, and 5 percentile values and 95 percentile values were calculated. The 5 percentile values and the 95 percentile values for the respective cell lines are as follow.

[Table 3]

| | 5 Percentile value (×10^6) | 95 Percentile value (×10^6) |
|---|---|---|
| **Jurkat** | **0. 67** | **2. 10** |
| **MDA-M8-231** | **1. 42** | **3. 70** |
| **T47D** | **1. 29** | **3. 55** |
| **SKBr3** | **1. 94** | **4. 71** |

The interval between the 5 percentile value and the 95 percentile value was considered to correspond to a principal population for each cell line. From the FSC-H intensity of each cell line in the interval, the particle size for each cell line was calculated based on the aforementioned regression equation.

2. Results

The range of the calculated particle size for each cell line is shown below.

[Table 4]

| | Particle size (μm) |
|---|---|
| Jurkat | 4. 2~10. 6 |
| MDA-MB-231 | 7. 8~16. 0 |
| T47D | 7. 3~15. 6 |
| SKBr3 | 10. 0~19. 0 |

From these results, it was shown that human breast cancer cells were separated from most lymphocytes by gating particles of 10 μm or more.

<Test 12>

Sorting of Lymphocytes and Cancer Cells in Formalin-Fixed Cells by Side Scatter Intensity

Regions where lymphocytes and cancer cells can be sorted by the side scatter (SSC) intensity obtained by the flow cytometer measurement in Test 10 were determined.

1. Method

From the flow cytometer measurement data for the four cell lines, which had been obtained in Test 10, histograms with the SSC intensity in the horizontal axis and the number of particles in the vertical axis (Fig. 22) were prepared using FlowJo v10.6.2, and 5 percentile values and 95 percentile values were calculated. The 5 percentile values and the 95 percentile values for the respective cell lines are as follow.

[Table 5]

| | 5 Percentile value (×10^6) | 95 Percentile value (×10^6) |
|---|---|---|
| Jurkat | 0. 21 | 0. 96 |
| MDA-MB-231 | 0. 50 | 1. 39 |
| T47D | 0. 50 | 1. 64 |
| SKBr3 | 0. 74 | 2. 16 |

2. Results

From the distribution of the obtained SSC-H intensity of each cell line, it was shown that most human breast cancer cells were separated from lymphocytes by gating a region with a SSC-H intensity of $1.0 \times 10^6$.

<Test 13>

Distribution of Breast Cancer-Derived Cell Nuclei or Lymphocyte-Derived Cell Nuclei on FSC-SSC Scattergram, and Sorting Based Thereon

Sections of a breast cancer FFPE tissue and a normal lymph node FFPE tissue were subjected to pretreatment including heating, thrombin treatment, water-flow crushing and ultrasonic crushing, then stained with a nucleic acid, and analyzed by a flow cytometer.

1. Materials and Method

1-1. FFPE Tissue Block

The same procedure as in Test 2 was carried out.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 1 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Nucleic Acid-Staining

The same procedure as in Test 2 was carried out.

1-8. Flow Cytometer Measurement

The same procedure as in Test 1 was carried out.

1-9. Data Analysis

As in Test 1, on histograms with the fluorescence intensity of DAPI-A in the horizontal axis and the number of particles in the vertical axis, a region of 2n or more nuclear chromosomes was gated as cells, and the gated population was developed on a scattergram with the forward scatter (FSC) in the horizontal axis and the side scatter (SSC) in the vertical axis.

2. Results

The FSC-SSC scattergrams for cancer cell-derived cell nuclei and lymphocyte-derived cell nuclei were superimposed (Fig. 23) to compare the distributions of the former cell nuclei and the latter cell nuclei. It was confirmed that the cancer cell-derived cell nuclei and the lymphocyte-derived cell nuclei had different distributions, and the cancer cell-derived cell nuclei were separated from the lymphocyte-derived cell nuclei in the region with a FSC-H intensity of $2.00 \times 10^6$ (10 μm) or more and a SSC-H intensity of $1.00 \times 10^6$ or more (upper right region of Fig. 23), i.e., the region determined in tests 11 and 12.

<Test 14>

Quantitative Determination of Particle Size of Cancer Cells and Lymphocytic Cells in Formalin-Fixed Cells by Other Types of Flow Cytometers

It was confirmed that it was also possible to perform quantitative determination of the particle sizes of cancer cells and lymphocytic cells in Test 11 by other types of equipment.

1. Materials and Method

1-1. Preparation of Size Calibration Beads and Bead Solution

The same procedure as in Test 11 was carried out.

1-2. Flow Cytometer Measurement

The bead solution was measured by Cyflow Space from Sysmex Corporation and FACSAriaII from Becton Dickinson and Co. A FSC laser intensity gain value of 200 and a SSC laser intensity gain value of 290 were applied to the measurement by Cyflow Space from Sysmex Corporation, whereas a FSC laser intensity voltage value of 65 and a SSC laser intensity voltage value of 320 were applied to the measurement by FACSAriaII from Becton Dickinson and Co.

1-3. Preparation of Regression Equation

As in Test 11, a regression equation was calculated from a graph prepared by plotting the particle size on the horizontal axis and the modal value of FSC-H intensity on the vertical axis.

2. Results

Fig. 24 shows graphs obtained by plotting the particle size on the horizontal axis and the modal value of FSC intensity on the vertical axis. The graphs were prepared from data obtained by testing the bead solution by Cyflow Space from Sysmex Corporation and FACSAriaII from Becton Dickinson and Co. From Cyflow Space, the linear regression equation of $y = 19.5x - 19.3$ ($R = 0.9996$) was obtained, and from FACSAriaII, the secondary regression equation of $y = 0.04x^2 + 5.32x - 2.27$ ($R = 0.9938$) was obtained. These regression equations dictated that FSC-H intensities at a particle size of 10 $\mu$m were 180 (Cyflow Space) and $55 \times 10^3$ (FACSAriaII).

<Test 15>

Sorting of Cancer Cells in Formalin-Fixed Cells by Other Types of Flow Cytometers

It was confirmed that it was also possible to sort cancer cells in formalin-fixed cells by other types of equipment.

1-1. Cells

The same cell lines as in Test 10 (human breast cancer cell lines T47D, MDA-MB-231 and SKBr3 and human T lymphocytic cell line Jurkat) were used.

1-2. Cell Culture and Formalin Fixation

The same procedure as in Test 10 was carried out.

1-3. Heating

The same procedure as in Test 1 was carried out.

1-4. Enzymatic Treatment (Thrombin)

The same procedure as in test 8 was carried out.

1-5. Nucleic Acid-Staining

The same procedure as in Test 2 was carried out.

1-6. Flow Cytometer Measurement

The same procedure as in Test 1 was carried out.

1-7. Data Analysis

As in Test 1, on histograms with the fluorescence intensity of DAPI-A in the horizontal axis and the number of particles in the vertical axis, a region of 2n or more nuclear chromosomes was gated as cells, and the gated population was displayed on a scattergram with the forward scatter (FSC) in the horizontal axis and the side scatter (SSC) in the vertical axis (Fig. 25: Cyflow Space, Fig. 26: FACSAriaII). Histograms with the SSC intensity in the horizontal axis and the number of particles in the vertical axis (Fig. 27: Cyflow Space, Fig. 28: FACSAriaII) using FlowJo v10.6.2, and 5 percentile values and 95 percentile values were calculated. The 5 percentile values and the 95 percentile values for respective cell lines, which were obtained by Cyflow Space and FACSAriaII, are shown below.

[Table 6]

| | 5 Percentile value | 95 Percentile value |
|---|---|---|
| Jurkat | 38 | 129 |
| MDA-MB-231 | 83 | 262 |
| T47D | 77 | 246 |
| SKBr3 | 103 | 317 |

[Table 7]

| | 5 Percentile value ($\times 10^3$) | 95 Percentile value ($\times 10^3$) |
|---|---|---|
| Jurkat | 24. 1 | 95. 6 |
| MDA-MB-231 | 48. 2 | 141. 1 |
| T47D | 46. 1 | 145. 9 |
| SKBr3 | 68. 5 | 191. 8 |

2. Results

As shown in Figs. 25 and 26, cancer cell-derived cell nuclei and lymphocytic cell nuclei were distributed at different locations on the scattergram regardless of Cyflow Space or FACSAriaII (Fig. 25: Cyflow Space, Fig. 26: FACSAriaII). The data shown in Figs. 27 and 28 indicated that the use of a specific SSC intensity enabled sorting of cancer cell nuclei and lymphocytic cell nuclei, and the numerical values shown in Table 6 and 7 dictated that the SSC intensities for separation and concentration of cancer cell nuclei as measured by Cyflow Space and FACSAriaII were 130 and $100 \times 10^3$. This was used in combination of the FSC intensities determined in Test 14, i.e., 180 (Cyflow Space) and $5 \times 10^3$ (FACSAriaII), to determine regions allowing cancer cell nuclei (upper right regions of Fig. 29) to be sorted on scattergrams with SSC in the vertical axis and FSC in the horizontal axis (Fig. 29). As is evident from Fig. 29, in this region, cancer cell nuclei are predominant, and can be sorted from lymphocytic cell nuclei.

<Test 16>

Observation of Particles in Region determined to Be Predominated by Cancer Cell-Derived Cell Nuclei, and Measurement of Sizes Thereof

Particles in a region determined to be predominated by cancer cell-derived cell nuclei were fractionated, and observed with a fluorescence microscope, and microscopic test image data was analyzed to quantitatively determine the sizes thereof.

1. Materials and Method

1-1. FFPE Tissue Block

The same procedure as in Test 2 was carried out.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 1 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Immunofluorescent Staining

4% BSA/TBS containing 10% normal goat serum (Wako) was added to the crushed product, and the mixture was placed still at room temperature for 30 minutes and subjected to blocking treatment. Immunofluorescent staining was performed with a pan-cytokeratin antibody (Abcam, rabbit polyclonal antibody: anti-wide spectrum cytokeratin antibody (AB9377-500)) as a primary antibody, and Goat anti-Rabbit IgG (H+L) Cross-Adsorbed Secondary Antibody Alexa Fluor 488 from Thermo Fisher Scientific as a secondary antibody. The primary antibody reaction was carried out at 37°C for 15 minutes, and the secondary antibody reaction was carried out at room temperature for 15 minutes. DAPI Solution (Wako) was added to stain cell nuclei 10 minutes after the addition of the secondary antibody. The reactions were carried out under a light-shielding condition after addition of the secondary antibody. 0.5% BSA/TBS was used as an antibody diluting liquid, and washing with 0.5% BSA/TBS was carried out three times between the steps.

1-8. Fractionation by Cell Sorter and Preparation of Sample

The cells were caused to pass through a 35 μm filter (380 meshes) (for flow cytometer) for Falcon (registered trademark) Cell Strainer 5 mL Tube, followed by measurement with FACSAriaII from Becton Dickinson and Co adjusted to a FSC laser intensity voltage value of 65 and a SSC laser intensity voltage value of 320. Fractionation was performed by gating a main region of 2n or more nuclear chromosomes in a histogram with the fluorescence intensity of DAPI-A in the horizontal axis and the number of particles in the vertical axis, then developing the gated population on a scattergram with FSC in the horizontal axis and SSC in the vertical axis (Fig. 30), and fractionating 100,000 cells from each of the region of Area 1 (FSC-H intensity: $125 \times 10^3$ to $200 \times 10^3$, SSC-H intensity: $100 \times 10^3$ to $200 \times 10^3$) and the region of Area 2 (FSC-H intensity: $40 \times 10^3$ to $100 \times 10^3$, SSC-H intensity: $10 \times 10^3$ to $90 \times 10^3$) as a control. The fractionated cells were put in a PBS buffer solution. About 20,000 cells were placed on slide glass, and covered with cover glass.

1-9. Fluorescence Microscope

The same procedure as in Test 1 was carried out.

1-10. Image Analysis

The major diameters and the minor diameters of cells were measured by image analysis, and average values were calculated. Image analysis was performed with image processing software ImageJ (developed by National Institutes of health). Based on luminance values of pixels derived from luminescence of DAPI, the luminance value was binarized to determine cell nuclei, information from the scale bar was used as a reference to calculate the major diameters and the minor diameters of cell nuclei, and average values of the major diameters and the minor diameters of cell nuclei in one visual field were determined (Table 8)

[Table 8]

| | Major diameter (μm) | Minor diameter (μm) |
|---|---|---|
| Area 1 (n=35) | 14. 3±0. 4 | 10. 2±0. 3 |
| Area 2 (n=8) | 9. 7±0. 5 | 7. 4±0. 2 |

2. Results

Fig. 31 shows the microscopic test images of DAPI and cytokeratin in areas 1 and 2. Particles in Area 1 are stained at cytokeratin, but particles in Area 2 are not stained. The results of image analysis in Table 8 show that the particle size in Area 1 was larger than the particle size in Area 2. From the above results, it was indicated that in Area 1, cancer cell-derived cell nuclei were concentrated, whereas in Area 2, a population of particles abundant in lymphocyte-derived cell nuclei was present. That is, a population predominated by cancer cell-derived cell nuclei was separated from lymphocyte-derived cell nuclei by fractionating a population of particles in a specific region.

<Test 17>

Measurement with fraction of lymphocytic cell nuclei by other types of equipment

A population of particles in Area 2, which was determined to be abundant in lymphocyte-derived cell nuclei and fractionated in Test 16, was measured by other types of flow cytometers, and how many lymphocyte-derived cell nuclei were mixed up in a region determined to allow cancer cell-derived cell nuclei to be sorted in Tests 13 and 15 was examined.

1. Materials and Method

1-1. Preparation of Sample

The fraction of Area 2 fractionated in Test 2 was used.

1-2. Flow Cytometer Measurement

The same procedure as in tests 1 and 14 was carried out.

1-3. Data analysis

The acquired flow cytometer data was not subjected to gating processing, and the population was developed on a FSC-SSC scattergram.

2. Results

As shown in Fig. 32, little of the fraction of Area 2 (Fig. 30) fractionated by a cell sorter from Becton Dickinson and Co was present in the region of cancer cell nuclei regardless of whether the flow cytometer used for measurement was NovoCyte Quanteon Flow Cytometer or Cyflow Space (0.48% and 1.25%). Regardless of which type of equipment was used, the region determined to be abundant in cancer cell-derived cell nuclei was free of lymphocyte-derived cell nuclei, and this indicated there was not a difference depending on the type of equipment.

<Test 18>

Sorting of Cancer Cells and Lymphocytic Cells When Formalin-Fixed Cells Are Treated with Different Enzymes

Breast cancer cells and lymphocytic cells fixed with formalin were subjected to pretreatment including treatments with different enzymes, nucleic acid was stained, the treated product was then analyzed by a flow cytometer, and distributions on FSC-SSC scattergrams were compared.

1. Materials and Method

1-1. Cells

Human breast cancer cell lines T47D, MDA-MB-231 and SKBr3 and the human T lymphocytic cell line Jurkat were acquired from ATCC, and used in the present test.

1-2. Cell Culture and Formalin Fixation

The same procedure as in Test 10 was carried out.

1-3. Heating

The same procedure as in Test 1 was carried out.

1-4A. Enzymatic Treatment (Thrombin)

The same procedure as in test 8 was carried out.

1-4B. Enzymatic Treatment (Dispase)

The same procedure as in test 8 was carried out.

1-5. Nucleic Acid-Staining

The same procedure as in Test 2 was carried out.

1-6. Flow Cytometer Measurement

The same procedure as in Test 1 was carried out.

1-7. Data Analysis

As in Test 1, populations were gated, and the gated populations were developed on scattergrams with the forward scatter (FSC) in the horizontal axis and the side scatter (SSC) in the vertical axis. For comparing effects of different enzymes used for the treatment, the scattergram for the lymphocytic cells was superimposed on the scattergram for each of the breast cancer cell lines.

2. Results

As shown in Fig. 33, some of the breast cancer cell lines treated with dispase were difficult to separate from the lymphocytic cells, whereas the breast cancer cell lines treated with thrombin were easily separated. It was indicated that maintenance of cell nuclear morphology was important for separation of cell nuclei.

<Test 19>

Distribution of Breast Cancer Tissue-Derived Cell Nuclei or Lymphocyte-Derived Cell Nuclei and Sorting Based Thereon When FFPE Tissue Section Are Treated with Different Enzymes

Breast cancer and normal lymph node FFPE tissues were subjected to pretreatment including treatments with different enzymes, nucleic acid was stained, the treated product was then analyzed by a flow cytometer, and distributions on FSC-SSC scattergrams were compared.

1. Materials and Method

1-1. FFPE Tissue Block

Breast cancer and normal lymph node FFPE tissues purchased from ProteoGenex, Inc were used as measurement samples.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 1 was carried out.

1-5A. Enzymatic Treatment (Thrombin)

The same procedure as in test 8 was carried out.

1-5B. Enzymatic Treatment (Dispase)

The same procedure as in test 8 was carried out.

1-6. Water-Flow Crushing and Ultrasonic Crushing

The same procedure as in test 7 was carried out.

1-7. Nucleic Acid-Staining

The same procedure as in Test 2 was carried out.

1-8. Flow Cytometer Measurement

The same procedure as in Test 1 was carried out.

1-9. Data Analysis

As in Test 1, populations were gated, and the gated populations were developed on scattergrams with the forward scatter (FSC) in the horizontal axis and the side scatter (SSC) in the vertical axis. For comparing effects of different enzymes used for the treatment, the scattergram for the normal lymph node FFPE tissue was superimposed on the scattergram for the breast cancer FFPE tissue.

2. Results

As shown in Fig. 34, cancer cell nuclei and lymphocytic cell nuclei were difficult to separate when the tissue was treated with dispase, whereas the cell nuclei were separated when the tissue was treated with thrombin.
<Test 20>

Comparative Examination of Ki-67-Positive Ratios with and without Gating of Cancer Cell-Derived Cell Nucleus Region and Positive Ratio by IHC Method by Pathologist, and Agreement

Ratio

A FFPE tissue section was subjected to pretreatment, immuno-fluorescently stained with a Ki-67 antibody, and then measured by a flow cytometer. Data analysis was performed to examine a correlation between positive ratios obtained with and without gating of the cancer cell-derived cell nucleus region and a positive ratio by the IHC method by a pathologist. In addition, positive and negative agreement ratios were examined by statistical analysis.

1. Materials and Method

1-1. FFPE Tissue Block

49 FFPE tissue blocks obtained from 49 patients with different Ki-67-positive ratios from the IHC method and purchased from ProteoGenex, Inc were used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 1 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Immuno-Fluorescent Staining

4% BSA/TBS containing 10% normal goat serum (Wako) was added, and the mixture was placed still at room temperature for 30 minutes and subjected to blocking treatment. Immunofluorescent staining was performed with a Ki-67 antibody (Agilent, clone: MIB-1, mouse monoclonal antibody) as a primary antibody, and Goat anti-Mouse IgG (H+L) Cross-Adsorbed Secondary Antibody Alexa Fluor 647 from Thermo Fisher Scientific as a secondary antibody. The primary antibody reaction was carried out at 37°C for 15 minutes, and the secondary antibody reaction was carried out at room temperature for 15 minutes. DAPI Solution (Wako) was added to stain cell nuclei 10 minutes after the addition of the secondary antibody. The reactions were carried out under a light-shielding condition after addition of the secondary antibody. 0.5% BSA/TBS was used as an antibody diluting liquid, and washing with 0.5% BSA/TBS was carried out three times between the steps. Instead of the primary antibody, a corresponding antibody identical in type and concentration to the primary antibody was used as an isotype matched control.

1-8. Flow Cytometer Measurement

The same procedure as in Test 1 was carried out.

1-9. Calculation of Ki-67-positive Ratio by Flow Cytometer

Analysis was performed using FlowJo v10.6.2. A main region of 2n or more nuclear chromosomes was gated in a histogram with the fluorescence intensity of DAPI-A in the horizontal axis and the number of particles in the vertical axis. Subsequently, for the gated population, a scattergram with FSC-H (forward scatter) in the horizontal axis and SSC-H (side scatter) in the vertical axis was developed, and a region with a FSC-H intensity of $2.00 \times 10^6$ to $5.00 \times 10^6$ and a SSC-H intensity of $1.00 \times 10^6$ to $2.00 \times 10^6$ was gated as a cancer cell nucleus region. For the population gated as a cancer cell nucleus region and the population without gating, histograms with the fluorescence intensity (corresponding to the amount of Ki-67) in the horizontal axis and the number of particles in the vertical axis were prepared. On each of the histograms, a histogram of an isotype matched control was superimposed. The threshold value of Ki-67-positive nuclei was set to the 95 percentile value of the isotype matched control. The Ki-67-positive ratio was calculated as a ratio of the number of Ki-67-positive nuclei with respect to the number of all cell nuclei. A value obtained by subtracting 5% from the obtained positive ratio was used because the cut-off of positive nuclei was set to the 95 percentile value of the isotype matched control.

1-10. Calculation of Ki-67-Positive Ratio by IHC Method

The Ki-67-positive ratio by the IHC method was calculated by a hotspot method with a Ki-67 antibody (Agilent, clone: MIB-1, mouse monoclonal antibody) as a primary antibody.

1-11. Statistical Analysis

Statistical analysis was performed using MedCalc v19.4.1 from MedCalc Software. The results by the IHC method were binarized with the cut-offs of Ki-67-positive and negative cell nuclei set to 20%, and ROC analysis was performed on the obtained values together with results with or without gating in the flow cytometer to calculate AUC.

2. Results

Fig. 35 shows correlations with the IHC method for the Ki-67-positive ratio. A graph, where the X-axis represents the IHC method and the Y-axis represents the present method by the flow cytometer, was prepared for examining the correlation. As shown in Fig. 35, the results of $y = 0.80x - 2.39$ and $R = 0.9126$ were obtained with gating of the region of cancer-derived cell nuclei, whereas the results of $y = 0.54x - 1.40$ and $R = 0.8874$ were obtained without the gating. Thus, it was confirmed that gating improved the gradient and the correlation coefficient. Fig. 36 shows the results of performing ROC analysis in a graph obtained by plotting 100 - specificity (%) on the X-axis and sensitivity (%) on the Y-axis. As shown in the table below, the result of AUC = 0.959 was obtained with gating, and the result of AUC = 0.899 was obtained without gating. Thus, it was also confirmed that there was a statistical difference between the results with and without gating ($p = 0.0442$, 95% confidence interval: 0.00156-0.118).

[Table 9]

| | AUC | 95% Confidence interval |
|---|---|---|
| Gated | 0. 959 | 0. 860-0. 995 |
| Without gating | 0. 899 | 0. 779-0. 967 |

From the above, it was confirmed that by gating, cancer cell nuclei were concentrated to improve the correlation with the IHC method and the positive/negative agreement, so that it was possible to accurately detect Ki-67 in cancer cell nuclei.

<Test 21>

Examination of ER- and PgR-Positive Ratios with and without Gating of Cancer Cell-Derived Cell Nucleus Region

A FFPE tissue section was subjected to pretreatment, immuno-fluorescently stained with an ER antibody and a PgR antibody, and then measured by a flow cytometer. Data analysis was performed to compare positive ratios with and without gating of the cancer cell-derived cell nucleus region.

1. Materials and Method

1-1. FFPE Tissue Block

ER-positive specimen A (Allred Score ER8) and PgR-positive specimen B (Allred Score PgR8) purchased from ProteoGenex, Inc were used as ER- and PgR-positive samples, and FFPE tissue sections of ER- and PgR-negative specimens C (Allred Score ER0 and PgR0) were used as ER- and PgR-negative samples.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Immuno-Fluorescent Staining

4% BSA/TBS containing 10% normal goat serum (Wako) was added, and the mixture was placed still at room temperature for 30 minutes and subjected to blocking treatment. Immunofluorescent staining was performed with an ER antibody (Agilent, clone: EP1, rabbit monoclonal antibody) or a PgR antibody (Agilent, clone: PgR636, mouse monoclonal antibody) as a primary antibody and Goat anti-Rabbit IgG (H+L) Cross-Adsorbed Secondary Antibody Alexa Fluor 488 and Goat anti-Mouse IgG (H+L) Cross-Adsorbed Secondary Antibody Alexa Fluor 647 from Thermo Fisher Scientific as secondary antibodies. The primary antibody reaction was carried out at 37°C for 15 minutes, and the secondary antibody reaction was carried out at room temperature for 15 minutes. DAPI Solution (Wako) was added to stain cell nuclei 10 minutes after the addition of the secondary antibody. The reactions were carried out under a light-shielding condition after addition of the secondary antibody. 0.5% BSA/TBS was used as an antibody diluting liquid, and washing with 0.5% BSA/TBS was carried out three times between the steps. Instead of the primary antibody, a corresponding antibody identical in type and concentration to the primary antibody was used as an isotype matched control. A rabbit IgG antibody from Cell Signaling Technology was used as the ER antibody, and a mouse IgG antibody from Agilent Technology was used as the PgR antibody.

1-8. Flow Cytometer Measurement

The same procedure as in Test 1 was carried out.

1-9. Calculation of ER- and PgR-Positive Ratios

Analysis was performed using FlowJo v10.6.2. A main region of 2n or more nuclear chromosomes was gated in a histogram with the fluorescence intensity of DAPI-A in the horizontal axis and the number of particles in the vertical axis. For the gated population, a scattergram with FSC-H (forward scatter) in the horizontal axis and SSC-H (side scatter) in the vertical axis was developed, and a region with a FSC-H intensity of $2.00 \times 10^6$ to $5.00 \times 10^6$ and a SSC-H intensity of $1.00 \times 10^6$ to $2.00 \times 10^6$ was gated as a cancer cell nucleus region. For the population gated as a cancer cell nucleus region or the population without gating, histograms with the fluorescence intensity (corresponding to the amount of ER or PgR) in the horizontal axis and the number of particles in the vertical axis were prepared. On each of the histograms, a histogram of an isotype matched control was superimposed (Figs. 37 and 38). The threshold values of ER- and PgR-positive nuclei were each set to the 95 percentile value of the isotype matched control. The ER- and PgR-positive ratios were calculated as ratios of the numbers of ER-positive nuclei and PgR-positive nuclei, respectively, with respect to the numbers of all cell nuclei. The positive ratios without gating of the cancer cell nucleus region were similarly calculated. Values obtained by subtracting 5% from the positive ratios were used because the cut-off of positive nuclei was set to the 95 percentile value of the isotype matched control.

2. Results

As shown in Figs. 37 and 38, the ER-positive ratio and the PgR-positive ratio were both improved by gating cancer-derived cell nucleus regions.

<Test 22>

Examination of Cytokeratin Subtype-Positive Ratios with and without Gating of Cancer Cell-Derived Cell Nucleus Regions

A FFPE tissue section was subjected to pretreatment, immuno-fluorescently stained with cytokeratin 7 and 20 antibodies, and then measured by a flow cytometer. Data analysis was performed to compare positive ratios with and without gating of the cancer cell-derived cell nucleus region.

1. Materials and Method

1-1. FFPE Tissue Block

A breast cancer FFPE tissue block purchased from ProteoGenex, Inc was used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Immunofluorescent Staining

4% BSA/TBS containing 10% normal goat serum (Wako) was added, and the mixture was placed still at room temperature for 30 minutes and subjected to blocking treatment. Immunofluorescent staining was performed by double staining with a cytokeratin 7 antibody (Abcam, rabbit monoclonal antibody) and a cytokeratin 20 antibody (Abcam, mouse monoclonal antibody) as primary antibodies and Goat anti-Rabbit IgG (H+L) Cross-Adsorbed Secondary Antibody Alexa Fluor 488 and Goat anti-Mouse IgG (H+L) Cross-Adsorbed Secondary Antibody Alexa Fluor 647 from Thermo Fisher Scientific as secondary antibodies. The primary antibody reaction was carried out at 37°C for 15 minutes, and the secondary antibody reaction was carried out at room temperature for 15 minutes. DAPI Solution (Wako) was added to stain cell nuclei 10 minutes after the addition of the secondary antibody. The reactions were carried out under a light-shielding condition after addition of the secondary antibody. 0.5% BSA/TBS was used as an antibody diluting liquid, and washing with 0.5% BSA/TBS was carried out three times between the steps. Instead of the primary antibodies, corresponding antibodies identical in type to the primary antibodies were used as isotype matched controls. A rabbit IgG antibody from Cell Signaling Technology was used as the cytokeratin 7 antibody, and a mouse IgG antibody from Agilent Technology was used as the cytokeratin 20 antibody.

1-8. Flow Cytometer Measurement

The same procedure as in Test 1 was carried out.

1-9. Calculation of Cytokeratin Subtype-Positive Ratios

Analysis was performed using FlowJo v10.6.2. A main region of 2n or more nuclear chromosomes was gated in a diagram with the fluorescence intensity of DAPI-A in the horizontal axis and the number of particles in the vertical axis. For the gated population, a scattergram with FSC-H (forward scatter) in the horizontal axis and SSC-H (side scatter) in the vertical axis was developed, and a region with a FSC-H intensity of $2.00 \times 10^6$ to $5.00 \times 10^6$ and a SSC-H intensity of $1.00 \times 10^6$ to $2.00 \times 10^6$ was gated as a cancer specific region. For the population gated as a cancer cell nucleus region or the population without gating, histograms with the fluorescence intensity (corresponding to the amount of cytokeratin 7 or 20) in the horizontal axis and the number of particles in the vertical axis were prepared. On each of the histograms, a histogram of an isotype matched control was superimposed (Fig. 39). The threshold values of cytokeratin 7 and 20-positive nuclei were each set to the 95 percentile value of the isotype matched control. The cytokeratin 7 and 20-positive ratios were calculated as ratios of the numbers of cytokeratin 7 and 20-positive nuclei, respectively, with respect to the numbers of all cell nuclei. The positive ratios without gating of the cancer specific region were similarly calculated.

2. Results

As shown in Fig. 39, the cytokeratin 7-positive ratio was improved by gating the cancer cell nucleus region. On the other hand, the cells were kept cytokeratin 20- negative regardless of whether gating was performed or not. It is known that cells in breast cancer are cytokeratin 7-positive and cytokeratin 20-negative. The present method was confirmed to give comparative results for cytokeratin 20, and enable improvement of the cytokeratin 7-positive ratio.

<Test 23>

Examination of Concentration of Lymphocyte-Derived Cell Nuclei and Normal Cell-derived Cell Nuclei by Gating Specific Region

FFPE tissue sections of a cancer part, a non-cancerous part (normal cells) and a normal lymph node derived from one patient with breast cancer were subjected to a predetermined pretreatment, nucleic acid was stained, the treated product was then analyzed by a flow cytometer, and proportions of lymphocyte-derived cell nuclei and normal cell-derived cell nuclei in a specific region on a FSC-SSC scattergram were examined.

1. Materials and Method

1-1. FFPE Tissue Block

FFPE tissue blocks of a cancer part, a non-cancerous part and a normal lymph node derived from one patient with breast cancer and purchased from ProteoGenex, Inc were used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Nucleic Acid-Staining

The same procedure as in Test 2 was carried out.

1-8. Flow Cytometer Measurement

The same procedure as in Test 1 was carried out.

1-9. Data Analysis

Analysis was performed using FlowJo v10.6.2. A main region of 2n or more nuclear chromosomes was gated in a diagram with the fluorescence intensity of DAPI-A in the horizontal axis and the number of particles in the vertical axis. Subsequently, the gated population was developed on a scattergram with FSC-H (forward scatter) in the horizontal axis and SSC-H (side scatter) in the vertical axis. Fig. 40 shows scattergrams of nuclei derived from cells of the cancer part tissue, nuclei derived from cells of the non-cancerous part tissue and nuclei derived from cells of the normal lymph node, and Fig. 41 shows a diagram obtained by overlaying the scattergrams. The two regions of areas 3 and 4 shown in Fig. 41 were gated, and proportions of the cell nuclei of respective types were calculated.

2. Results

The proportions of the cell nuclei of respective types in areas 3 and 4 are shown below.

[Table 10]

| | Lymphocyte-derived cell nuclei | Normal cell-derived cell nuclei | Cancer cell-derived cell nuclei |
|---|---|---|---|
| Area 3 | **-** | **4. 7%** | **3. 3%** |
| Area 4 | **0. 6%** | **-** | **1. 9 %** |

The proportions of normal cell nuclei and cancer cell nuclei present in Area 3 are 4.7% and 3.3%, respectively. In Area 3, lymphocyte-derived cell nuclei were concentrated. The proportions of lymphocyte-derived cell nuclei and cancer cell nuclei present in Area 4 are 0.6% and 1.9%, respectively. In Area 4, normal cell nuclei were concentrated. Thus, it was shown that it was possible to separate and concentrate lymphocyte-derived cell nuclei and normal cell nuclei by gating areas 3 and 4.

<Test 24>

Examination of Ki-67-Positive Ratios with and without gating of Cancer-Derived Cell Nucleus Region in Bowel Cancer Case

Bowel cancer FFPE tissue sections were subjected to predetermined pretreatment, immuno-fluorescently stained with a Ki-67 antibody, and then measured by a flow cytometer. Data analysis was performed to compare positive ratios with and without gating of the cancer cell-derived cell nucleus region.

1. Materials and Method

1-1. FFPE Tissue Block

Bowel FFPE tissue blocks (case 1: sigmoid, case 2: rectum) purchased from Proteogenex, Inc were used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Immunofluorescent Staining

The tissues after physical crushing were immuno-fluorescently stained in the same manner as in Test 20.

1-8. Flow Cytometer Measurement

The same procedure as in Test 1 was carried out.

1-9. Calculation of Ki-67-Positive Ratio by Flow Cytometer

Analysis was performed using FlowJo v10.6.2. A main region of 2n or more nuclear chromosomes was gated in a diagram with the fluorescence intensity of DAPI-A in the horizontal axis and the number of particles in the vertical axis. Subsequently, a scattergram with FSC-H (forward scatter) in the horizontal axis and SSC-H (side scatter) in the vertical axis was developed, and a region with a FSC-H intensity of $2.00 \times 10^6$ to $5.00 \times 10^6$ and a SSC-H intensity of $1.00 \times 10^6$ to $2.00 \times 10^6$ was gated as a cancer cell nucleus region. The Ki-67-positive ratio was calculated as a ratio of the number of Ki-67-positive nuclei with respect to the number of all cell nuclei. The threshold value of positive nuclei was set to the 95 percentile value of the isotype matched control. The positive ratio without gating of the cancer cell nucleus region was similarly calculated.

2. Results

Fig. 42 shows histograms with the fluorescence intensity (corresponding to the amount of Ki-67) in the horizontal axis and the number of particles in the vertical axis for populations gated as cancer cell nucleus regions or populations without gating in the tissues in the respective cases, where on each of the histograms, a histogram of an isotype matched control is superimposed.

As shown in Fig. 42, in both cases, the Ki-67-positive ratio was improved by gating the cancer cell nucleus region. Thus, concentration by gating the cancer cell nucleus region was shown to be also effective for cancers other than breast cancer.

<Test 25>

Examination of Cytokeratin Subtype-Positive Ratios with and without Gating of Cancer-Derived Cell Nucleus Region in Bowel Cancer Case

Bowel cancer FFPE tissue sections were subjected to predetermined pretreatment, immuno-fluorescently stained with cytokeratin 7 and 20 antibodies, and then measured by a flow cytometer. Data analysis was performed to compare positive ratios from with and without gating of the cancer cell-derived cell nucleus region.

1. Materials and Method

1-1. FFPE Tissue Block

Bowel cancer FFPE tissue blocks purchased from Proteogenex, Inc were used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Immunofluorescent Staining

The tissues after physical crushing were immuno-fluorescently stained in the same manner as in Test 22.

1-8. Flow Cytometer Measurement

The same procedure as in Test 1 was carried out.

1-9. Calculation of Cytokeratin Subtype-Positive Ratio

The same procedure as in Test 22 was carried out.

2. Results

Fig. 43 shows histograms with the fluorescence intensity (corresponding to the amount of cytokeratin 20 or 7) in the horizontal axis and the number of particles in the vertical axis for populations gated as cancer cell nucleus regions or populations without gating, where on each of the histograms, a histogram of an isotype matched control is superimposed.

As shown in Fig. 43, the cytokeratin 20-positive ratio was improved by gating the cancer cell nucleus region. On the other hand, the cells were kept cytokeratin 7-negative regardless of whether gating was performed or not. It is known that cells in bowel cancer are cytokeratin 20-positive and cytokeratin 7-negative. The present method was confirmed to give comparative results for cytokeratin 7, and enable improvement of the cytokeratin 20-positive ratio. Thus, concentration by gating the cancer cell nucleus region was shown to be also effective for cancers other than breast cancer.

<Test 26>

Examination of detection of genetic mutation by Fraction of Concentrated Cancer Cell Nuclei

FFPE tissue sections of a cancer part and a non-cancerous part (normal cells) derived from a patient with bowel cancer having KRAS gene mutation were subjected to predetermined pretreatment, and then mixed to prepare a sample having a low cancer proportion. Subsequently, the cancer cell nuclei were concentrated by a cell sorter, and real time PCR was used to detect KRAS gene mutation.

1. Materials and Method

1-1. FFPE Tissue Block

FFPE tissue blocks of a cancer part and a non-cancerous part derived from one patient with bowel cancer having $KRAS^{G12D}$ mutation purchased from ProteoGenex, Inc were used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Measurement of Concentration of Particles

The suspension liquid obtained by crushing was filtered through a 35 μm filter (380 meshes) (for flow cytometer) for Falcon (registered trademark) Cell Strainer 5 mL Tube, followed by measurement with a laser intensity gain value (FSC: 400, SSC: 400) using a flow cytometer (Agilent: NovoCyte Quanteon Flow Cytometer), whereby the concentration of particles (number of particles/mL) was determined.

1-8. Preparation of Samples Having Different Cancer Proportions

Cancer part- and non-cancerous part-derived suspension liquids of which particle concentrations had been measured were mixed as necessary to prepare suspension liquids in which the proportion of cells from the cancer part was 10% or 100% and the total number of particles was 100,000.

1-9. Fractionation by Cell Sorter

The samples having different proportions of particles from the cancer part were measured using FACSAriaII from Becton Dickinson and Co (FSC laser intensity voltage value: 65, SSC laser intensity voltage value: 320). A scattergram with FSC in the horizontal axis and SSC in the vertical axis was prepared, about 30,000 cells were fractionated from a region identical to Area 1 of Fig. 30.

1-10. Extraction and Purification of DNA

Using QIAamp DNA FFPE Tissue Kit from QUIAGEN N.V., DNA was extracted from samples of cells fractionated by the cell sorter and samples of non- fractionated cells, and the DAN was purified. The concentration of the purified DNA was calculated from an absorbance at 260 nm using a micro spectrophotometer DS-11+ manufactured by Scrum Inc.

1-11. Detection of Amplification by Real Time PCR

PCR was performed on the purified DNA using the following reagents and Step One Plus (registered trademark) Real Time PCR System from ThermoFisher Scientific.

Composition of Mixed Reagent Solution for Real Time PCR

- TB Green (registered trademark) Premix DimerEraser: 10 μL
- Forward primer for detection of KRAS (20 μM: BNA Clamp KRAS Enrichment Kit Gly 12/Gly 13): 0.6 μL
- Reverse primer for detection of KRAS (20 μM: BNA Clamp KRAS Enrichment Kit Gly 12/Gly 13): 0.6 μL
- ROX: 0.4 μL
- BNA (BNA Clamp KRAS Enrichment Kit Gly 12/Gly 13): 0.2 μL
- Template DNA: 10 ng
- Water: amount necessary for increasing the total volume to 20 μL

A cycle of reaction, in which the reaction solution is exposed to a first temperature of 95°C for 20 seconds (degeneration reaction), a second temperature of 58°C for 30 seconds (annealing reaction) and a third temperature of 72°C for 45 seconds (extension reaction) once, was carried out 50 times, and fluorescence was detected.

2. Results

Fig. 44 shows graphs showing difference in PCR amplification rate between cells fractionated from Area 1 and non-fractionated cells using samples containing cells from a cancer region in different proportions. The horizontal axis represents the number of heat cycles, and the vertical axis represents the fluorescent intensity measured by the measuring apparatus. CT values are also shown below.

[Table 11]

| CT value | | Isolated (concentrated) | Non-isolated (not concentrated) |
|---|---|---|---|
| | Cancer proportion 100% | 30.75 | 31.21 |
| | Cancer proportion 10% | 32.98 | 35.91 |

When the cancer proportion was as low as 10%, fractionation of the cancer cell nucleus region made amplification faster by about 3 cycles. On the other hand, when the cancer proportion was 100%, there was no change in amplification rate. Thus, it is indicated that DNA derived from cancer cells was also concentrated by fractionating the cancer cell nucleus region.

<Test 27>

Examination of DNA Aneuploidy Peak Detections with and without Gating of Cancer Cell-Derived Cell Nucleus Regions

A FFPE tissue section was subjected to pretreatment, nucleic acid was stained, and the treated product was then tested by a flow cytometer. Data analysis was performed to compare DNA aneuploidy peak detections with and without gating of the cancer cell-derived cell nucleus region.

1. Materials and Method

1-1. FFPE Tissue Block

Breast cancer FFPE tissue blocks (n=3) purchased from ProteoGenex, Inc were used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5. Enzymatic Treatment (Thrombin)

The same procedure as in test 8 was carried out.

1-6. Water-Flow Crushing and Ultrasonic Crushing

The same procedure as in test 7 was carried out.

1-7. Nucleic Acid-Staining

The same procedure as in Test 2 was carried out.

1-8. Flow Cytometer Measurement

The same procedure as in Test 1 was carried out.

1-9. Data Analysis

Analysis was performed using FlowJo v10.6.2. Data analysis was performed by the following procedure (Fig. 45). A main region of 2n or more nuclear chromosomes was gated in a diagram with the fluorescence intensity of DAPI-A in the horizontal axis and the number of particles in the vertical axis (Fig. 45(a)). Next, a scattergram with DAPI-H in the horizontal axis and DAPI-A in the vertical axis was prepared, and the doublets were removed (Fig. 45(b)). Subsequently, a scattergram with FSC-H in the horizontal axis and SSC-H in the vertical axis was developed, and a region with a FSC-H intensity of $2.00 \times 10^6$ to $5.00 \times 10^6$ and a SSC-H intensity of $1.00 \times 10^6$ to $2.00 \times 10^6$ was gated as a cancer cell nucleus region (Fig. 45(c)). For the population gated as a cancer cell nucleus region and the population without gating, a histogram was prepared in which the horizontal axis represents the fluorescence intensity of DAPI-A and the vertical axis represents a value normalized so that the maximum number of particles is 100 (n=3, Figs. 46(a), 46(b) and 46(c)).

2. Results

As shown in Fig. 46, the DNA aneuploidy peak was intensified by gating the cancer cell nucleus region. Thus, concentration by gating the cancer cell nucleus region was shown to be also effective for detection of the DNA aneuploidy peak.

<Test 28> Comparison Examination of DNA Index Calculated from DNA Aneuploidy Peak and DNA Index Calculated from DNA Aneuploidy Peak Detected by Cytokeratin and Vimentin Staining after Gating of Cancer Cell-Derived Cell Nucleus Region

A correlation between a DNA index calculated with DNA aneuploidy peaks obtained in the same manner as in Test 27 except that the number of specimens was 17 and a DNA index calculated from a DNA aneuploidy peak detected by cytokeratin and vimentin staining after gating of a cancer cell-derived cell nucleus region was examined.

1. Materials and Method

1-1. FFPE Tissue Block

A breast cancer FFPE tissue block purchased from ProteoGenex, Inc was used.

1-2. Preparation of FFPE Section

The same procedure as in Test 2 was carried out.

1-3. Deparaffinization/Hydrophilization

The same procedure as in Test 2 was carried out.

1-4. Heating

The same procedure as in Test 2 was carried out.

1-5A. Enzymatic Treatment (Thrombin), Water-Flow Crushing and Ultrasonic Crushing, and Nucleic Acid-Staining

The same procedures as in tests 8, 7 and 2 were carried out, respectively.

1-5B. Conventional Methods (Enzymatic Treatment (Collagenase), Immunofluorescent Staining)

A collagenase reagent (RPMI 1640, 3,000 PU/mL dispase, 2,500 U/mL collagenase type III, 10 mg/mL RNase I) was added to the sample after the heating treatment, and the mixture was heated on a heat block at 37°C for 20 minutes. Subsequently, 4% BSA/TBS containing 10% normal goat serum (Wako) was added, and the mixture was placed still at room temperature for 30 minutes and subjected to blocking treatment. Immunofluorescent staining was performed by double staining with a cytokeratin antibody (Abcam, rabbit monoclonal antibody) and a vimentin antibody (Abcam, mouse monoclonal antibody) as primary antibodies and Goat anti-Rabbit IgG (H+L) Cross-Adsorbed Secondary Antibody Alexa Fluor 488 and Goat anti-Mouse IgG (H+L) Cross-Adsorbed Secondary Antibody Alexa Fluor 647 from Thermo Fisher Scientific as secondary antibodies. The primary antibody reaction was carried out at 37°C for 15 minutes, and the secondary antibody reaction was carried out at room temperature for 15 minutes. DAPI Solution (Wako) was added to stain cell nuclei 10 minutes after the addition of the secondary antibody. The reactions were carried out under a light-shielding condition after addition of the secondary antibody. 0.5% BSA/TBS was used as an antibody diluting liquid, and washing with 0.5% BSA/TBS was carried out three times between the steps. Instead of the primary antibodies, corresponding antibodies identical in type to the primary antibodies were used as isotype matched controls. A rabbit IgG antibody from Cell Signaling Technology was used as the cytokeratin antibody, and a mouse IgG antibody from Agilent Technology was used as the vimentin antibody.

1-6. Flow Cytometer Measurement

The same procedure as in Test 1 was carried out.

1-7. Data Analysis

As illustrated in Fig. 47, a histogram prepared in the same manner as in Test 27 except that the number of specimens was 17 was used to identify a modal value of the fluorescence intensity of DAPI-A for the population gated as a cancer cell nucleus region or the population without gating. The modal value for the population without gating was regarded as an amount of DNA in a region of 2n nuclear chromosomes, and the ratio between the modal value for the population without gating and the modal value for the cancer cell nucleus region (modal value for gated population/modal value for population without gating) was calculated, and defined as a DNA index.

In the conventional method, gating of a main region of 2n or more nuclear chromosomes and removal of doublets were similarly performed. Subsequently, a scattergram with the fluorescence intensity of cytokeratin in the horizontal axis and the fluorescence intensity of vimentin in the vertical axis was developed. Cytokeratin-positive and vimentin-negative regions were gated as cancer cell regions, and cytokeratin-negative and vimentin-positive regions were gated as interstitial cell regions. For each of the gated populations, a modal value of the fluorescence intensity of DAPI-A was acquired, and the ratio of the modal value for the cancer cell region to the modal value for the interstitial cell region (modal value for cancer cell region/modal value for interstitial region) was calculated, and defined as a DNA index.

2. Results

Fig. 48 shows a correlation between the calculated DNA indices (n=17). The correlation was examined using a graph in which the X-axis represents the conventional method and the Y-axis represents the present method. As shown in Fig. 48, a high correlation of R = 0.9407 was obtained. From the above, it was confirmed that the present method enabled calculation of a DNA index.

## Claims

1. A method for obtaining a sample enriched with cells or cell nuclei of interest from a piece of tissue, the method comprising:

   performing pretreatment including subjecting the piece of tissue to treatment with a hydrolase which does not degrade a protein necessary for maintaining cell nuclear morphology, wherein the hydrolase is thrombin, proline endopeptidase or hyaluronidase, and water-flow crushing and/or ultrasonic crushing to obtain a population of particles including individually separated cells or cell nuclei, and

   visualizing components in cell nuclei of the population of particles obtained in the pretreatment, and then sorting or fractionating by flow cytometry the population of particles to obtain at least a fraction of the population of particles significantly abundant in the cells or cell nuclei of interest based on optical characteristics of cell nuclei in the separated cells or the separated cell nuclei.

2. The method according to claim 1, wherein the water-flow crushing is performed by rotating a crushing member at a speed of 6,000 to 13,000 rpm and/or wherein the ultrasonic crushing is performed with an amplitude of 20 to 40%, for example for 30 seconds to 3 minutes.

3. The method according to any one of claims 1 to 2, wherein the cells of interest are tumor cells, lymphocytes, or normal cells derived from a non-cancerous part of tissue.

4. The method according to any one of claims 1 to 3, wherein the piece of tissue is fixed with a fixing agent, for example wherein the fixed piece of tissue is embedded in an embedding agent.

5. The method according to claim 4, wherein the fixed piece of tissue is embedded in an embedding agent before the pretreatment, the embedding agent is removed from the piece of tissue, and the piece of tissue is hydrophilized.

6. The method according to any one of claims 4 to 5, wherein before the pretreatment, a crosslinked structure formed by the fixation is destroyed by heat treatment.

7. The method according to any one of claims 1 to 6, wherein the population of particles obtained through the pretreatment is analyzed by flow cytometry to sort or fractionate a population of particles abundant in the cells or cell nuclei of interest based on forward scatter (FSC) intensity and/or side scatter (SSC) intensity in a scattergram obtained in the analysis.

8. The method according to claim 7, wherein

a plurality of known cell types are analyzed by flow cytometry to generate a histogram of forward scatter (FSC) intensity or side scatter (SSC) intensity and the number of particles, or a scattergram of forward scatter (FSC) intensity and side scatter (SSC) intensity for cell nuclei derived from each cell type, and an area or region more abundant in nuclei of each cell type than in nuclei of other cell types is determined based on the forward scatter (FSC) intensity and/or side scatter (SSC) intensity; and
a treated product after the pretreatment is measured by flow cytometry to generate a histogram or scattergram in the same manner, and in the histogram or scattergram, a population of particles present in the area or region predetermined for cell nuclei identical in type to the cell nuclei of interest is sorted or fractionated.

9. The method according to claim 8, wherein:

standard particles having different known particle sizes are analyzed by flow cytometry to generate a calibration curve from forward scatter (FSC) intensity obtained by the analysis and the known particle sizes of the standard substances;
a treated product after the pretreatment is analyzed by flow cytometry, and forward scatter (FSC) intensity obtained by the analysis is applied to the calibration curve to calculate a particle size, and
a population of particles having predetermined particle sizes is sorted or fractionated; and preferably:

wherein a region composed of a population of particles having particle sizes of 10 μm or more, 95% or more of which have particle sizes of 11 μm to 20 μm, is selected to sort or fractionate a population of particles enriched with particles derived from tumor cells; or
wherein a region composed of a population of particles having particle sizes of 8 μm or less, 95% or more of which have particle sizes of 4 μm to 7 μm, is selected to sort or fractionate a population of particles enriched with particles derived from lymphocytes.

10. A method for analyzing biomarkers present in cells or cell nuclei of interest using a sample enriched with the cells and cell nuclei obtained by the method according to any one of claims 1 to 9.

11. The method according to claim 10, wherein the cells of interest are tumor cells, and/or wherein the biomarker is a polynucleotide, a nuclear protein, a nuclear membrane protein or a cytoskeletal protein, wherein preferably the polynucleotide comprises a cancer-related gene, and the nuclear protein comprises a transcription factor or a cell cycle-related protein.

**12.** A method of determining a DNA index, comprising:

obtaining a sample enriched with tumor cells or cell nuclei thereof (tumor cell nuclei-enriched sample) from a piece of tissue by the method according to any one of claims 7 to 9,
obtaining a sample which is not enriched with tumor cells or cell nuclei thereof (tumor cell nucleus-non-enriched sample or normal sample) from the same piece of tissue or a piece of normal tissue in the same manner except that the step of sorting or fractionating a population of particles significantly abundant in tumor cells or cell nuclei thereof is not carried out, and
preparing a histogram of the number of particles and the amount of DNA for each sample, identifying an amount of DNA at a largest number of particles, and determining a DNA index from the following equation:

DNA index = amount of DNA at largest number of particles in tumor cell nucleus- enriched sample / amount of DNA at largest number of particles in tumor cell nucleus-non- enriched sample or normal cell sample.

**Patentansprüche**

**1.** Verfahren zur Gewinnung einer Probe, die mit Zellen oder Zellkernen von Interesse angereichert ist, aus einem Gewebestück, wobei das Verfahren umfasst:

Durchführen einer Vorbehandlung, die beinhaltet, das Gewebestück einer Behandlung mit einer Hydrolase zu unterziehen, die ein für den Erhalt der Zellkernmorphologie notwendiges Protein nicht abbaut, wobei die Hydrolase Thrombin, Prolin-Endopeptidase oder Hyaluronidase ist, und Wasserfluss-Zerkleinerung ("water-flow crushing") und/oder
Ultraschallzerkleinerung, um eine Partikelpopulation zu erhalten, die einzeln getrennte Zellen oder Zellkerne beinhaltet, und
Visualisieren von Komponenten innerhalb der Zellkerne der in der Vorbehandlung erhaltenen Partikelpopulation und anschließend das Sortieren oder Fraktionieren der Partikelpopulation mittels Durchflusszytometrie, um mindestens eine Fraktion der Partikelpopulation, die in den Zellen oder Zellkernen von Interesse signifikant reichlich vorhanden ist, auf der Grundlage der optischen Eigenschaften der Zellkerne in den getrennten Zellen oder der getrennten Zellkerne, zu erhalten.

**2.** Verfahren nach Anspruch 1, wobei die Wasserfluss-Zerkleinerung durch Drehen eines Zerkleinerungselements ("crushing member") mit einer Geschwindigkeit von 6.000 bis 13.000 rpm durchgeführt wird und/oder wobei die Ultraschallzerkleinerung mit einer Amplitude von 20 bis 40 %, beispielsweise für 30 Sekunden bis 3 Minuten, durchgeführt wird.

**3.** Verfahren nach irgendeinem der Ansprüche 1 bis 2, wobei die Zellen von Interesse Tumorzellen, Lymphozyten oder aus einem nicht-krebsartigen Gewebeteil stammenden normale Zellen sind.

**4.** Verfahren nach irgendeinem der Ansprüche 1 bis 3, wobei das Gewebestück mit einem Fixiermittel fixiert wird, wobei beispielsweise das fixierte Gewebestück in ein Einbettungsmittel eingebettet wird.

**5.** Verfahren nach Anspruch 4, wobei das fixierte Gewebestück vor der Vorbehandlung in ein Einbettungsmittel eingebettet wird, das Einbettungsmittel von dem Gewebestück entfernt wird und das Gewebestück hydrophilisiert wird.

**6.** Verfahren nach irgendeinem der Ansprüche 4 bis 5, wobei vor der Vorbehandlung eine durch die Fixierung gebildete vernetzte Struktur durch Wärmebehandlung zerstört wird.

**7.** Verfahren nach irgendeinem der Ansprüche 1 bis 6, wobei die durch die Vorbehandlung erhaltene Partikelpopulation mittels Durchflusszytometrie analysiert wird, um eine Partikelpopulation, die in den Zellen oder Zellkernen von Interesse reichlich vorhanden ist, auf der Grundlage der Intensität der Vorwärtsstreuung (FSC) und/oder der Intensität der Seitenstreuung (SSC) in einem in der Analyse erhaltenen Streudiagramm zu sortieren oder zu fraktionieren.

**8.** Verfahren nach Anspruch 7, wobei

eine Vielzahl bekannter Zelltypen mittels Durchflusszytometrie analysiert wird, um ein Histogramm der Intensität der Vorwärtsstreuung (FSC) oder der Intensität der Seitenstreuung (SSC) und der Partikelanzahl oder ein Streuungsdiagramm der Intensität der Vorwärtsstreuung (FSC) und der Intensität der Seitenstreuung (SSC) für Zellkerne, die von jedem Zelltyp stammen, zu erstellen und einen Bereich oder eine Region, in der die Zellkerne jedes Zelltyps reichlicher vorkommen als die Zellkerne anderer Zelltypen, auf der Grundlage der Vorwärtsstreuungsintensität (FSC) und/oder der Seitenstreuungsintensität (SSC) zu bestimmen; und

ein behandeltes Produkt nach der Vorbehandlung mittels Durchflusszytometrie gemessen wird, um auf dieselbe Weise ein Histogramm oder Streudiagramm zu erzeugen, und in dem Histogramm oder Streudiagramm eine Partikelpopulation, die in dem vorab für Zellkerne, die vom Typ her mit den Zellkernen von Interesse identisch sind, festgelegten Bereich oder der Region vorhanden ist, sortiert oder fraktioniert wird.

**9.** Verfahren nach Anspruch 8, wobei:

Standardpartikel mit unterschiedlichen bekannten Partikelgrößen mittels Durchflusszytometrie analysiert werden, um aus der durch die Analyse erhaltenen Intensität der Vorwärtsstreuung (FSC)) und den bekannten Partikelgrößen der Standardsubstanzen eine Kalibrierungskurve zu erstellen;

ein behandeltes Produkt nach der Vorbehandlung mittels Durchflusszytometrie analysiert wird und die durch die Analyse erhaltene Intensität der Vorwärtsstreuung (FSC) auf die Kalibrierungskurve angewendet wird, um eine Partikelgröße zu berechnen, und

eine Partikelpopulation mit vorbestimmten Partikelgrößen sortiert oder fraktioniert wird;

und vorzugsweise:

wobei ein Bereich, der aus einer Partikelpopulation mit Partikelgrößen von 10 μm oder mehr besteht, von denen 95 % oder mehr Partikelgrößen von 11 μm bis 20 μm aufweisen, ausgewählt wird, um eine Partikelpopulation zu sortieren oder zu fraktionieren, die mit aus Tumorzellen stammenden Partikeln angereichert ist; oder

wobei ein Bereich, der aus einer Partikelpopulation mit Partikelgrößen von 8 μm oder weniger besteht, von denen 95 % oder mehr Partikelgrößen von 4 μm bis 7 μm aufweisen, ausgewählt wird, um eine Partikelpopulation zu sortieren oder zu fraktionieren, die mit aus Lymphozyten stammenden Partikeln angereichert ist.

**10.** Verfahren zur Analyse von Biomarkern, die in Zellen oder Zellkernen von Interesse vorhanden sind, unter Verwendung einer Probe, die mit Zellen und Zellkernen, erhalten durch dem Verfahren gemäß irgendeinem der Ansprüchen 1 bis 9, angereichert ist.

**11.** Verfahren nach Anspruch 10, wobei die Zellen von Interesse Tumorzellen sind und/oder wobei der Biomarker ein Polynukleotid, ein Zellkernprotein, ein Zellkernmembranprotein oder ein Zytoskelettprotein ist, wobei das Polynukleotid vorzugsweise ein krebsrelevantes Gen umfasst und das Zellkernprotein einen Transkriptionsfaktor oder ein zellzyklusrelevantes Protein umfasst.

**12.** Verfahren zur Bestimmung eines DNA-Index, umfassend:

Gewinnen einer mit Tumorzellen oder deren Zellkernen angereicherten Probe (mit Tumorzellkernen angereicherte Probe) aus einem Gewebestück nach dem Verfahren gemäß irgendeinem der Ansprüche 7 bis 9, Gewinnen einer Probe, die nicht mit Tumorzellen oder deren Zellkernen angereichert ist (nicht mit Tumorzellkernen angereicherte Probe oder Normalprobe) aus demselben Gewebestück oder einem Stück Normalgewebe auf dieselbe Weise, mit der Ausnahme, dass der Schritt des Sortierens oder Fraktionierens einer Partikelpopulation, die in Tumorzellen oder deren Zellkernen reichlich vorhanden sind, nicht durchgeführt wird, und

Erstellen eines Histogramms der Partikelanzahl und der DNA-Menge für jede Probe, Bestimmen einer DNA-Menge bei der größten Partikelanzahl und Ermitteln eines DNA-Indexes anhand der folgenden Gleichung:

DNA-Index = DNA-Menge bei der größten Partikelanzahl in der mit Tumorzellkernen angereicherten Probe / DNA-Menge bei der größten Partikelanzahl in der nicht mit Tumorzellkernen angereicherten Probe oder der normalen Zellprobe.

## Revendications

1. Procédé pour obtenir un échantillon enrichi en cellules ou en noyaux cellulaires d'intérêt à partir d'un fragment de tissu, le procédé comprenant :

   la réalisation d'un prétraitement comprenant l'application au fragment de tissu d'un traitement par une hydrolase qui ne dégrade pas une protéine nécessaire au maintien de la morphologie du noyau cellulaire,
   l'hydrolase étant la thrombine, une endopeptidase de proline ou l'hyaluronidase, ainsi qu'un broyage par jet d'eau et/ou un broyage par ultrasons afin d'obtenir une population de particules comprenant des cellules ou des noyaux cellulaires séparés individuellement, et
   la visualisation des composants dans les noyaux cellulaires de la population de particules obtenue lors du prétraitement, puis le tri ou le fractionnement par cytométrie en flux de la population de particules afin d'obtenir au moins une fraction de la population de particules significativement abondante en cellules ou en noyaux cellulaires d'intérêt sur la base des caractéristiques optiques des noyaux cellulaires dans les cellules séparées ou les noyaux cellulaires séparés.

2. Le procédé selon la revendication 1, dans lequel le broyage par jet d'eau est effectué en faisant tourner un élément de broyage à une vitesse de 6000 à 13000 tr/min et/ou dans lequel le broyage par ultrasons est effectué avec une amplitude de 20 à 40 %, par exemple pendant 30 secondes à 3 minutes.

3. Le procédé selon l'une quelconque des revendications 1 à 2, dans lequel les cellules d'intérêt sont des cellules tumorales, des lymphocytes ou des cellules normales provenant d'une partie non cancéreuse du tissu.

4. Le procédé selon l'une quelconque des revendications 1 à 3, dans lequel le fragment de tissu est fixé à l'aide d'un agent de fixation, par exemple dans lequel le fragment de tissu fixé est inclus dans un agent d'inclusion.

5. Le procédé selon la revendication 4, dans lequel le fragment de tissu fixé est inclus dans un agent d'inclusion avant le prétraitement, l'agent d'inclusion est retiré du fragment de tissu, et le fragment de tissu est rendu hydrophile.

6. Le procédé selon l'une quelconque des revendications 4 à 5, dans lequel, avant le prétraitement, une structure réticulée formée par la fixation est détruite par traitement thermique.

7. Le procédé selon l'une quelconque des revendications 1 à 6, dans lequel la population de particules obtenue par le prétraitement est analysée par cytométrie en flux afin de trier ou de fractionner une population de particules abondantes dans les cellules ou les noyaux cellulaires d'intérêt sur la base de l'intensité de diffusion vers l'avant (FSC) et/ou de l'intensité de diffusion latérale (SSC) dans un diagramme de diffusion obtenu lors de l'analyse.

8. Le procédé selon la revendication 7, dans lequel une pluralité de types cellulaires connus sont analysés par cytométrie en flux afin de générer un histogramme de l'intensité de diffusion vers l'avant (FSC) ou de l'intensité de diffusion latérale (SSC) et du nombre de particules, ou un diagramme de diffusion de l'intensité de diffusion vers l'avant (FSC) et de l'intensité de diffusion latérale (SSC) pour les noyaux cellulaires dérivés de chaque type cellulaire, et une zone ou une région plus abondante en noyaux de chaque type cellulaire qu'en noyaux d'autres types cellulaires est déterminée sur la base de l'intensité de diffusion vers l'avant et/ou de l'intensité de diffusion latérale (SSC) ; et un produit traité après le prétraitement est mesuré par cytométrie en flux pour générer un histogramme ou un scattergramme de la même manière, et dans l'histogramme ou le scattergramme, une population de particules présentes dans la zone ou la région prédéterminée pour les noyaux cellulaires de type identique aux noyaux cellulaires d'intérêt est triée ou fractionnée.

9. Le procédé selon la revendication 8, dans lequel :

   des particules étalons ayant différentes tailles de particules connues sont analysées par cytométrie en flux afin de générer une courbe d'étalonnage à partir de l'intensité de diffusion vers l'avant (FSC) obtenue par l'analyse et des tailles de particules connues des substances étalons ;
   un produit traité après le prétraitement est analysé par cytométrie en flux, et l'intensité de diffusion vers l'avant (FSC) obtenue par l'analyse est appliquée à la courbe d'étalonnage pour calculer une taille de particule, et une population de particules ayant des tailles de particules prédéterminées est triée ou fractionnée ; et
   de préférence :

dans lequel une région composée d'une population de particules ayant des tailles de particules de 10 μm ou plus, dont 95 % ou plus ont des tailles de particules comprises entre 11 μm et 20 μm, est sélectionnée pour trier ou fractionner une population de particules enrichie en particules dérivées de cellules tumorales ; ou dans lequel une région composée d'une population de particules ayant des tailles de particules de 8 μm ou moins, dont 95 % ou plus ont des tailles de particules comprises entre 4 μm et 7 μm, est sélectionnée pour trier ou fractionner une population de particules enrichie en particules dérivées de lymphocytes.

**10.** Procédé d'analyse de biomarqueurs présents dans des cellules ou des noyaux cellulaires d'intérêt à l'aide d'un échantillon enrichi en cellules et en noyaux cellulaires obtenus par le procédé selon l'une quelconque des revendications 1 à 9.

**11.** Le procédé selon la revendication 10, dans lequel les cellules d'intérêt sont des cellules tumorales, et/ou dans lequel le biomarqueur est un polynucléotide, une protéine nucléaire, une protéine de la membrane nucléaire ou une protéine du cytosquelette, dans lequel, de préférence, le polynucléotide comprend un gène lié au cancer, et la protéine nucléaire comprend un facteur de transcription ou une protéine liée au cycle cellulaire.

**12.** Procédé de détermination d'un indice d'ADN, comprenant :

l'obtention d'un échantillon enrichi en cellules tumorales ou en noyaux cellulaires de celles-ci (échantillon enrichi en noyaux de cellules tumorales) à partir d'un fragment de tissu par le procédé selon l'une quelconque des revendications 7 à 9,
l'obtention d'un échantillon qui n'est pas enrichi en cellules tumorales ou en noyaux cellulaires de celles-ci (échantillon non enrichi en noyaux de cellules tumorales ou échantillon normal) à partir du même fragment de tissu ou d'un fragment de tissu normal de la même manière, à l'exception du fait que l'étape consistant à trier ou à fractionner une population de particules significativement abondantes en cellules tumorales ou en noyaux de celles-ci n'est pas effectuée, et
la préparation d'un histogramme du nombre de particules et de la quantité d'ADN pour chaque échantillon, l'identification d'une quantité d'ADN correspondant au plus grand nombre de particules, et la détermination d'un indice d'ADN à partir de l'équation suivante :

Indice d'ADN = quantité d'ADN au nombre maximal de particules dans l'échantillon enrichi en noyaux de cellules tumorales / quantité d'ADN au nombre maximal de particules dans l'échantillon non enrichi en noyaux de cellules tumorales ou dans l'échantillon de cellules normales.

FIG. 1

1.5K
1.0K
500
0
COUNT
0
20M
40M
60M
80M
DAPI-A

FIG. 2

80M
60M
40M
20M
0
DAPI-A
5.0M
10M
15M
20M
25M
DAPI-H

UNTREATED
+ WATER-FLOW CRUSHING
7, 100 r p m
12, 800 r p m
D A P I
LAMIN

FIG. 3

FIG. 4
UNTREATED
+ WATER-FLOW CRUSHING
7, 100 r p m
12, 800 r p m
SCATTERGRAM
LAMIN
S S C − H
F S C − H
3.0M
2.0M
1.0M
0
2.0M
4.0M
6.0M
COUNT
1.5K
1.0K
500
LAMIN : A l e x a 4 8 8
LAMIN ANTIBODY
ISOTYPE MATCHED CONTROL

FIG. 5

LESS THAN 2n
1.0K
800
600
400
200
0
COUNT
2 n
2n OR MORE
0
10M
20M
30M
40M
DAPI－A

# FIG. 6

2n RECOVERY RATIO
30
20
10
0
%
●T10 5G
▲T10 8G
■RP-10
5000
7000
9000
11000
13000
rpm

RATIO OF
LESS THAN 2n TO 2n
15
10
5
0
fold
5000
7000
9000
11000
13000
rpm

RATIO OF
2n TO 2n OR MORE
100
80
60
40
20
0
%
5000
7000
9000
11000
13000
rpm

FIG. 7

DAPI
LAMIN
RP-10
T10

FIG. 8

RP-10

COUNT

300
200
100
0

$10^2$ $10^3$ $10^4$ $10^5$ $10^6$ $10^7$

LAMIN : Alexa488

● LAMIN ANTIBODY
● ISOTYPE MATCHED CONTROL

T10

COUNT

150
100
50
0

$10^2$ $10^3$ $10^4$ $10^5$ $10^6$ $10^7$

LAMIN : Alexa488

# FIG. 9

UNTREATED
+ ULTRASONIC CRUSHING
20%
30%
40%
DAPI
LAMIN

# FIG. 10

UNTREATED
+ ULTRASONIC CRUSHING
20%
30%
40%
SCATTERGRAM
SSC-H
FSC-H
LAMIN
LAMIN : Alexa488
LAMIN ANTIBODY
ISOTYPE MATCHED CONTROL

# FIG. 11

2n RECOVERY RATIO
%
30
20
10
0
0
10
20
30
40
50
CRUSHING INTENSITY (%)

RATIO OF
LESS THAN 2n TO 2n

15
10
5
0
f o l d
0
10
20
30
40
50
CRUSHING INTENSITY (%)

RATIO OF
2n TO 2n OR MORE
%
100
80
60
40
20
0
0
10
20
30
40
50
CRUSHING INTENSITY (%)

# FIG. 12

AMPLITUDE 20%
AMPLITUDE 30%
2n RECOVERY RATIO
%
30
20
10
0
0
1
2
3
CRUSHING TIME (min)
RATIO OF LESS THAN 2n TO 2n
f o l d
15
10
5
RATIO OF 2n TO 2n OR MORE
100
80
60
40
20

FIG. 13

D A P I

LAMIN

FIG. 14

600
400
200
0
COUNT
$10^2$
$10^3$
$10^4$
$10^5$
$10^6$
$10^7$
LAMIN : Alexa488

● LAMIN ANTIBODY
● ISOTYPE MATCHED CONTROL

FIG. 15

DAPI
LAMIN
DAPI
LAMIN
UNTREATED
THROMBIN
DISPASE
PROLINE ENDOPEPTIDASE
TRYPSIN
HYALURONIDASE

FIG. 16

UNTREATED
THROMBIN
DISPASE
PROLINE ENDOPEPTIDASE
TRYPSIN
HYALURONIDASE
COUNT
6.0K
4.0K
2.0K
0
10²
10³
10⁴
10⁵
10⁶
10⁷
LAMIN : Alexa488

FIG. 17

DAPI
LAMIN
THROMBIN
+
WATER-FLOW CRUSHING
AND ULTRASONIC
CRUSHING
DISPASE

FIG. 18

THROMBIN
+
WATER-FLOW CRUSHING
AND ULTRASONIC
CRUSHING

300
200
100
0
COUNT
$10^2$ $10^3$ $10^4$ $10^5$ $10^6$ $10^7$
LAMIN : Alexa488

DISPASE

2.0K
1.5K
1.0K
500
0
COUNT
$10^2$ $10^3$ $10^4$ $10^5$ $10^6$ $10^7$
LAMIN : Alexa488

● LAMIN ANTIBODY
● ISOTYPE MATCHED CONTROL

# FIG. 19

Jurkat
MDA-MB-231
T47D
SKBr3
SSC-H
FSC-H
0
1.0M
2.0M
3.0M
2.0M
4.0M
6.0M

# FIG. 20

×10^6
4
3
2
1
0
FSC-H INTENSITY
0
5
10
15
20
SIZE (μm)

FIG. 21

Jurkat
COUNT
250
200
150
100
50
0
0
2.0M
4.0M
6.0M
FSC-H
MDA-MB-231
COUNT
150
100
50
0
0
2.0M
4.0M
6.0M
FSC-H
T47D
COUNT
80
60
40
20
0
0
2.0M
4.0M
6.0M
FSC-H
SKBr3
COUNT
60
40
20
0
0
2.0M
4.0M
6.0M
FSC-H

FIG. 22

Jurkat
MDA-MB-231
T47D
SKBr3
COUNT
SSC-H
300
200
100
0
150
50
80
60
40
20
1.0M
2.0M
3.0M

# FIG. 23

3.0M
2.0M
1.0M
0
SSC−H
0
2.0M
4.0M
6.0M
FSC−H
CANCER-DERIVED CELL NUCLEI
LYMPHOCYTE-DERIVED CELL NUCLEI

# FIG. 24

Cyflow Space
FSC INTENSITY
300
250
200
150
100
50
0
0
5
10
15
20
SIZE (μm)

FACSAriaII
×10³
FSC-H INTENSITY
100
80
60
40
20
0
0
5
10
15
20
SIZE (μm)

# FIG. 25

Jurkat
MDA-MB-231
T47D
SKBr3
SSC
FSC
0
100
200
300
400

# FIG. 26

Jurkat
MDA-MB-231
T47D
SKBr3
SSC-H
FSC-H
0
50K
100K
150K
200K
250K

# FIG. 27

Jurkat
COUNT
250
200
150
100
50
0
0
100
200
300
400
SSC
MDA-MB-231
COUNT
500
400
300
200
100
0
0
100
200
300
400
SSC
T47D
COUNT
400
300
200
100
0
0
100
200
300
400
SSC
SKBr3
COUNT
60
40
20
0
0
100
200
300
400
SSC

# FIG. 28

Jurkat
COUNT
600
400
200
0
0
50K
100K
150K
200K
250K
SSC-H
MDA-MB-231
COUNT
300
200
100
0
0
50K
100K
150K
200K
250K
SSC-H
T47D
COUNT
300
200
100
0
0
50K
100K
150K
200K
250K
SSC-H
SKBr3
COUNT
200
150
100
50
0
0
50K
100K
150K
200K
250K
SSC-H

FIG. 29

Cyflow Space
FACSAriaII
SSC
FSC
SSC−H
FSC−H
400
300
200
100
0
250K
200K
150K
100K
50K
SKBr3
Jurkat

FIG. 30

Area 1
Area 2
SSC−H
FSC−H
250K
200K
150K
100K
50K
0

# FIG. 31

DAPI
CYTOKERATIN
Area 1
Area 2

# FIG. 32

NovoCyte Quanteon Flow Cytometer
Cyflow Space
3.0M
2.0M
1.0M
0
SSC−H
0.48%
0
2.0M
4.0M
6.0M
FSC−H
400
300
200
100
0
SSC
1.25%
0
100
200
300
400
FSC

FIG. 33

THROMBIN
DISPASE
MDA-MB-231
T47D
SKBr3
SSC-H
FSC-H
3.0M
2.0M
1.0M
0
2.0M
4.0M
6.0M
CELL LINE
Jurkat

# FIG. 34

THROMBIN
DISPASE
SSC−H
FSC−H
3.0M
2.0M
1.0M
0
4.0M
6.0M
CANCER-DERIVED CELL NUCLEI
LYMPHOCYTE-DERIVED CELL NUCLEI

FIG. 35

N=49
y=0. 80x-2. 39
R=0. 9126
Ki-67-POSITIVE RATIO (%)
WITH GATING OF
PARTICULAR REGION
0
20
40
60
80
100
Ki-67 LABELING INDEX (%)
BY PATHOLOGIST

N=49
y=0. 54x-1. 40
R=0. 8874
Ki-67-POSITIVE RATIO (%)
WITHOUT GATING OF
PARTICULAR REGION
0
20
40
60
80
100
Ki-67 LABELING INDEX (%)
BY PATHOLOGIST

FIG. 36

100
80
60
40
20
0
SENSITIVITY (%)
0
20
40
60
80
100
100 - SPECIFICITY (%)
WITH GATING OF CANCER CELL-DERIVED CELL NUCLEUS REGION
WITHOUT GATING OF CANCER CELL-DERIVED CELL NUCLEUS REGION

# FIG. 37

WITH GATING OF CANCER CELL-DERIVED CELL NUCLEUS REGION
WITHOUT GATING OF CANCER CELL-DERIVED CELL NUCLEUS REGION
ER-POSITIVE SPECIMEN A
ER-NEGATIVE SPECIMEN C
POSITIVE RATIO 80. 1%
POSITIVE RATIO 62. 0%
POSITIVE RATIO 5. 3%
POSITIVE RATIO 5. 2%
COUNT
ER : Alexa488
ER ANTIBODY
ISOTYPE MATCHED CONTROL

FIG. 38

WITH GATING OF CANCER CELL-DERIVED CELL NUCLEUS REGION
WITHOUT GATING OF CANCER CELL-DERIVED CELL NUCLEUS REGION
PgR-POSITIVE SPECIMEN B
PgR-NEGATIVE SPECIMEN C
POSITIVE RATIO 62. 1%
POSITIVE RATIO 42. 3%
POSITIVE RATIO 5. 7%
POSITIVE RATIO 5. 5%
COUNT
PgR : Alexa647
ER : Alexa647
PgR ANTIBODY
ISOTYPE MATCHED CONTROL

# FIG. 39

WITH GATING OF CANCER CELL-DERIVED CELL NUCLEUS REGION
WITHOUT GATING OF CANCER CELL-DERIVED CELL NUCLEUS REGION
POSITIVE RATIO 78. 9%
POSITIVE RATIO 48. 8%
POSITIVE RATIO 7. 1%
POSITIVE RATIO 6. 5%
COUNT
CYTOKERATIN 7 : Alexa488
CYTOKERATIN 20 : Alexa647
CYTOKERATIN ANTIBODY
ISOTYPE MATCHED CONTROL

# FIG. 40

LYMPHOCYTE-
DERIVED CELL NUCLEI

5.0M
4.0M
3.0M
2.0M
1.0M
0
SSC-H
0
2.0M
4.0M
6.0M
8.0M
10M
FSC-H

NORMAL CELL-
DERIVED CELL NUCLEI

5.0M
4.0M
3.0M
2.0M
1.0M
0
SSC-H
0
2.0M
4.0M
6.0M
8.0M
10M
FSC-H

CANCER CELL-
DERIVED CELL NUCLEI

5.0M
4.0M
3.0M
2.0M
1.0M
0
SSC-H
0
2.0M
4.0M
6.0M
8.0M
10M
FSC-H

FIG. 41

5.0M
4.0M
3.0M
2.0M
1.0M
0
SSC-H
0
2.0M
4.0M
6.0M
8.0M
10M
FSC-H
Area 3
Area 4
CANCER CELL-DERIVED CELL NUCLEI
NORMAL CELL-DERIVED CELL NUCLEI
LYMPHOCYTE-DERIVED CELL NUCLEI

FIG. 42

WITH GATING OF CANCER CELL-DERIVED CELL NUCLEUS REGION
WITHOUT GATING OF CANCER CELL-DERIVED CELL NUCLEUS REGION
CASE 1 (SIGMOID)
CASE 2 (RECTUM)
POSITIVE RATIO 87. 5%
POSITIVE RATIO 79. 0%
POSITIVE RATIO 82. 1%
POSITIVE RATIO 71. 9%
COUNT
Ki-67:Alexa647
Ki-67 ANTIBODY
ISOTYPE MATCHED CONTROL

# FIG. 43

WITH GATING OF CANCER CELL-DERIVED CELL NUCLEUS REGION
WITHOUT GATING OF CANCER CELL-DERIVED CELL NUCLEUS REGION
POSITIVE RATIO 4. 7%
POSITIVE RATIO 5. 0%
POSITIVE RATIO 44. 4%
POSITIVE RATIO 29. 7%
COUNT
CYTOKERATIN 7 : Alexa488
CYTOKERATIN 20 : Alexa647
CYTOKERATIN ANTIBODY
ISOTYPE MATCHED CONTROL


# FIG. 44

CANCER PROPORTION 100%
CANCER PROPORTION 10%
FLUORESCENT INTENSITY
CYCLE
FRACTIONATED (CONCENTRATED)
NON-FRACTIONATED (NOT CONCENTRATED)

# FIG. 45

(a)
COUNT
2.0K
1.5K
1.0K
500
0
0
5.0M
10M
15M
20M
25M
DAPI－A
(b)
DAPI－A
40M
30M
20M
10M
0
0
5.0M
10M
15M
20M
DAPI－H
(c)
SSC－H
3.0M
2.0M
1.0M
0
0
2.0M
4.0M
6.0M
FSC－H

# FIG. 46

GATING OF CANCER CELL-DERIVED CELL NUCLEUS REGION
DONE
NOT DONE

(a)

SPECIMEN 1
COUNT (NORMALIZED)
100
80
60
40
20
0
0
5.0M
10M
15M
20M
25M
DAPI-A

(b)

SPECIMEN 2
COUNT (NORMALIZED)
100
80
60
40
20
0
0
5.0M
10M
15M
20M
25M
DAPI-A

(c)

SPECIMEN 3
COUNT (NORMALIZED)
100
80
60
40
20
0
0
5.0M
10M
15M
20M
25M
DAPI-A

# FIG. 47

COUNT (NORMALIZED)
100
80
60
40
20
0
0 5.0M 10M 15M 20M 25M
DAPI－A
GATING OF CANCER CELL-DERIVED CELL NUCLEUS REGION
DONE
NOT DONE

# FIG. 48

N＝17
y＝0. 93x＋0. 30
R＝0. 9407
PRESENT METHOD DNA index
2.4
2.2
2.0
1.8
1.6
1.4
1.2
1.0
0.8
0.8 1.0 1.2 1.4 1.6 1.8 2.0 2.2 2.4
CONVENTIONAL METHOD DNA index

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005315862 A
- JP 2014001949 A
- JP 2019207201 A
- WO 2010041423 A
- JP 2009063508 A
- JP 2009122115 A
- JP 2009537822 A
- WO 2018203568 A
- JP 2015190922 A
- WO 2019168085 A
- US 2020408770 A


### Non-patent literature cited in the description


- **MEI-YIN C POLLEY et al.** An international Ki67 reproducibility study. *J Natl Cancer Inst*, 18 December 2013, vol. 105 (24), 1897-906
- **LEERS MP et al.** Multi-parameter flow cytometric analysis with detection of the Ki67-Ag in paraffin embedded mammary carcinomas. *Cytometry*, 01 March 1997, vol. 27 (3), 283-9
- The Japanese Society of Pathology. *Guidelines on the Handling of Pathological Tissue Samples for Clinical Genomics*
- **ATSUKO KITANO et al.** Tumour-infiltrating lymphocytes are correlated with higher expression levels of PD-1 and PD-L1 in early breast cancer. *ESMO Open*, 02 May 2017, vol. 2 (2), e000150
- **HIROKI KUSAMA et al.** Prognostic value of tumor cell DNA content determined by flow cytometry using formalin-fixed paraffin-embedded breast cancer tissues. *Breast Cancer Res Treat*, 2019, vol. 176, 75-85
- **WILLEM E. CORVER et al.** High-Resolution Multiparameter DNA Flow Cytometry for the Detection and Sorting of Tumor and Stromal Subpopulations from Paraffin-Embedded Tissues. *Current Protocols in Cytometry*, 01 January 2011